# EUROPEAN PATENT APPLICATION

(11) **EP 4 410 775 A1**
(43) Date of publication of application: **07.08.2024**
(21) Application number: 22876577.2
(22) Date of filing: 30.09.2022
(51) Int. Cl.: C07D 211/46, C07B 61/00, C07D 401/12, C07D 401/14, C07D 409/14, C07D 417/14, C40B 50/14

(54) **METHOD FOR FORMING BOND THROUGH COUPLING REACTION**

(30) Priority: 30.09.2021 JP 2021161903; 06.01.2022 JP 2022000841; 06.07.2022 JP 2022109266
(71) Applicant: CHUGAI SEIYAKU KABUSHIKI KAISHA, Tokyo 115-8543 (JP)
(72) Inventor: ITO, Taisuke, Gotemba-shi, Shizuoka 412-8513 (JP)
(74) Representative: Vossius & Partner Patentanwälte Rechtsanwälte mbB
(86) International application number: PCT/JP2022/036853
(87) International publication number: WO 2023/054715

(57) **Abstract**

The present invention provides a method of producing a compound by a cross-coupling reaction, the method comprising: reacting compound 1 having a leaving group X¹ on a carbon atom of an aromatic ring with compound 2 having a reactive group capable of a C-O bond formation reaction or a C-N bond formation reaction by substitution with the leaving group, in the presence of a catalyst and a base, in a solvent containing an amide-based solvent represented by formula A, wherein R¹, R², and R³ are each independently C₁₋₄ alkyl provided that the sum of carbon atoms of R¹, R², and R³ is 4 or more and 6 or less, wherein X¹ is a halogen atom or -O-SO₂-R⁴.

## Description

### TECHNICAL FIELD

The present invention relates to a method of forming a bond by a coupling reaction, in particular a method of forming a carbon-oxygen bond (C-O bond) or a carbon-nitrogen bond (C-N bond). The present invention further relates to a method of producing a compound, comprising a step of forming a bond by a coupling reaction.

### BACKGROUND ART

Methods of forming carbon-oxygen or carbon-nitrogen bonds by cross-coupling reactions are widely used in compound synthesis in pharmaceutical developments and the like. For example, there have been many reports of cross-coupling reactions using palladium catalysts for aryl halides (Non Patent Literatures 1 to 3).

As one of the side reactions in the cross-coupling reaction described above, it is known that de-halogenated products are generated when the halogen atom of the reaction substrate, such as aryl halide, is replaced by a hydrogen atom (Non Patent Literature 1). It has been reported that the generation of de-halogenated productscan be suppressed by changing the ligand or reacting at low temperatures (Non Patent Literature 2).

It has also been reported that, when a substrate containing a functional group that is highly sensitive to bases, such as a carboxylic acid ester structure, is used in the reaction, it is preferable to use a P2Et phosphazene base, rather than an alkali metal base such as NaOtBu or LiHMDS, as the base used in the reaction (Non Patent Literature 6).

The effects of solvents in cross-coupling reactions using palladium catalysts have been reported (Non Patent Literatures 4 and 5). It has also been reported that, for the purpose of preventing flow path blockage in flow chemistry, N,N-dimethyloctanamide is added to the solvent (Non Patent Literature 7).

### CITATION LIST

### PATENT LITERATURE

[Patent Literature 1] U.S. Patent No. 4,453,017
[Patent Literature 2] Japanese Patent Laid-Open No. 2006-213692

### NON PATENT LITERATURE

[Non Patent Literature 1] Dorel, R. et al., Angew. Chem. Int. Ed. 2019, 58, 17118.
[Non Patent Literature 2] Surry, D.S. et al. Chem. Sci. 2011, 2, 27.
[Non Patent Literature 3] Anderson, K.W. et al., J. Am. Chem. Soc. 2006, 128, 10694.
[Non Patent Literature 4] Sherwood, J. et al., Green. Chem. 2019, 21, 2164.
[Non Patent Literature 5] Molina De La Torre, J.A. et al., Organometallics 2013, 32, 5428.
[Non Patent Literature 6] Santanilla, A.B. et al., Organic Letters, 2015, 17, 3370.
[Non Patent Literature 7] Yang, J.C. et al., Angew. Chem. Int. Ed. 2016, 55, 2531.

### SUMMARY OF INVENTION

### TECHNICAL PROBLEM

Cross-coupling reactions using palladium catalysts are widely used in compound synthesis in pharmaceutical developments and the like, and numerous palladium catalysts have been developed. However, it has been confirmed by the present inventors that they have some problems such as low conversion rates depending on the substrates and/or the large number of by-products in which the leaving group is substituted with hydrogen, and there are still problems that have been not solved by conventional coupling conditions. For example, in the carbon-nitrogen bond formation reaction by coupling using aryl halides and amines as substrates, even under the conditions referred to Non Patent Literature 2, problems such as the low conversion rate of reaction and the prominent generation of de-halogenated productsoccur when specific substrates are used. In addition, in the synthesis of arenol by carbon-oxygen bond formation reaction by coupling using aryl halides and water as substrates, even under the conditions referred to Non Patent Literature 3, the generation of impurities such as de-halogenated products cannot be sufficiently suppressed when specific substrates are used. Furthermore, no improvement effect has been obtained even with the use of reaction solvents as described in literatures referred to Non Patent Literatures 4, 5, and 7.

The present invention has been made in view of such circumstances, and in one aspect, an object of the present invention is to provide a method of forming a carbon-oxygen bond or a carbon-nitrogen bond, capable of improving the conversion rate and/or inhibiting by-products in cross-coupling reactions using a palladium catalyst for aryl halides. In one aspect, another object of the present invention is to provide a method of synthesizing a compound, comprising the method described above. In another aspect, yet another object of the present invention is to provide a method of improving the conversion rate and/or suppressing by-products such as de-halogenated products, the method is applicable in cross-coupling reactions using a palladium catalyst for aryl halides.

### SOLUTION TO PROBLEM

The present inventors have found that the use of a specific solvent demonstrates a preferred reactivity in cross-coupling reactions using a palladium catalyst, and further have found that this solvent is applicable to carbon-oxygen bond (C-O bond) or carbon-nitrogen bond (C-N bond) formation reactions, and have finally completed the present invention. In one aspect, the present invention discloses the following invention.

[A-1] A method of producing a compound by a cross-coupling reaction, the method comprising:
reacting compound 1 having a leaving group X¹ on a carbon atom of an aromatic ring with compound 2 having a reactive group capable of a C-O bond formation reaction or a C-N bond formation reaction by substitution with the leaving group, in the presence of a catalyst and a base, in a solvent containing an amide-based solvent represented by formula A: 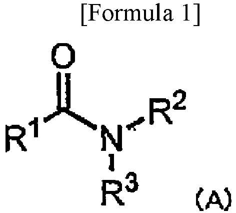
wherein R¹, R², and R³ are each independently C₁₋₄ alkyl provided that the sum of carbon atoms of R¹, R², and R³ is 4 or more and 6 or less, wherein
X¹ is a halogen atom or -O-SO₂-R⁴;
R⁴ is C₁₋₆ alkyl optionally substituted with one or more fluorine atoms, or phenyl optionally substituted with one or more fluorine atoms or C₁₋₆ alkyl optionally substituted with a fluorine atom; and
compound 2 has a hydroxy capable of forming a C-O bond or an H-N group capable of forming a C-N bond.

[A-2] The method according to [A-1], wherein the catalyst is a palladium catalyst or a nickel catalyst.

[A-3] The method according to [A-1] or [A-2], wherein the catalyst is a palladium catalyst.

[A-4] The method according to any of [A-1] to [A-3], wherein the number of the leaving group in compound 1 is one, or two or three where the leaving groups may be the same or different.

[A-5] The method according to any of [A-1] to [A-4], wherein the number of the leaving group in compound 1 is one.

[A-6] The method according to any of [A-1] to [A-5], wherein the number of the reactive group of compound 2 is one, or two or three where the reactive groups may be the same or different.

[A-7] The method according to any of [A-1] to [A-6], wherein the number of the reactive group of compound 2 is one.

[A-8] The method according to any of [A-1] to [A-7], wherein compound 1 or compound 2 is supported on a resin for solid-phase synthesis.

[A-9] The method according to any of [A-1] to [A-8], wherein compound 2 is
1) water or a compound having a hydroxy capable of forming a C-O bond, represented by HO-R⁵, wherein
   R⁵ is C₁₋₆ alkyl, C₂₋₆ alkenyl, C₂₋₆ alkynyl, C₃₋₈ cycloalkyl, C₇₋₁₄ aralkyl, C₆₋₁₀ aryl, or 5- to 10-membered heteroaryl containing one or more ring heteroatoms independently selected from O, N and S, each of which is optionally substituted with one or more groups independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, aminocarbonyl, (C₁₋₆ alkyl)aminocarbonyl, di(C₁₋₆ alkyl)aminocarbonyl, and 4- to 8-membered cyclic aminocarbonyl; or
2) a compound having an H-N group capable of forming a C-N bond, represented by HNR⁶R⁷, wherein
   R⁶ and R⁷ together with the nitrogen atom to which they are bonded form a 5- to 7-membered saturated heterocycle, wherein the heterocycle is optionally substituted with one or more substituents independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl, or
   R⁶ and R⁷ are each independently a hydrogen atom, C₁₋₆ alkyl, C₂₋₆ alkenyl, C₂₋₆ alkynyl, C₃₋₈ cycloalkyl, (C₁₋₆ alkyl)carbonyl, (C₆₋₁₀ aryl)carbonyl, 5- to 10-membered heteroarylcarbonyl containing one or more ring heteroatoms independently selected from O, N and S, C₇₋₁₄ aralkyl, C₆₋₁₀ aryl, or 5- to 10-membered heteroaryl containing one or more ring heteroatoms independently selected from O, N and S, each of which is optionally substituted with one or more substituents independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl.

[A-10] The method according to any of [A-1] to [A-9], wherein compound 1 is a compound represented by X¹-Ar², wherein
X¹ is a chlorine atom, a bromine atom, an iodine atom, or -O-SO₂-R⁴;
R⁴ is C₁₋₆ alkyl optionally substituted with one or more fluorine atoms, or phenyl optionally substituted with one or more fluorine atoms or C₁₋₆ alkyl optionally substituted with a fluorine atom; and
Ar² is C₆₋₁₀ aryl, or 5- to 10-membered heteroaryl containing one or more ring heteroatoms independently selected from O, N and S, each of which is optionally substituted with one or more groups independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl.

[A-11] The method according to any of [A-1] to [A-10], wherein compound 1 is supported on a resin for solid-phase synthesis and includes the compound represented by X¹-Ar² described in [A-10] as a part of a chemical structure.

[A-12] The method according to any of [A-10] to [A-11], wherein X¹ is a chlorine atom, a bromine atom, or an iodine atom.

[A-13] The method according to any of [A-10] to [A-12], wherein Ar² is independently selected from the group consisting of phenyl, naphthyl, pyrrolyl, thienyl, furyl, pyridyl, thiazolyl, isothiazolyl, pyrazolyl, oxazolyl, isoxazolyl, imidazolyl, triallyl, pyrimidyl, pyrazinyl, pyridazinyl, quinolinyl, isoquinolinyl, 4H-quinolidinyl, phthalazinyl, naphthyridinyl, quinoxalinyl, quinazolinyl, cinnolinyl, pteridinyl, indolyl, indolinyl, benzothiophenyl, benzofuranyl, benzisothiazolyl, benzisoxazolyl, indazolyl, benzimidazolyl, benzotriazolyl, azaindolyl, and imidazopyridyl, each of which is optionally substituted.

[A-14] The method according to any of [A-10] to [A-13], wherein Ar² is phenyl or pyridyl, each of which is optionally substituted.

[A-15] The method according to any of [A-10] to [A-14], wherein X¹ is a bromine atom;
Ar² is phenyl or pyridyl, each of which is optionally substituted with one or more groups independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl.

[A-16] The method according to any of [A-1] to [A-15], wherein the solvent is selected from the group consisting of N,N-dimethylpropionamide (DMPr), N,N-diethylacetamide (DEAc) and N,N-diethylpropionamide (DEPr).

[A-17] The method according to any of [A-1] to [A-16], wherein the solvent is N,N-dimethylpropionamide (DMPr).

[A-18] The method according to any of [A-1] to [A-17], wherein the solvent is a solvent containing at least one selected from the group consisting of N,N-dimethylpropionamide (DMPr), N,N-diethylacetamide (DEAc) and N,N-diethylpropionamide (DEPr) at 30 v/v% or more, 50 v/v% or more, 70 v/v% or more, or 90 v/v% or more.

[A-19] The method according to any of [A-1] to [A-18], wherein the solvent is a solvent containing N,N-dimethylpropionamide (DMPr) at 30 v/v% or more, 50 v/v% or more, 70 v/v% or more, or 90 v/v% or more.

[A-20] The method according to any of [A-1] to [A-19], wherein the cross-coupling reaction is performed at 0 to 200°C, 0 to 150°C, 0 to 100°C, 10 to 80°C, or 25 to 80°C.

[A-21] The method according to any of [A-1] to [A-20], wherein the molar ratio of compound 1 to compound 2 used is compound 1/compound 2 = from 0.0005 to 500, from 0.005 to 200, or from 0.05 to 20.

[A-22] The method according to any of [A-1] to [A-21], wherein the molar ratio of the catalyst used is from 0.01 to 100 mol%, from 0.1 to 50 mol%, or from 1 to 25 mol% relative to compound 1 or compound 2.

[A-23] The method according to any of [A-1] to [A-22], wherein the catalyst is a catalyst containing a palladium complex represented by any of the following general formulae (Cat1), (Cat2), (Cat3), (Cat4) and (Cat5): 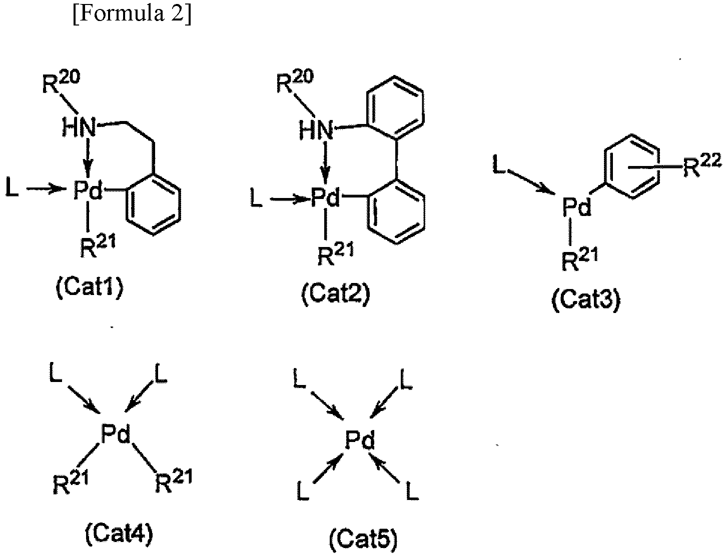
wherein R²⁰ is a hydrogen atom, C₁₋₆ alkyl, or C₆₋₁₀ aryl; R²¹ is halogen or -O-SO₂-CH₃; R²² is a hydrogen atom, C₁₋₆ alkyl optionally substituted with one or more fluorine atoms, or (C₁₋₆ alkoxy)carbonyl optionally substituted with tri(C₁₋₆ alkyl)silyl;
L is independently a monodentate ligand of the following general formula (L1), (L2), (L3), (L4), (L5), (L6) or (L7), or two L are a bidentate ligand of the following general formula (L8), (L9), (L10), (L1 1) or (L12):

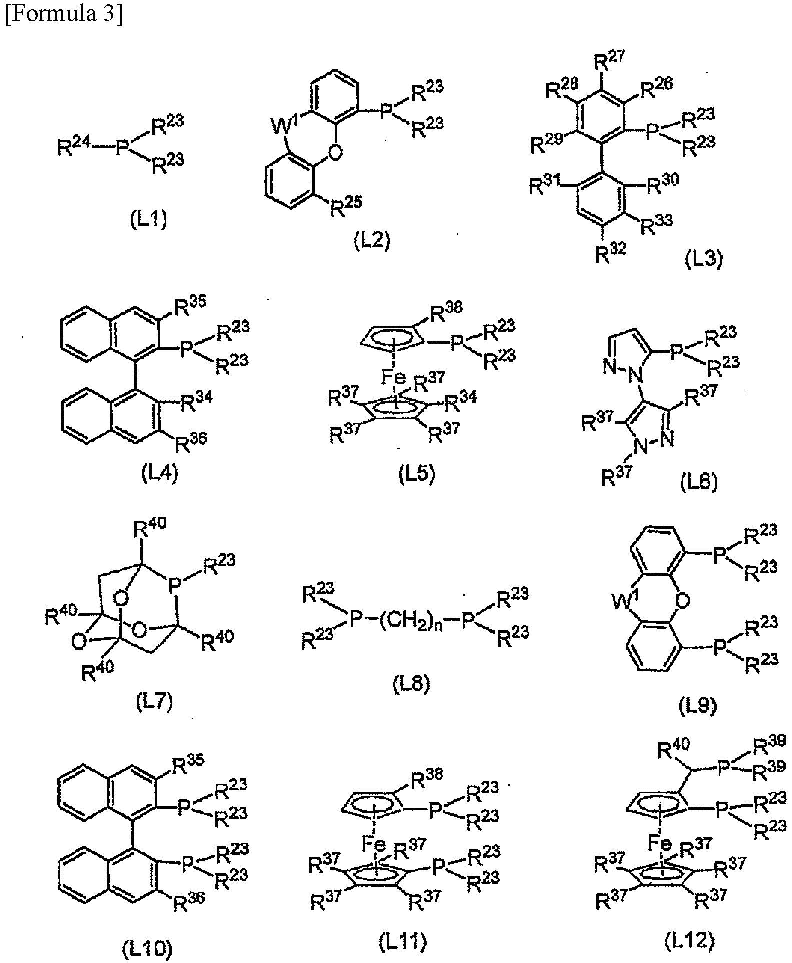

wherein R²³ is independently tert-butyl, cyclohexyl, 2-furanyl, 2-thienyl, 2-pyridyl, phenyl, or adamantyl, wherein the phenyl is optionally substituted with one or more fluorine atoms, C₁₋₆ alkyl optionally substituted with a fluorine atom, C₁₋₆ alkoxy, or dimethylamino;
R²⁴ is C₁₋₆ alkyl, cyclohexyl, 2-furanyl, 2-thienyl, 2-pyridyl, N-phenyl-2-pyrrolyl, N-phenyl-2-indolyl, phenyl, or adamantyl, wherein the phenyl is optionally substituted with one or more fluorine atoms, C₁₋₆ alkyl optionally substituted with a fluorine atom, C₁₋₆ alkoxy, morpholino, or dimethylamino;
R²⁵ is a hydrogen atom, C₁₋₆ alkyl, or C₁₋₆ alkoxy;
W¹ is -C(CH₃)₂-, or -NH-;
R²⁶, R²⁷, R²⁸, and R²⁹ are each independently a hydrogen atom, C₁₋₆ alkyl, C₁₋₆ alkoxy, or morpholino;
R³⁰, R³¹, and R³² are each independently a hydrogen atom, C₁₋₆ alkyl, C₁₋₆ alkoxy, or dimethylamino;
R³³ is a hydrogen atom or -SO₂-O-M, wherein M is lithium, sodium, or potassium;
R³⁴ is a hydrogen atom, C₁₋₆ alkyl, or C₁₋₆ alkoxy;
R³⁵ and R³⁶ are each independently a hydrogen atom, C₁₋₆ alkyl, or C₁₋₆ alkoxy;
R³⁷ is a hydrogen atom, or phenyl optionally substituted with C₁₋₆ alkyl;
R³⁸ is independently a hydrogen atom, phenyl optionally substituted with C₁₋₆ alkyl, or -CH(CH₃)-N(CH₃)₂;
R³⁹ is tert-butyl, cyclohexyl, or adamantyl;
R⁴⁰ is a hydrogen atom or C₁₋₆ alkyl; and an arrow represents a coordination bond.

[A-24] The method according to any of [A-1] to [A-22], wherein the catalyst is a catalyst containing a palladium complex represented by any of the following general formulae (Cat6) and (Cat7): 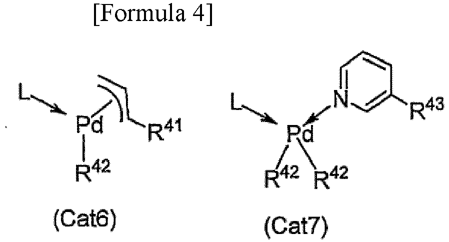 wherein R⁴¹ is a hydrogen atom or phenyl optionally substituted with C₁₋₆ alkyl; R⁴² is independently halogen; R⁴³ is a fluorine atom or a chlorine atom; and L is an N-heterocyclic carbene ligand represented by the following general formula (L12) or (L13): 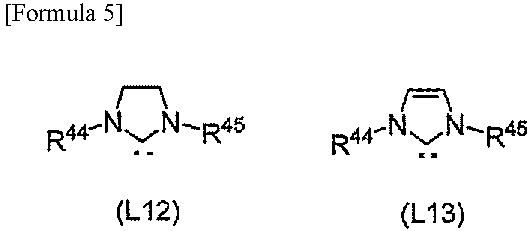 wherein R⁴⁴ and R⁴⁵ each independently represent C₁₋₆ alkyl, cyclohexyl, adamantyl, or phenyl, wherein the phenyl is optionally substituted with one or more C₁₋₆ alkyl, C₁₋₆ alkoxy, or dimethylamino, and a carbon atom with × × represents a carbene, and an arrow represents a coordination bond.

[A-25] The method according to any of [A-1] to [A-22], wherein the catalyst is a catalyst containing a palladium complex formed by a combination of a palladium compound selected from the group consisting of bis(allylchloropalladium(II)), tetrakis(triphenylphosphine)palladium(0), tris(dibenzylideneacetone)dipalladium(0)-chloroform adduct, a palladium(p-cinnamyl)chloride dimer, a (1-methylallyl)palladium chloride dimer, (1,5-cyclooctadiene)bis(trimethylsilylmethyl)palladium(II), a (2'-amino-1,1'-biphenyl-2-yl)methanesulfonatopalladium(II) dimer, and palladium(II) acetate, with a ligand selected from a ligand represented by a monodentate ligand of the following general formula (L1), (L2), (L3), (L4), (L5), (L6) or (L7), or a bidentate ligand of the following general formula (L8), (L9), (L10), (L11) or (L12): 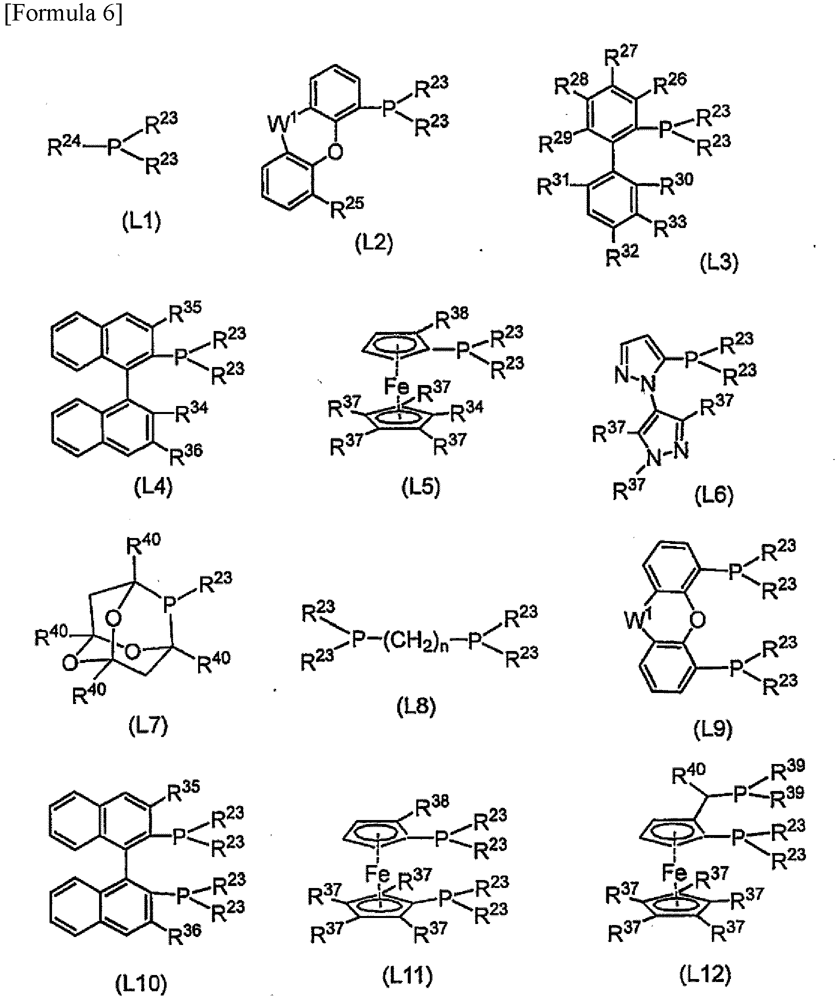
wherein R²³ is tert-butyl, cyclohexyl, 2-furanyl, 2-thienyl, 2-pyridyl, phenyl, or adamantyl, wherein the phenyl is optionally substituted with one or more fluorine atoms, C₁₋₆ alkyl optionally substituted with a fluorine atom, C₁₋₆ alkoxy, or dimethylamino;
R²⁴ is C₁₋₆ alkyl, cyclohexyl, 2-furanyl, 2-thienyl, 2-pyridyl, N-phenyl-2-pyrrolyl, N-phenyl-2-indolyl, phenyl, or adamantyl, wherein the phenyl is optionally substituted with one or more fluorine atoms, C₁₋₆ alkyl optionally substituted with a fluorine atom, C₁₋₆ alkoxy, morpholino, or dimethylamino;
R²⁵ is a hydrogen atom, C₁₋₆ alkyl, or C₁₋₆ alkoxy;
W¹ is -C(CH₃)₂-, or -NH-;
R²⁶, R²⁷, R²⁸, and R²⁹ are each independently a hydrogen atom, C₁₋₆ alkyl, C₁₋₆ alkoxy, or morpholino;
R³⁰, R³¹, and R³² are each independently a hydrogen atom, C₁₋₆ alkyl, C₁₋₆ alkoxy, or dimethylamino;
R³³ is a hydrogen atom or -SO₂-O-M, wherein M is lithium, sodium, or potassium;
R³⁴ is a hydrogen atom, C₁₋₆ alkyl, or C₁₋₆ alkoxy;
R³⁵ and R³⁶ are each independently a hydrogen atom, C₁₋₆ alkyl, or C₁₋₆ alkoxy;
R³⁷ is a hydrogen atom, or phenyl optionally substituted with C₁₋₆ alkyl;
R³⁸ is a hydrogen atom, phenyl optionally substituted with C₁₋₆ alkyl, or -CH(CH₃)-N(CH₃)₂;
R³⁹ is tert-butyl, cyclohexyl, or adamantyl;
R⁴⁰ is a hydrogen atom or C₁₋₆ alkyl; and an arrow represents a coordination bond, or a ligand being a salt thereof.

[A-26] The method according to any of [A-1] to [A-23], wherein the catalyst is a catalyst containing a palladium complex selected from a Buchwald first-generation catalyst precursor (G1), a Buchwald second-generation catalyst precursor (G2), a Buchwald third-generation catalyst precursor (G3), a Buchwald fourth-generation catalyst precursor (G4), a Buchwald fifth-generation catalyst precursor (G5), or a Buchwald sixth-generation catalyst precursor (G6).

[A-27] The method according to [A-25], wherein the catalyst is a catalyst containing a palladium complex formed by a combination of a palladium compound selected from the group consisting of bis(allylchloropalladium(II)), tetrakis(triphenylphosphine)palladium(0), tris(dibenzylideneacetone)dipalladium(0)-chloroform adduct, a palladium(p-cinnamyl)chloride dimer, a (1-methylallyl)palladium chloride dimer, (1,5-cyclooctadiene)bis(trimethylsilylmethyl)palladium(II), a (2'-amino-1,1'-biphenyl-2-yl)methanesulfonatopalladium(II) dimer, and palladium(II) acetate, with a ligand selected from a ligand represented by a monodentate ligand of the following general formula (L1), (L3), or (L6): 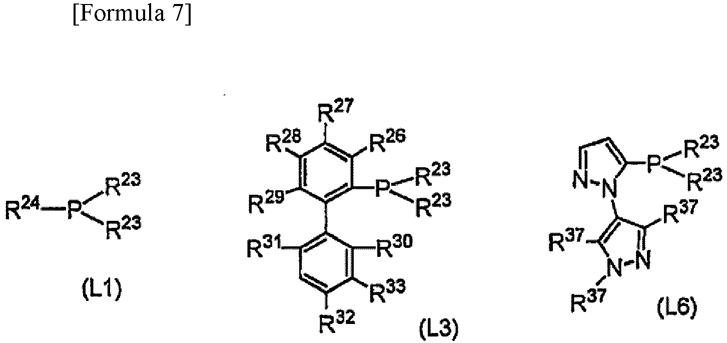
R²³ is independently tert-butyl, cyclohexyl, or adamantyl;
R²⁴ is C₁₋₆ alkyl, cyclohexyl, N-phenyl-2-indolyl, or adamantyl;
R²⁶, R²⁷, R²⁸, and R²⁹ are each independently a hydrogen atom, C₁₋₆ alkyl, C₁₋₆ alkoxy, or morpholino;
R³⁰, R³¹, and R³² are each independently a hydrogen atom, C₁₋₆ alkyl, C₁₋₆ alkoxy, or dimethylamino;
R³³ is a hydrogen atom or -SO₂-O-M, wherein M is lithium, sodium, or potassium;
R³⁷ is phenyl optionally substituted with C₁₋₆ alkyl, and a ligand being a salt thereof, and a palladium catalyst formed in combination with a compound represented by a salt thereof.

[A-28] The method according to any of [A-1] to [A-22], wherein the catalyst is a nickel catalyst.

[A-29] The method according to any of [A-1] to [A-22] and [A-28], wherein the catalyst is a catalyst containing a nickel complex formed by a combination of a nickel compound selected from the group consisting of bis(1,5-cyclooctadiene)nickel, dichloro(1,2-dimethoxyethane)nickel, dibromo(1,2-dimethoxyethane)nickel, nickel trifluoromethanesulfonate(II), bis(trifluoromethanesulfonimide)nickel(II), nickel(II) acetylacetonate, nickel(II) nitrate, nickel(II) bromide, nickel(II) chloride, and hydrates thereof, with a ligand selected from the group consisting tricyclohexylphosphine, 1,1'-bis(diphenylphosphino)ferocene, and 1,3-bis(diphenylphosphino)propane.

[A-30] The method according to any of [A-1] to [A-22], [A-28] and [A-29], wherein the catalyst is a catalyst containing a nickel complex selected from the group consisting of
dichlorobis(tricyclohexylphosphine)nickel(II),
dichloro[1,1'-bis(diphenylphosphino)ferrocene]nickel(II), and
dichloro[1,3-bis(diphenylphosphino)propane]nickel(II).

[A-31] The method according to any of [A-1] to [A-30], wherein the base includes at least one base selected from the group consisting of an organic base having a conjugate acid pKa of 23 or more in acetonitrile and an inorganic base having a conjugate acid pKa of 9 to 20 in water.

[A-32] The method according to any of [A-1] to [A-31], wherein the base is selected from the group consisting of amidines, guanidines, phosphazenes, carbonates of alkali metals, phosphates of alkali metals, C₁₋₆ alkoxides of alkali metals, and hydroxides of alkali metals.

[A-33] The method according to any of [A-1] to [A-32], wherein the base is selected from the group consisting of 2-tert-butyl-1,1,3,3-tetramethylguanidine (BTMG), 7-methyl-1,5,7-triazabicyclo[4.4.0]dec-5-ene (MTBD), 1-ethyl-2,2,4,4,4-pentakis(dimethylamino)-2l⁵,4l⁵-catenadi(phosphazene) (P2Et), 1-tert-butyl-2,2,4,4,4-pentakis(dimethylamino)-2l⁵,4⁵-catenadi(phosphazene) (P2tBu), tert-butylimino-tris(dimethylamino)phosphorane (P 1 tBu), 2-tert-butylimino-2-diethylamino-1,3-dimethylperhydro-1,3,2-diazaphosphorine (BEMP), tert-butylimino-tri(pyrrolidino)phosphorane (BTPP), carbonates of alkali metals, phosphates of alkali metals, C₁₋₆ alkoxides of alkali metals, and hydroxides of alkali metals.

[A-34] The method according to any of [A-1] to [A-33], wherein the base is selected from the group consisting of 2-tert-butyl-1,1,3,3-tetramethylguanidine (BTMG), 7-methyl-1,5,7-triazabicyclo[4.4.0]dec-5-ene (MTBD), 1-tert-butyl-2,2,4,4,4-pentakis(dimethylamino)-2l⁵,4l⁵-catenadi(phosphazene) (P2tBu), tert-butylimino-tris(dimethylamino)phosphorane (P1tBu), 2-tert-butylimino-2-diethylamino-1,3-dimethylperhydro-1,3,2-diazaphosphorine (BEMP), tert-butylimino-tri(pyrrolidino)phosphorane (BTPP), cesium carbonate, tripotassium phosphate, potassium hydroxide, and sodium tert-butoxide.

[A-35] The method according to any of [A-1] to [A-34], wherein a reaction system contains the base and further contains a salt.

[A-36] The method according to [A-35], wherein the salt is an alkali metal salt of an acid selected from the group consisting of trifluoroacetic acid, trifluoromethanesulfonic acid, trifluoromethanesulfonimide, tetrafluoroboric acid, hexafluorophosphoric acid, and hexafluoroantimonic(V) acid.

[A-37] The method according to [A-35], wherein the salt is sodium trifluoroacetate or potassium trifluoroacetate.

[A-38] The method according to any of [A-1] to [A-37], wherein the molar ratio of the base used to compound 1 or compound 2 is from 0.05 to 100, from 0.2 to 50, or from 1 to 30.

[A-39] The method according to any of [A-1] to [A-38], wherein a mixture containing two or more kinds of compound 1 is reacted.

[A-40] The method according to any of [A-1] to [A-39] for producing a compound constituting a compound library.

[A-41] The method according to any of [A-1] to [A-40], wherein two or more kinds of compound 1 are supported on a resin for solid-phase synthesis via a linker.

[A-42] The method according to any of [A-1] to [A-41], wherein three or more, four or more, five or more, seven or more, or ten or more kinds of compound 1 are supported on a resin for solid-phase synthesis via a linker.

[A-43] A method of producing a compound constituting a compound library, the method comprising producing the compound by the method according to any of [A-1] to [A-42].

[A-44] The method according to any of [A-1] to [A-43], wherein compound 1 is a resin for solid-phase synthesis, having a leaving group X¹ on a carbon atom of an aromatic ring in a side chain, or a resin for solid-phase synthesis, having a reactive group capable of a C-O bond formation reaction or a C-N bond formation reaction by substitution with the leaving group in a side chain.

[B-1] The method according to any of [A-1] to [A-44], wherein the cross-coupling reaction is a C-O bond formation reaction and compound 2 has a hydroxy capable of forming a C-O bond.

[B-2] The method according to [B-1], wherein compound 2 is water or a compound represented by HO-R⁵, wherein
R⁵ is C₁₋₆ alkyl, C₂₋₆ alkenyl, C₂₋₆ alkynyl, C₃₋₈ cycloalkyl, C₇₋₁₄ aralkyl, C₆₋₁₀ aryl, or 5- to 10-membered heteroaryl containing one or more ring heteroatoms independently selected from O, N, and S, each of which is optionally substituted with one or more groups independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, aminocarbonyl, (C₁₋₆ alkyl)aminocarbonyl, di(C₁₋₆ alkyl)aminocarbonyl, and 4- to 8-membered cyclic aminocarbonyl.

[B-3] The method according to [B-2], wherein compound 2 is supported on a resin for solid-phase synthesis and includes the compound represented by HO-R⁵ as a part of a chemical structure.

[B-4] The method according to [B-2] or [B-3], wherein compound 2 is water.

[B-5] The method according to any of [B-1] to [B-4], wherein a product of the coupling reaction is a compound represented by Ar²-OH or Ar²-OR⁵, or a compound including a compound represented by Ar²-OH or Ar²-OR⁵ as a part of a chemical structure, wherein Ar² and R⁵ are as already defined.

[C-1] The method according to any of [A-1] to [A-44], wherein the cross-coupling reaction is a C-N bond formation reaction and compound 2 has an H-N group capable of forming a C-N bond.

[C-2] The method according to [C-1], wherein compound 2 is a compound represented by HNR⁶R⁷, wherein
R⁶ and R⁷ together with the nitrogen atom to which they are bonded form a 5- to 7-membered saturated heterocycle, wherein the heterocycle is optionally substituted with one or more substituents independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl, or
R⁶ and R⁷ are each independently a hydrogen atom, C₁₋₆ alkyl, C₂₋₆ alkenyl, C₂₋₆ alkynyl, C₃₋₈ cycloalkyl, (C₁₋₆ alkyl)carbonyl, (C₆₋₁₀ aryl)carbonyl, 5- to 10-membered heteroarylcarbonyl containing one or more ring heteroatoms independently selected from O, N and S, C₇₋₁₄ aralkyl, C₆₋₁₀ aryl, or 5- to 10-membered heteroaryl containing one or more ring heteroatoms independently selected from O, N and S, each of which is optionally substituted with one or more substituents independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl.

[C-3] The method according to [C-1] or [C-2], wherein compound 2 is supported on a resin for solid-phase synthesis and includes the compound represented by HNR⁶R⁷ as a part of a chemical structure.

[C-4] The method according to any of [C-1] to [C-3], wherein a product of the coupling reaction is a compound represented by Ar²-NR⁶R⁷ or a compound including a compound represented by Ar²-NR⁶R⁷ as a part of a chemical structure, wherein Ar², R⁶ and R⁷ are as already defined.

[D-1] A method of producing a compound, comprising the method according to any of [A-1] to [A-44], [B-1] to [B-5], [C-1] to [C-4], and [H-1] to [H-44].

[E-1] Use of a solvent in a cross-coupling reaction, the solvent containing an amide-based solvent represented by formula A: 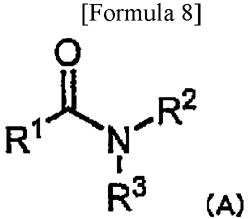 wherein R¹, R² and R³ are each independently C₁₋₄ alkyl provided that the sum of carbon atoms of R¹, R² and R³ is 4 or more and 6 or less.

[E-2] The use according to [E-1], wherein the solvent is used in the method according to any of [A-1] to [A-44], [B-1] to [B-4], and [C-1] to [C-3].

[F-1] A method of producing a compound by a cross-coupling reaction in the presence of a palladium catalyst, the method comprising:
performing a cross-coupling reaction, in the presence of a palladium catalyst and a base, in a solvent containing an amide-based solvent represented by formula A: 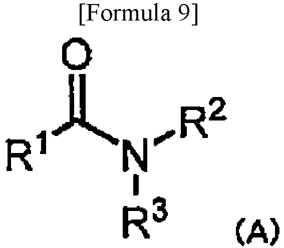
wherein R¹, R² and R³ are each independently C₁₋₄ alkyl provided that the sum of carbon atoms of R¹, R² and R³ is 4 or more and 6 or less, wherein the palladium catalyst is a palladium complex containing a phosphine ligand.

[F-2] The method according to [F-1], wherein the solvent is selected from the group consisting of N,N-dimethylpropionamide (DMPr), N,N-diethylacetamide (DEAc) and N,N-diethylpropionamide (DEPr).

[F-3] The method according to [F-1] or [F-2], wherein the solvent is N,N-dimethylpropionamide (DMPr).

[F-4] The method according to any of [F-1] to [F-3], wherein the solvent is a solvent containing at least one selected from the group consisting of N,N-dimethylpropionamide (DMPr), N,N-diethylacetamide (DEAc) and N,N-diethylpropionamide (DEPr) at 30 v/v% or more, 50 v/v% or more, 70 v/v% or more, or 90 v/v% or more.

[F-5] The method according to any of [F-1] to [F-4], wherein the solvent is a solvent containing N,N-dimethylpropionamide (DMPr) at 30 v/v% or more, 50 v/v% or more, 70 v/v% or more, or 90 v/v% or more.

[F-6] The method according to any of [F-1] to [F-5], wherein the cross-coupling reaction is performed at 0 to 200°C, 0 to 150°C, 0 to 100°C, 0 to 80°C, or 25 to 80°C.

[F-7] The method according to any of [F-1] to [F-6], wherein the catalyst is a catalyst containing a palladium complex represented by any of the general formulae (Cat1), (Cat2), (Cat3), (Cat4) and (Cat5) described in [A-23].

[F-8] The method according to any of [F-1] to [F-7], wherein the catalyst is a catalyst containing a palladium catalyst represented by any of the general formulae (Cat6) and (Cat7) described in [A-24].

[F-9] The method according to any of [F-1] to [F-8], wherein the catalyst is a catalyst containing a palladium complex formed by a combination of at least one selected from the group consisting of bis(allylchloropalladium(II)), tetrakis(triphenylphosphine)palladium(0), tris(dibenzylideneacetone)dipalladium(0)-chloroform adduct, a palladium(p-cinnamyl)chloride dimer, a (1-methylallyl)palladium chloride dimer, (1,5-cyclooctadiene)bis(trimethylsilylmethyl)palladium(II), a (2'-amino-1,1'-biphenyl-2-yl)methanesulfonatopalladium(II) dimer, and palladium(II) acetate, with a ligand selected from a monodentate ligand of the following general formula (L1), (L2), (L3), (L4), (L5), (L6) or (L7), or a bidentate ligand of the following general formula (L8), (L9), (L10), (L11) or (L12) described in [A-25], or a salt thereof.

[F-10] The method according to any of [F-1] to [F-9], wherein the catalyst is a catalyst containing a palladium complex selected from the group consisting of a Buchwald first-generation catalyst precursor (G1), a Buchwald second-generation catalyst precursor (G2), a Buchwald third-generation catalyst precursor (G3), a Buchwald fourth-generation catalyst precursor (G4), a Buchwald fifth-generation catalyst precursor (G5), or a Buchwald sixth-generation catalyst precursor (G6).

[F-1 1] The method according to any of [F-1] to [F-8], wherein the catalyst is a catalyst containing a palladium complex formed by a combination of a palladium compound selected from the group consisting of bis(allylchloropalladium(II)), tetrakis(triphenylphosphine)palladium(0), tris(dibenzylideneacetone)dipalladium(0)-chloroform adduct, a palladium(p-cinnamyl)chloride dimer, a (1-methylallyl)palladium chloride dimer, (1,5-cyclooctadiene)bis(trimethylsilylmethyl)palladium(II), a (2'-amino-1,1'-biphenyl-2-yl)methanesulfonatopalladium(II) dimer, and palladium(II) acetate, with a ligand selected from a ligand represented by a monodentate ligand of the following general formula (L1), (L3), or (L6) described in [A-27], and a ligand being a salt thereof.

[F-12] The method according to any of [F-1] to [F-11], wherein the base includes at least one base selected from the group consisting of an organic base having a conjugate acid pKa of 23 or more in acetonitrile and an inorganic base having a conjugate acid pKa of 9 to 20 in water.

[F-13] The method according to any of [F-1] to [F-12], wherein the base is selected from the group consisting of amidines, guanidines, phosphazenes, carbonates of alkali metals, phosphates of alkali metals, C₁₋₆ alkoxides of alkali metals, and hydroxides of alkali metals.

[F-14] The method according to any of [F-1] to [F-13], wherein the base is selected from the group consisting of 2-tert-butyl-1,1,3,3-tetramethylguanidine (BTMG), 7-methyl-1,5,7-triazabicyclo[4.4.0]dec-5-ene (MTBD), 1-ethyl-2,2,4,4,4-pentakis(dimethylamino)-2l⁵,4l⁵-catenadi(phosphazene) (P2Et), 1-tert-butyl-2,2,4,4,4-pentakis(dimethylamino)-2l⁵,4⁵-catenadi(phosphazene) (P2tBu), tert-butylimino-tris(dimethylamino)phosphorane (P 1 tBu), 2-tert-butylimino-2-diethylamino-1,3-dimethylperhydro-1,3,2-diazaphosphorine (BEMP), tert-butylimino-tri(pyrrolidino)phosphorane (BTPP), carbonates of alkali metals, phosphates of alkali metals, C₁₋₆ alkoxides of alkali metals, and hydroxides of alkali metals.

[F-15] The method according to any of [F-1] to [F-14], wherein the base is selected from the group consisting of 2-tert-butyl-1,1,3,3-tetramethylguanidine (BTMG), 7-methyl-1,5,7-triazabicyclo[4.4.0]dec-5-ene (MTBD), 1-tert-butyl-2,2,4,4,4-pentakis(dimethylamino)-2l⁵,4l⁵-catenadi(phosphazene) (P2tBu), tert-butylimino-tris(dimethylamino)phosphorane (P1tBu), 2-tert-butylimino-2-diethylamino-1,3-dimethylperhydro-1,3,2-diazaphosphorine (BEMP), tert-butylimino-tri(pyrrolidino)phosphorane (BTPP), cesium carbonate, tripotassium phosphate, potassium hydroxide, and sodium tert-butoxide.

[F-16] The method according to any of [F-1] to [F-15], wherein a reaction system contains the base and further contains a salt.

[F-17] The method according to [F-16], wherein the salt is an alkali metal salt of an acid selected from the group consisting of trifluoroacetic acid, trifluoromethanesulfonic acid, trifluoromethanesulfonimide, tetrafluoroboric acid, hexafluorophosphoric acid, and hexafluoroantimonic(V) acid.

[F-18] The method according to [F-17], wherein the salt is sodium trifluoroacetate or potassium trifluoroacetate.

[F-19] The method according to any of [F-1] to [F-18], wherein the cross-coupling reaction is a C-O bond formation reaction using compound 1 having a leaving group and compound 2 having a hydroxy as substrates and accompanied by a removal of the leaving group.

[F-20] The method according to [F-19], wherein compound 1 is represented by X¹-Ar², wherein X¹ and Ar² are as already defined.

[F-21] The method according to [F-19] or [F-20], wherein compound 2 is water or represented by HO-R⁵, wherein R⁵ is as already defined.

[F-22] The method according to [F-19] or [F-20], wherein compound 2 is supported on a resin for solid-phase synthesis and includes the compound represented by HO-R⁵ as a part of a chemical structure, wherein R⁵ is as already defined.

[F-23] The method according to any of [F-19] to [F-22], wherein a product of the coupling reaction is a compound represented by Ar²-OH or Ar²-O-R⁵, or a compound including a compound represented by Ar²-OH or Ar²-O-R⁵ as a part of a chemical structure, wherein Ar² and R⁵ are as already defined.

[F-24] The method according to any of [F-1] to [F-18], wherein the cross-coupling reaction is a C-N bond formation reaction using compound 1 having a leaving group and compound 2 having H-N as substrates and accompanied by a removal of the leaving group.

[F-25] The method according to [F-24], wherein compound 1 is represented by X¹-Ar², wherein X¹ and Ar² are as already defined.

[F-26] The method according to [F-24] or [F-25], wherein compound 2 is represented by HNR⁶R⁷, wherein R⁶ and R⁷ are as already defined.

[F-27] The method according to [F-24] or [F-25], wherein compound 2 is supported on a resin for solid-phase synthesis and includes a compound represented by HNR⁶R⁷ as a part of a chemical structure, wherein R⁶ and R⁷ are as already defined.

[F-28] The method according to any of [F-24] to [F-27], wherein a product of the coupling reaction is a compound represented by Ar²-NR⁶R⁷, or a compound including a compound represented by Ar²-NR⁶R⁷ as a part of a chemical structure, wherein Ar², R⁶ and R⁷ are as already defined.

[F-29] The method according to [F-19] or [F-28], wherein compound 1 is supported on a resin for solid-phase synthesis and includes the compound represented by X¹-Ar² as a part of a chemical structure, wherein X¹ and Ar² are as already defined.

[G-1] A method of suppressing a generation of a by-product in a cross-coupling reaction, the method comprising:
performing a cross-coupling reaction, in the presence of a catalyst and a base, in a solvent containing an amide-based solvent represented by formula A: 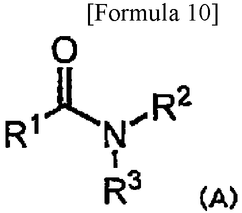
wherein R¹, R² and R³ are each independently C₁₋₄ alkyl provided that the sum of carbon atoms of R¹, R² and R³ is 4 or more and 6 or less, wherein
the cross-coupling reaction is a substitution reaction of a leaving group X¹ in compound 1 having the leaving group on a carbon atom of an aromatic ring, wherein the by-product is a compound in which the leaving group is substituted with a hydrogen atom.

[G-2] The method according to [G-1], wherein the catalyst is a palladium catalyst or a nickel catalyst.

[G-3] The method according to [G-1] or [G-2], wherein the catalyst is a palladium catalyst.

[G-4] The method according to any of [G-1] to [G-3], wherein the number of the leaving group in compound 1 is one, or two or three where the leaving groups may be the same or different.

[G-5] The method according to any of [G-1] to [G-4], wherein the number of the leaving group in compound 1 is one.

[G-6] The method according to any of [G-1] to [G-5], wherein the number of the reactive group of compound 2 is one, or two or three where the reactive groups may be the same or different.

[G-7] The method according to any of [G-1] to [G-6], wherein the number of the reactive group of compound 2 is one.

[G-8] The method according to any of [G-1] to [G-7], wherein compound 1 or compound 2 is supported on a resin for solid-phase synthesis.

[G-9] The method according to any of [G-1] to [G-8], wherein compound 2 is
1) water or a compound having a hydroxy capable of forming a C-O bond, represented by HO-R⁵, wherein
   R⁵ is C₁₋₆ alkyl, C₂₋₆ alkenyl, C₂₋₆ alkynyl, C₃₋₈ cycloalkyl, C₇₋₁₄ aralkyl, C₆₋₁₀ aryl, or 5- to 10-membered heteroaryl containing one or more ring heteroatoms independently selected from O, N and S, each of which is optionally substituted with one or more groups independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, aminocarbonyl, (C₁₋₆ alkyl)aminocarbonyl, di(C₁₋₆ alkyl)aminocarbonyl, and 4- to 8-membered cyclic aminocarbonyl; or
2) a compound having an H-N group capable of forming a C-N bond, represented by HNR⁶R⁷, wherein
   R⁶ and R⁷ together with the nitrogen atom to which they are bonded form a 5- to 7-membered saturated heterocycle, wherein the heterocycle is optionally substituted with one or more substituents independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl, or
   R⁶ and R⁷ are each independently a hydrogen atom, C₁₋₆ alkyl, C₂₋₆ alkenyl, C₂₋₆ alkynyl, C₃₋₈ cycloalkyl, (C₁₋₆ alkyl)carbonyl, (C₆₋₁₀ aryl)carbonyl, 5- to 10-membered heteroarylcarbonyl containing one or more ring heteroatoms independently selected from O, N and S, C₇₋₁₄ aralkyl, C₆₋₁₀ aryl, or 5- to 10-membered heteroaryl containing one or more ring heteroatoms independently selected from O, N and S, each of which is optionally substituted with one or more substituents independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl.

[G-10] The method according to any of [G-1] to [G-9], wherein compound 1 is a compound represented by X¹-Ar², wherein
X¹ is a chlorine atom, a bromine atom, an iodine atom, or -O-SO₂-R⁴;
R⁴ is C₁₋₆ alkyl optionally substituted with one or more fluorine atoms, or phenyl optionally substituted with one or more fluorine atoms or C₁₋₆ alkyl optionally substituted with a fluorine atom;
Ar² is C₆₋₁₀ aryl, or 5- to 10-membered heteroaryl containing one or more ring heteroatoms independently selected from O, N and S, each of which is optionally substituted with one or more groups independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, (C₁₋₆ alkyl)aminocarbonyl, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl; and
the by-product is a compound represented by H-Ar².

[G-11] The method according to any of [G-1] to [G-10], wherein compound 1 is supported on a resin for solid-phase synthesis and includes the compound represented by X¹-Ar² described in [G-10] as a part of a chemical structure.

[G-12] The method according to [G-10] or [G-11], wherein X¹ is a chlorine atom, a bromine atom, or an iodine atom.

[G-13] The method according to any of [G-10] to [G-12], wherein Ar² is independently selected from the group consisting of phenyl, naphthyl, pyrrolyl, thienyl, furyl, pyridyl, thiazolyl, isothiazolyl, pyrazolyl, oxazolyl, isoxazolyl, imidazolyl, triallyl, pyrimidyl, pyrazinyl, pyridazinyl, quinolinyl, isoquinolinyl, 4H-quinolidinyl, phthalazinyl, naphthyridinyl, quinoxalinyl, quinazolinyl, cinnolinyl, pteridinyl, indolyl, indolinyl, benzothiophenyl, benzofuranyl, benzisothiazolyl, benzisoxazolyl, indazolyl, benzimidazolyl, benzotriazolyl, azaindolyl, and imidazopyridyl, each of which is optionally substituted.

[G-14] The method according to any of [G-10] to [G-13], wherein Ar² is phenyl or pyridyl, each of which is optionally substituted.

[G-15] The method according to any of [G-10] to [G-14], wherein X¹ is a bromine atom; and
Ar² is phenyl or pyridyl, each of which is optionally substituted with one or more groups independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, (C₁₋₆ alkyl)aminocarbonyl, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl.

[G-16] The method according to any of [G-1] to [G-15], wherein the solvent is selected from the group consisting of N,N-dimethylpropionamide (DMPr), N,N-diethylacetamide (DEAc) and N,N-diethylpropionamide (DEPr).

[G-17] The method according to any of [G-1] to [G-16], wherein the solvent is N,N-dimethylpropionamide (DMPr).

[G-18] The method according to any of [G-1] to [G-17], wherein the solvent is a solvent containing at least one selected from the group consisting of N,N-dimethylpropionamide (DMPr), N,N-diethylacetamide (DEAc) and N,N-diethylpropionamide (DEPr) at 30 v/v% or more, 50 v/v% or more, 70 v/v% or more, or 90 v/v% or more.

[G-19] The method according to any of [G-1] to [G-18], wherein the solvent is a solvent containing N,N-dimethylpropionamide (DMPr) at 30 v/v% or more, 50 v/v% or more, 70 v/v% or more, or 90 v/v% or more.

[G-20] The method according to any of [G-1] to [G-19], wherein the cross-coupling reaction is performed at 0 to 200°C, 0 to 150°C, 0 to 100°C, 10 to 80°C, or 25 to 80°C.

[G-21] The method according to any of [G-1] to [G-20], wherein the molar ratio of compound 1 to compound 2 used is compound 1/compound 2 = from 0.0005 to 500, from 0.005 to 200, or from 0.05 to 20.

[G-22] The method according to any of [G-1] to [G-21], wherein the molar ratio of the catalyst used is from 0.01 to 100 mol%, from 0.1 to 50 mol%, from 1 to 25 mol% relative to compound 1 or compound 2.

[G-23] The method according to any of [G-1] to [G-22], wherein the catalyst is a catalyst containing a palladium complex represented by any of the following general formulae (Cat1), (Cat2), (Cat3), (Cat4) and (Cat5): 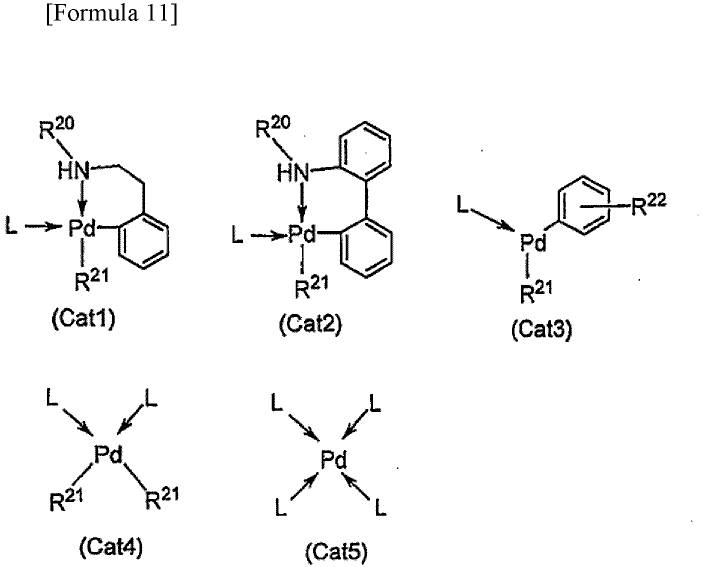 wherein R²⁰ is a hydrogen atom, C₁₋₆ alkyl, or C₆₋₁₀ aryl; R²¹ is halogen or -O-SO₂-CH₃; R²² is a hydrogen atom, C₁₋₆ alkyl optionally substituted with one or more fluorine atoms, or (C₁₋₆ alkoxy)carbonyl optionally substituted with triC₁₋₆ alkylsilyl; L is independently a monodentate ligand of the following general formula (L1), (L2), (L3), (L4), (L5), (L6) or (L7), or two L are a bidentate ligand of the following general formula (L8), (L9), (L10), (L11) or (L12): 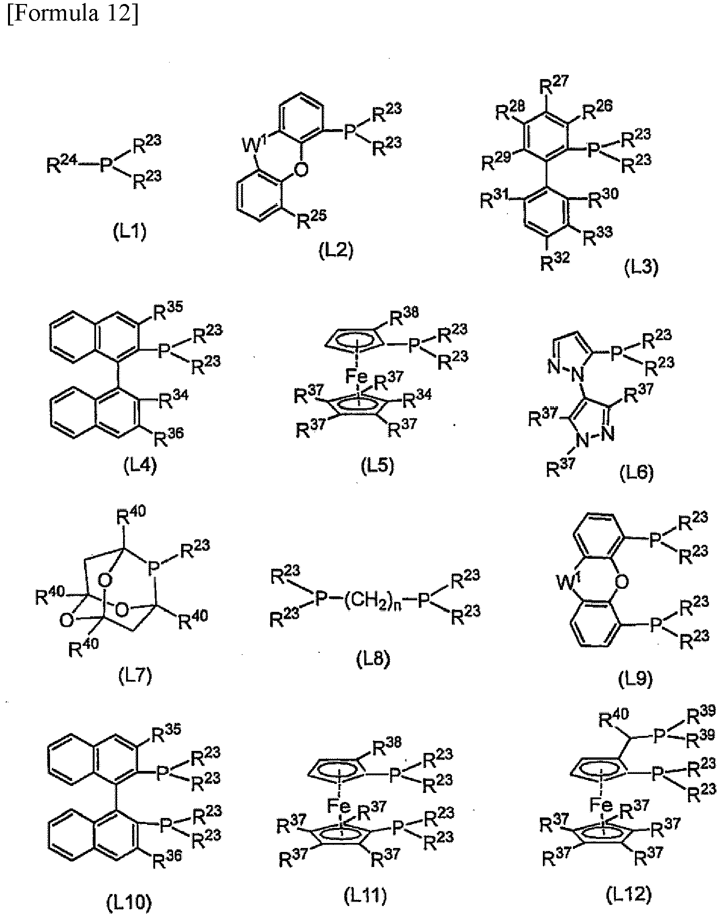
wherein R²³ is independently tert-butyl, cyclohexyl, 2-furanyl, 2-thienyl, 2-pyridyl, phenyl, or adamantyl, wherein the phenyl is optionally substituted with one or more fluorine atoms, C₁₋₆ alkyl optionally substituted with a fluorine atom, C₁₋₆ alkoxy, or dimethylamino;
R²⁴ is C₁₋₆ alkyl, cyclohexyl, 2-furanyl, 2-thienyl, 2-pyridyl, N-phenyl-2-pyrrolyl, N-phenyl-2-indolyl, phenyl, or adamantyl, wherein the phenyl is optionally substituted with one or more fluorine atoms, C₁₋₆ alkyl optionally substituted with a fluorine atom, C₁₋₆ alkoxy, morpholino, or dimethylamino;
R²⁵ is a hydrogen atom, C₁₋₆ alkyl, or C₁₋₆ alkoxy;
W¹ is -C(CH₃)₂-, or -NH-;
R²⁶, R²⁷, R²⁸, and R²⁹ are each independently a hydrogen atom, C₁₋₆ alkyl, C₁₋₆ alkoxy, or morpholino;
R³⁰, R³¹, and R³² are each independently a hydrogen atom, C₁₋₆ alkyl, C₁₋₆ alkoxy, or dimethylamino;
R³³ is a hydrogen atom or -SO₂-O-M, wherein M is lithium, sodium, or potassium;
R³⁴ is a hydrogen atom, C₁₋₆ alkyl, or C₁₋₆ alkoxy;
R³⁵ and R³⁶ are each independently a hydrogen atom, C₁₋₆ alkyl, or C₁₋₆ alkoxy;
R³⁷ is a hydrogen atom, or phenyl optionally substituted with C₁₋₆ alkyl;
R³⁸ is independently a hydrogen atom, phenyl optionally substituted with C₁₋₆ alkyl, or -CH(CH₃)-N(CH₃)₂;
R³⁹ is tert-butyl, cyclohexyl, or adamantyl;
R⁴⁰ is a hydrogen atom or C₁₋₆ alkyl; and an arrow represents a coordination bond.

[G-24] The method according to any of [G-1] to [G-22], wherein the catalyst is a catalyst containing a palladium complex represented by any of the following general formulae (Cat6) and (Cat7): 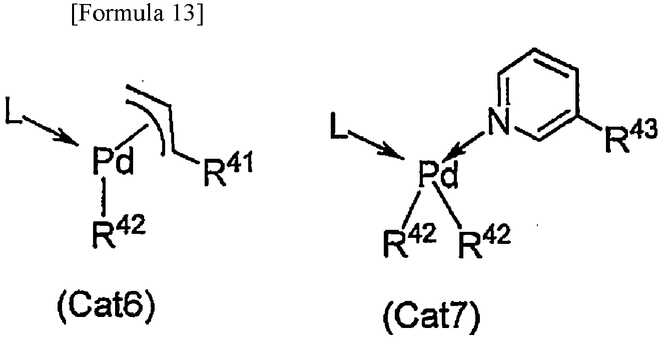 wherein R⁴¹ is a hydrogen atom or phenyl optionally substituted with C₁₋₆ alkyl; R⁴² is independently halogen; R⁴³ is a fluorine atom or a chlorine atom; and L is an N-heterocyclic carbene ligand represented by the following general formula (L12) or (L13): 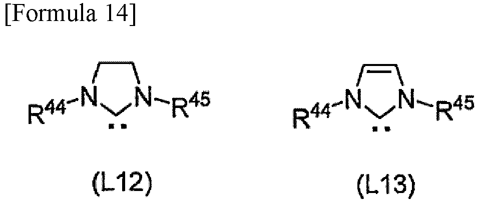 wherein R⁴⁴ and R⁴⁵ each independently represent C₁₋₆ alkyl, cyclohexyl, adamantyl, or phenyl, wherein the phenyl is optionally substituted with one or more C₁₋₆ alkyl, C₁₋₆ alkoxy, or dimethylamino, and a carbon atom with × × represents a carbene, and an arrow represents a coordination bond.

[G-25] The method according to any of [G-1] to [G-22], wherein the catalyst is a catalyst containing a palladium complex formed by a combination of a palladium compound selected from the group consisting of bis(allylchloropalladium(II)), tetrakis(triphenylphosphine)palladium(0), tris(dibenzylideneacetone)dipalladium(0)-chloroform adduct, a palladium(p-cinnamyl)chloride dimer, a (1-methylallyl)palladium chloride dimer, (1,5-cyclooctadiene)bis(trimethylsilylmethyl)palladium(II), a (2'-amino-1,1'-biphenyl-2-yl)methanesulfonatopalladium(II) dimer, and palladium(II) acetate, with a ligand selected from a ligand represented by a monodentate ligand of the following general formula (L1), (L2), (L3), (L4), (L5), (L6) or (L7), or a bidentate ligand of the following general formula (L8), (L9), (L10), (L11) or (L12): 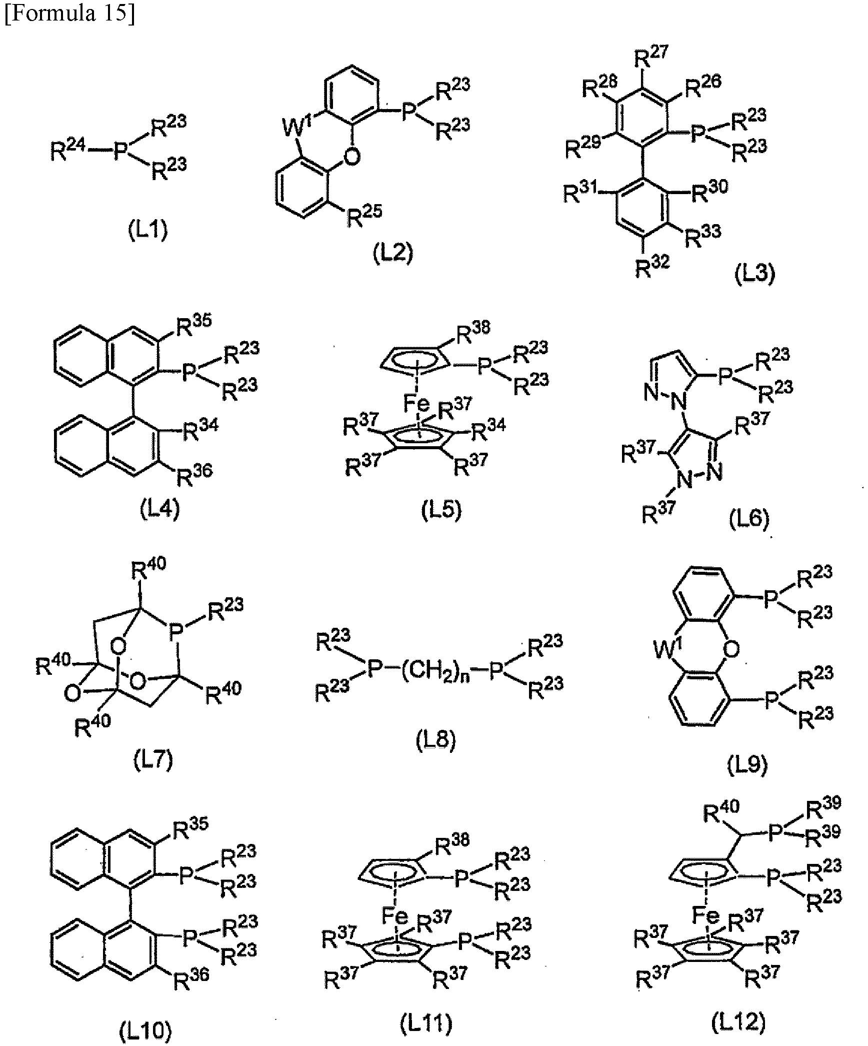
wherein R²³ is tert-butyl, cyclohexyl, 2-furanyl, 2-thienyl, 2-pyridyl, phenyl, or adamantyl, wherein the phenyl is optionally substituted with one or more fluorine atoms, C₁₋₆ alkyl optionally substituted with a fluorine atom, C₁₋₆ alkoxy, or dimethylamino;
R²⁴ is C₁₋₆ alkyl, cyclohexyl, 2-furanyl, 2-thienyl, 2-pyridyl, N-phenyl-2-pyrrolyl, N-phenyl-2-indolyl, phenyl, or adamantyl, wherein the phenyl is optionally substituted with one or more fluorine atoms, C₁₋₆ alkyl optionally substituted with a fluorine atom, C₁₋₆ alkoxy, morpholino, or dimethylamino;
R²⁵ is a hydrogen atom, C₁₋₆ alkyl, or C₁₋₆ alkoxy;
W¹ is -C(CH₃)₂-, or -NH-;
R²⁶, R²⁷, R²⁸, and R²⁹ are each independently a hydrogen atom, C₁₋₆ alkyl, C₁₋₆ alkoxy, or morpholino;
R³⁰, R³¹, and R³² are each independently a hydrogen atom, C₁₋₆ alkyl, C₁₋₆ alkoxy, or dimethylamino;
R³³ is a hydrogen atom or -SO₂-O-M, wherein M is lithium, sodium, or potassium;
R³⁴ is a hydrogen atom, C₁₋₆ alkyl, or C₁₋₆ alkoxy;
R³⁵ and R³⁶ are each independently a hydrogen atom, C₁₋₆ alkyl, or C₁₋₆ alkoxy;
R³⁷ is a hydrogen atom, or phenyl optionally substituted with C₁₋₆ alkyl;
R³⁸ is a hydrogen atom, phenyl optionally substituted with C₁₋₆ alkyl, or -CH(CH₃)-N(CH₃)₂;
R³⁹ is tert-butyl, cyclohexyl, or adamantyl;
R⁴⁰ is a hydrogen atom or C₁₋₆ alkyl; and an arrow represents a coordination bond, or a ligand being a salt thereof.

[G-26] The method according to any of [G-1] to [G-23], wherein the catalyst is a catalyst containing a palladium complex selected from a Buchwald first-generation catalyst precursor (G1), a Buchwald second-generation catalyst precursor (G2), a Buchwald third-generation catalyst precursor (G3), a Buchwald fourth-generation catalyst precursor (G4), a Buchwald fifth-generation catalyst precursor (G5), or a Buchwald sixth-generation catalyst precursor (G6).

[G-27] The method according to [G-25], wherein the catalyst is a catalyst containing a palladium complex formed by a combination of a palladium compound selected from the group consisting of bis(allylchloropalladium(II)), tetrakis(triphenylphosphine)palladium(0), tris(dibenzylideneacetone)dipalladium(0)-chloroform adduct, a palladium(p-cinnamyl)chloride dimer, a (1-methylallyl)palladium chloride dimer, (1,5-cyclooctadiene)bis(trimethylsilylmethyl)palladium(II), a (2'-amino-1,1'-biphenyl-2-yl)methanesulfonatopalladium(II) dimer, and palladium(II) acetate, with a ligand selected from a ligand represented by a monodentate ligand of the following general formula (L1), (L3), or (L6): 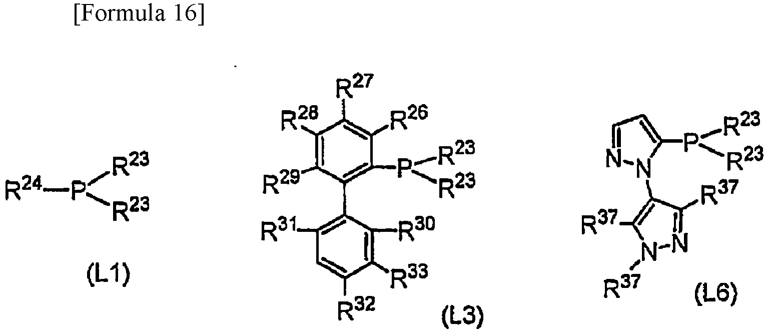
R²³ is independently tert-butyl, cyclohexyl, or adamantyl;
R²⁴ is C₁₋₆ alkyl, cyclohexyl, N-phenyl-2-indolyl, or adamantyl;
R²⁶, R²⁷, R²⁸, and R²⁹ are each independently a hydrogen atom, C₁₋₆ alkyl, C₁₋₆ alkoxy, or morpholino;
R³⁰, R³¹, and R³² are each independently a hydrogen atom, C₁₋₆ alkyl, C₁₋₆ alkoxy, or dimethylamino;
R³³ is a hydrogen atom or -SO₂-O-M, wherein M is lithium, sodium, or potassium;
R³⁷ is phenyl optionally substituted with C₁₋₆ alkyl, and a ligand being a salt thereof, and a palladium catalyst formed in combination with a compound represented by a salt thereof.

[G-28] The method according to any of [G-1] to [G-22], wherein the catalyst is a nickel catalyst.

[G-29] The method according to any of [G-1] to [G-22] and [G-28], wherein the catalyst is a catalyst containing a nickel complex formed by a combination of a nickel compound selected from the group consisting of bis(1,5-cyclooctadiene)nickel, dichloro(1,2-dimethoxyethane)nickel, dibromo(1,2-dimethoxyethane)nickel, nickel trifluoromethanesulfonate(II), bis(trifluoromethanesulfonimide)nickel(II), nickel(II) acetylacetonate, nickel(II) nitrate, nickel(II) bromide, nickel(II) chloride, and hydrates thereof, with a ligand selected from the group consisting tricyclohexylphosphine, 1,1'-bis(diphenylphosphino)ferocene, and 1,3-bis(diphenylphosphino)propane.

[G-30] The method according to any of [G-1] to [G-22], [G-28] and [G-29], wherein the catalyst is a catalyst containing a nickel complex selected from the group consisting of
dichlorobis(tricyclohexylphosphine)nickel(II),
dichloro[1,1'-bis(diphenylphosphino)ferrocene]nickel(II), and
dichloro[1,3-bis(diphenylphosphino)propane]nickel(II).

[G-31] The method according to any of [G-1] to [G-30], wherein the base includes at least one base selected from the group consisting of an organic base having a conjugate acid pKa of 23 or more in acetonitrile and an inorganic base having a conjugate acid pKa of 9 to 20 in water.

[G-32] The method according to any of [G-1] to [G-31], wherein the base is selected from the group consisting of amidines, guanidines, phosphazenes, carbonates of alkali metals, phosphates of alkali metals, C₁₋₆ alkoxides of alkali metals, and hydroxides of alkali metals.

[G-33] The method according to any of [G-1] to [G-32], wherein the base is selected from the group consisting of 2-tert-butyl-1,1,3,3-tetramethylguanidine (BTMG), 7-methyl-1,5,7-triazabicyclo[4.4.0]dec-5-ene (MTBD), 1-ethyl-2,2,4,4,4-pentakis(dimethylamino)-2l⁵,4l⁵-catenadi(phosphazene) (P2Et), 1-tert-butyl-2,2,4,4,4-pentakis(dimethylamino)-2l⁵,4⁵-catenadi(phosphazene) (P2tBu), tert-butylimino-tris(dimethylamino)phosphorane (P 1 tBu), 2-tert-butylimino-2-diethylamino-1,3-dimethylperhydro-1,3,2-diazaphosphorine (BEMP), tert-butylimino-tri(pyrrolidino)phosphorane (BTPP), carbonates of alkali metals, phosphates of alkali metals, C₁₋₆ alkoxides of alkali metals, and hydroxides of alkali metals.

[G-34] The method according to any of [G-1] to [G-33], wherein the base is selected from the group consisting of 2-tert-butyl-1,1,3,3-tetramethylguanidine (BTMG), 7-methyl-1,5,7-triazabicyclo[4.4.0]dec-5-ene (MTBD), 1-tert-butyl-2,2,4,4,4-pentakis(dimethylamino)-2l⁵,4l⁵-catenadi(phosphazene) (P2tBu), tert-butylimino-tris(dimethylamino)phosphorane (P1tBu), 2-tert-butylimino-2-diethylamino-1,3-dimethylperhydro-1,3,2-diazaphosphorine (BEMP), tert-butylimino-tri(pyrrolidino)phosphorane (BTPP), cesium carbonate, tripotassium phosphate, potassium hydroxide, and sodium tert-butoxide.

[G-35] The method according to any of [G-1] to [G-34], wherein a reaction system contains the base and further contains a salt.

[G-36] The method according to [G-35], wherein the salt is an alkali metal salt of an acid selected from the group consisting of trifluoroacetic acid, trifluoromethanesulfonic acid, trifluoromethanesulfonimide, tetrafluoroboric acid, hexafluorophosphoric acid, and hexafluoroantimonic(V) acid.

[G-37] The method according to [G-35], wherein the salt is sodium trifluoroacetate or potassium trifluoroacetate.

[G-38] The method according to any of [G-1] to [G-37], wherein the molar ratio of the base used to compound 1 or compound 2 is from 0.05 to 100, from 0.2 to 50, or from 1 to 30.

[G-39] The method according to any of [G-1] to [G-38], wherein compound 2 is a compound as defined in any of [B-1] to [B-4] and [C-1] to [C-3].

[G-40] The method according to any of [G-1] to [G-38], wherein a product of the coupling reaction is a compound as defined in any of [B-5] and [C-4].

[H-1] A method of producing a compound by a cross-coupling reaction, the method comprising:
reacting compound 1 having a leaving group X¹ on a carbon atom of an aromatic ring with compound 2 having a reactive group capable of a C-O bond formation reaction or a C-N bond formation reaction by substitution with the leaving group, in the presence of a catalyst and a base, in a solvent containing an amide-based solvent represented by formula A: 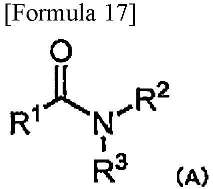
wherein R¹, R², and R³ are each independently C₁₋₄ alkyl provided that the sum of carbon atoms of R¹, R², and R³ is 4 or more and 6 or less, wherein
X¹ is a halogen atom or -O-SO₂-R⁴;
R⁴ is C₁₋₆ alkyl optionally substituted with one or more fluorine atoms, or phenyl optionally substituted with one or more fluorine atoms or C₁₋₆ alkyl optionally substituted with a fluorine atom;
compound 2 has a hydroxy capable of forming a C-O bond or an H-N group capable of forming a C-N bond; and
compound 1 is a resin for solid-phase synthesis, having a leaving group X¹ on a carbon atom of an aromatic ring in a side chain, or a resin for solid-phase synthesis, having a reactive group capable of a C-O bond formation reaction or a C-N bond formation reaction by substitution with the leaving group in a side chain.

[H-2] The method according to [H-1], wherein the catalyst is a palladium catalyst or a nickel catalyst.

[H-3] The method according to [H-1] or [H-2], wherein the catalyst is a palladium catalyst.

[H-4] The method according to any of [H-1] to [H-3], wherein the number of the leaving group in compound 1 is one, or two or three where the leaving groups may be the same or different.

[H-5] The method according to any of [H-1] to [H-4], wherein the number of the leaving group in compound 1 is one.

[H-6] The method according to any of [H-1] to [H-5], wherein the number of the reactive group of compound 2 is one, or two or three where the reactive groups may be the same or different.

[H-7] The method according to any of [H-1] to [H-6], wherein the number of the reactive group of compound 2 is one.

[H-8] The method according to any of [H-1] to [H-7], wherein a side chain of the resin for solid-phase synthesis includes a degradable linker; and
the method further comprises cleaving a compound containing a C-O bond or C-N bond formed by the cross-coupling reaction from the resin for solid phase synthesis by degrading the linker after the cross-coupling reaction.

[H-9] The method according to any of [H-1] to [H-8], wherein compound 2 is
1) a resin for solid phase synthesis having a side chain to which a compound having a hydroxy capable of forming a C-O bond, represented by HO-R⁵, is bound via a linker, wherein
   R⁵ is C₁₋₆ alkyl, C₂₋₆ alkenyl, C₂₋₆ alkynyl, C₃₋₈ cycloalkyl, C₇₋₁₄ aralkyl, C₆₋₁₀ aryl, or 5- to 10-membered heteroaryl containing one or more ring heteroatoms independently selected from O, N and S, each of which is optionally substituted with one or more groups independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, aminocarbonyl, (C₁₋₆ alkyl)aminocarbonyl, di(C₁₋₆ alkyl)aminocarbonyl, and 4- to 8-membered cyclic aminocarbonyl; or
2) a resin for solid phase synthesis having a side chain to which a compound having a H-N group capable of forming a C-N bond, represented by HNR⁶R⁷, is bound via a linker, wherein
   R⁶ and R⁷ together with the nitrogen atom to which they are bonded form a 5- to 7-membered saturated heterocycle, wherein the heterocycle is optionally substituted with one or more substituents independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl, or
   R⁶ and R⁷ are each independently a hydrogen atom, C₁₋₆ alkyl, C₂₋₆ alkenyl, C₂₋₆ alkynyl, C₃₋₈ cycloalkyl, (C₁₋₆ alkyl)carbonyl, (C₆₋₁₀ aryl)carbonyl, 5- to 10-membered heteroarylcarbonyl containing one or more ring heteroatoms independently selected from O, N and S, C₇₋₁₄ aralkyl, C₆₋₁₀ aryl, or 5- to 10-membered heteroaryl containing one or more ring heteroatoms independently selected from O, N and S, each of which is optionally substituted with one or more substituents independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl.

[H-10] The method according to any of [H-1] to [H-9], wherein compound 1 is a resin for solid phase synthesis having a side chain to which a compound represented by X¹-Ar² is bound via a linker, wherein
X¹ is a chlorine atom, a bromine atom, an iodine atom, or -O-SO₂-R⁴;
R⁴ is C₁₋₆ alkyl optionally substituted with one or more fluorine atoms, or phenyl optionally substituted with one or more fluorine atoms or C₁₋₆ alkyl optionally substituted with a fluorine atom;
Ar² is C₆₋₁₀ aryl, or 5- to 10-membered heteroaryl containing one or more ring heteroatoms independently selected from O, N and S, each of which is optionally substituted with one or more groups independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl.

[H-11] The method according to [H-9] or [H-10], wherein compound 1 has a chemical structure in which a compound represented by R⁵O-Ar², HOAr², R⁶R⁷N-Ar², Ar¹-Ar², R⁸R⁹R¹⁰C-Ar², R¹³R¹⁴R¹⁵C-Ar² or R¹⁶R¹⁷R¹⁸C-Ar² can be produced by degrading a linker contained in a side chain after a cross-coupling reaction.

[H-12] The method according to [H-10] or [H-11], wherein X¹ is a chlorine atom, a bromine atom, or an iodine atom.

[H-13] The method according to any of [H-10] to [H-12], wherein Ar² is independently selected from the group consisting of phenyl, naphthyl, pyrrolyl, thienyl, furyl, pyridyl, thiazolyl, isothiazolyl, pyrazolyl, oxazolyl, isoxazolyl, imidazolyl, triallyl, pyrimidyl, pyrazinyl, pyridazinyl, quinolinyl, isoquinolinyl, 4H-quinolidinyl, phthalazinyl, naphthyridinyl, quinoxalinyl, quinazolinyl, cinnolinyl, pteridinyl, indolyl, indolinyl, benzothiophenyl, benzofuranyl, benzisothiazolyl, benzisoxazolyl, indazolyl, benzimidazolyl, benzotriazolyl, azaindolyl, and imidazopyridyl, each of which is optionally substituted.

[H-14] The method according to any of [H-10] to [H-13], wherein Ar² is phenyl or pyridyl, each of which is optionally substituted.

[H-15] The method according to any of [H-10] to [H-14], wherein X¹ is a bromine atom; and
Ar² is phenyl or pyridyl, each of which is optionally substituted with one or more groups independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl.

[H-16] The method according to any of [H-1] to [H-15], wherein the solvent is selected from the group consisting of N,N-dimethylpropionamide (DMPr), N,N-diethylacetamide (DEAc) and N,N-diethylpropionamide (DEPr).

[H-17] The method according to any of [H-1] to [H-16], wherein the solvent is N,N-dimethylpropionamide (DMPr).

[H-18] The method according to any of [H-1] to [H-17], wherein the solvent is a solvent containing at least one selected from the group consisting of N,N-dimethylpropionamide (DMPr), N,N-diethylacetamide (DEAc) and N,N-diethylpropionamide (DEPr) at 30 v/v% or more, 50 v/v% or more, 70 v/v% or more, or 90 v/v% or more.

[H-19] The method according to any of [H-1] to [H-18], wherein the solvent is a solvent containing N,N-dimethylpropionamide (DMPr) at 30 v/v% or more, 50 v/v% or more, 70 v/v% or more, or 90 v/v% or more.

[H-20] The method according to any of [H-1] to [H-19], wherein the cross-coupling reaction is performed at 0 to 200°C, 0 to 150°C, 0 to 100°C, 10 to 80°C, or 25 to 80°C.

[H-21] The method according to any of [H-1] to [H-20], wherein the molar ratio of compound 1 to compound 2 used is compound 1/compound 2 = from 0.0005 to 500, from 0.005 to 200, or from 0.05 to 20.

[H-22] The method according to any of [H-1] to [H-21], wherein the molar ratio of the catalyst used is from 0.01 to 100 mol%, from 0.1 to 50 mol%, from 1 to 25 mol% relative to compound 1 or compound 2.

[H-23] The method according to any of [H-1] to [H-22], wherein the catalyst is a catalyst containing a palladium complex represented by any of the following general formulae (Cat1), (Cat2), (Cat3), (Cat4) and (Cat5): 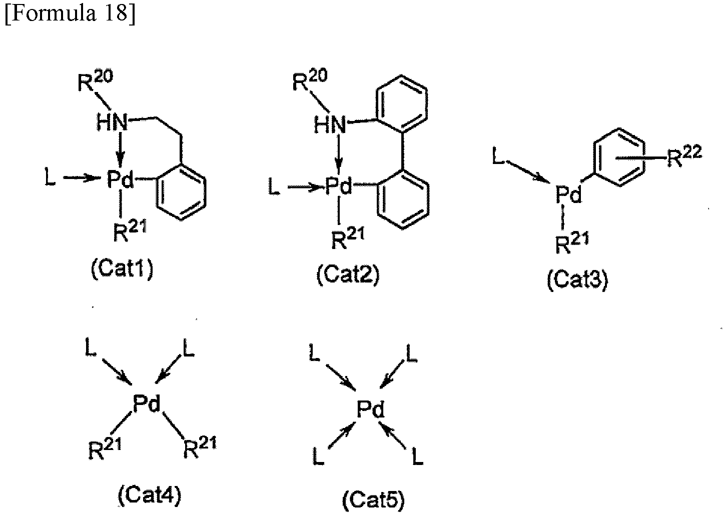
wherein R²⁰ is a hydrogen atom, C₁₋₆ alkyl, or C₆₋₁₀ aryl; R²¹ is halogen or -O-SO₂-CH₃; R²² is a hydrogen atom, C₁₋₆ alkyl optionally substituted with one or more fluorine atoms, or (C₁₋₆ alkoxy)carbonyl optionally substituted with tri(C₁₋₆ alkyl)silyl;
L is independently a monodentate ligand of the following general formula (L1), (L2), (L3), (L4), (L5), (L6) or (L7), or two L are a bidentate ligand of the following general formula (L8), (L9), (L10), (L1 1) or (L12):

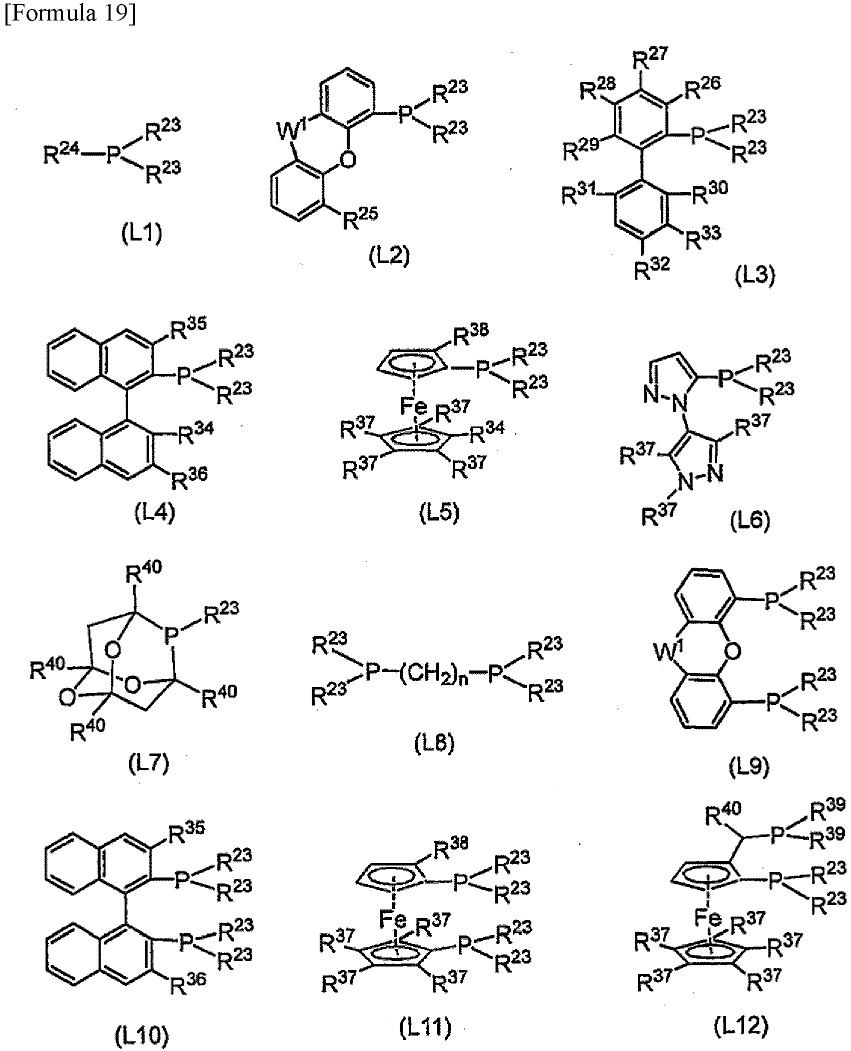

wherein R²³ is independently tert-butyl, cyclohexyl, 2-furanyl, 2-thienyl, 2-pyridyl, phenyl, or adamantyl, wherein the phenyl is optionally substituted with one or more fluorine atoms, C₁₋₆ alkyl optionally substituted with a fluorine atom, C₁₋₆ alkoxy, or dimethylamino;
R²⁴ is C₁₋₆ alkyl, cyclohexyl, 2-furanyl, 2-thienyl, 2-pyridyl, N-phenyl-2-pyrrolyl, N-phenyl-2-indolyl, phenyl, or adamantyl, wherein the phenyl is optionally substituted with one or more fluorine atoms, C₁₋₆ alkyl optionally substituted with a fluorine atom, C₁₋₆ alkoxy, morpholino, or dimethylamino;
R²⁵ is a hydrogen atom, C₁₋₆ alkyl, or C₁₋₆ alkoxy;
W¹ is -C(CH₃)₂-, or -NH-;
R²⁶, R²⁷, R²⁸, and R²⁹ are each independently a hydrogen atom, C₁₋₆ alkyl, C₁₋₆ alkoxy, or morpholino;
R³⁰, R³¹, and R³² are each independently a hydrogen atom, C₁₋₆ alkyl, C₁₋₆ alkoxy, or dimethylamino;
R³³ is a hydrogen atom or -SO₂-O-M, wherein M is lithium, sodium, or potassium;
R³⁴ is a hydrogen atom, C₁₋₆ alkyl, or C₁₋₆ alkoxy;
R³⁵ and R³⁶ are each independently a hydrogen atom, C₁₋₆ alkyl, or C₁₋₆ alkoxy;
R³⁷ is a hydrogen atom, or phenyl optionally substituted with C₁₋₆ alkyl;
R³⁸ is independently a hydrogen atom, phenyl optionally substituted with C₁₋₆ alkyl, or -CH(CH₃)-N(CH₃)₂;
R³⁹ is tert-butyl, cyclohexyl, or adamantyl;
R⁴⁰ is a hydrogen atom or C₁₋₆ alkyl; and an arrow represents a coordination bond.

[H-24] The method according to any of [H-1] to [H-22], wherein the catalyst is a catalyst containing a palladium complex represented by any of the following general formulae (Cat6) and (Cat7): 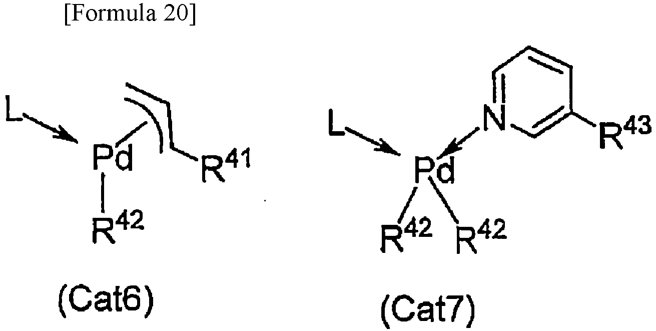 wherein R⁴¹ is a hydrogen atom or phenyl optionally substituted with C₁₋₆ alkyl; R⁴² is independently halogen; R⁴³ is a fluorine atom or a chlorine atom; and L is an N-heterocyclic carbene ligand represented by the following general formula (L12) or (L13): 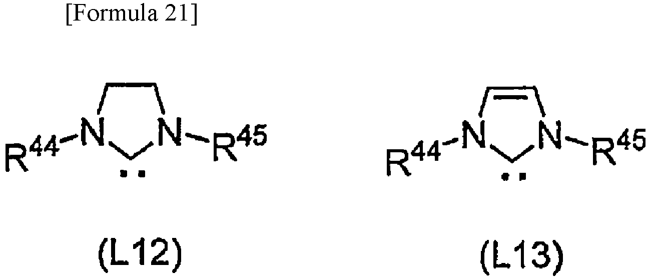 wherein R⁴⁴ and R⁴⁵ each independently represent C₁₋₆ alkyl, cyclohexyl, adamantyl, or phenyl, wherein the phenyl is optionally substituted with one or more C₁₋₆ alkyl, C₁₋₆ alkoxy, or dimethylamino, and a carbon atom with × × represents a carbene, and an arrow represents a coordination bond.

[H-25] The method according to any of [H-1] to [H-22], wherein the catalyst is a catalyst containing a palladium complex formed by a combination of a palladium compound selected from the group consisting of bis(allylchloropalladium(II)), tetrakis(triphenylphosphine)palladium(0), tris(dibenzylideneacetone)dipalladium(0)-chloroform adduct, a palladium(p-cinnamyl)chloride dimer, a (1-methylallyl)palladium chloride dimer, (1,5-cyclooctadiene)bis(trimethylsilylmethyl)palladium(II), a (2'-amino-1,1'-biphenyl-2-yl)methanesulfonatopalladium(II) dimer, and palladium(II) acetate, with a ligand selected from a ligand represented by a monodentate ligand of the following general formula (L1), (L2), (L3), (L4), (L5), (L6) or (L7), or a bidentate ligand of the following general formula (L8), (L9), (L10), (L11) or (L12): 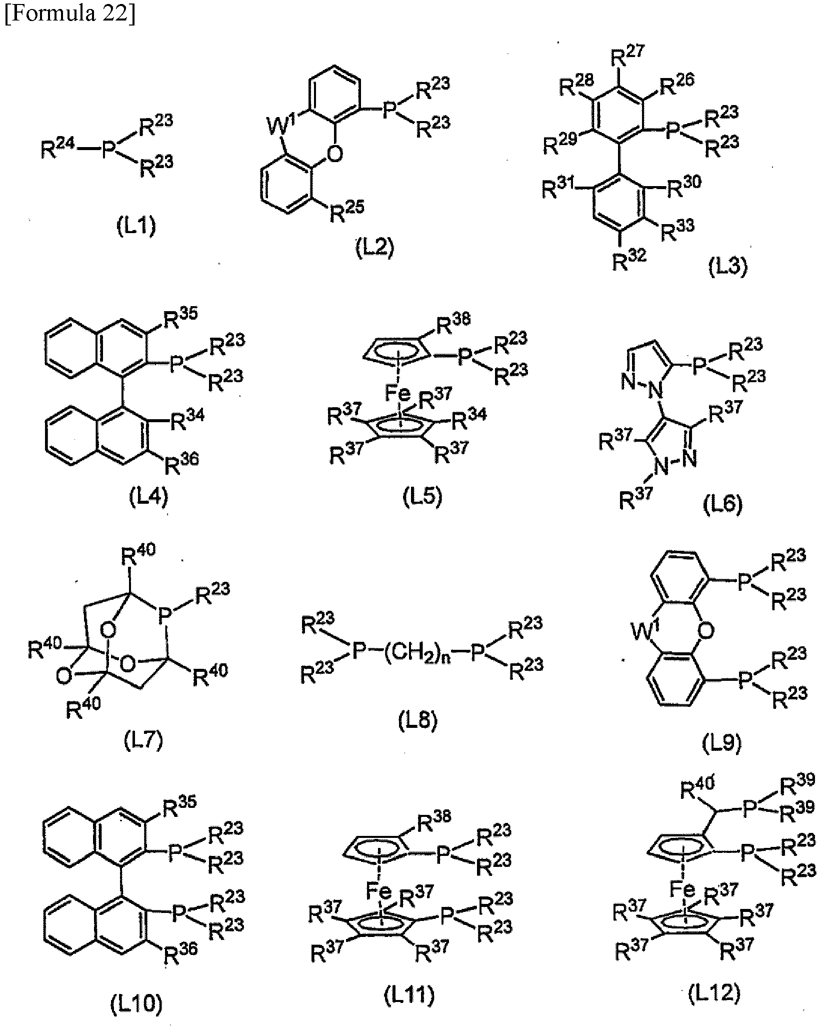
wherein R²³ is tert-butyl, cyclohexyl, 2-furanyl, 2-thienyl, 2-pyridyl, phenyl, or adamantyl, wherein the phenyl is optionally substituted with one or more fluorine atoms, C₁₋₆ alkyl optionally substituted with a fluorine atom, C₁₋₆ alkoxy, or dimethylamino;
R²⁴ is C₁₋₆ alkyl, cyclohexyl, 2-furanyl, 2-thienyl, 2-pyridyl, N-phenyl-2-pyrrolyl, N-phenyl-2-indolyl, phenyl, or adamantyl, wherein the phenyl is optionally substituted with one or more fluorine atoms, C₁₋₆ alkyl optionally substituted with a fluorine atom, C₁₋₆ alkoxy, morpholino, or dimethylamino;
R²⁵ is a hydrogen atom, C₁₋₆ alkyl, or C₁₋₆ alkoxy;
W¹ is -C(CH₃)₂-, or -NH-;
R²⁶, R²⁷, R²⁸, and R²⁹ are each independently a hydrogen atom, C₁₋₆ alkyl, C₁₋₆ alkoxy, or morpholino;
R³⁰, R³¹, and R³² are each independently a hydrogen atom, C₁₋₆ alkyl, C₁₋₆ alkoxy, or dimethylamino;
R³³ is a hydrogen atom or -SO₂-O-M, wherein M is lithium, sodium, or potassium;
R³⁴ is a hydrogen atom, C₁₋₆ alkyl, or C₁₋₆ alkoxy;
R³⁵ and R³⁶ are each independently a hydrogen atom, C₁₋₆ alkyl, or C₁₋₆ alkoxy;
R³⁷ is a hydrogen atom, or phenyl optionally substituted with C₁₋₆ alkyl;
R³⁸ is a hydrogen atom, phenyl optionally substituted with C₁₋₆ alkyl, or -CH(CH₃)-N(CH₃)₂;
R³⁹ is tert-butyl, cyclohexyl, or adamantyl;
R⁴⁰ is a hydrogen atom or C₁₋₆ alkyl; and an arrow represents a coordination bond, or a ligand being a salt thereof.

[H-26] The method according to any of [H-1] to [H-23], wherein the catalyst is a catalyst containing a palladium complex selected from a Buchwald first-generation catalyst precursor (G1), a Buchwald second-generation catalyst precursor (G2), a Buchwald third-generation catalyst precursor (G3), a Buchwald fourth-generation catalyst precursor (G4), a Buchwald fifth-generation catalyst precursor (G5), or a Buchwald sixth-generation catalyst precursor (G6).

[H-27] The method according to [H-25], wherein the catalyst is a catalyst containing a palladium complex formed by a combination of a palladium compound selected from the group consisting of bis(allylchloropalladium(II)), tetrakis(triphenylphosphine)palladium(0), tris(dibenzylideneacetone)dipalladium(0)-chloroform adduct, a palladium(p-cinnamyl)chloride dimer, a (1-methylallyl)palladium chloride dimer, (1,5-cyclooctadiene)bis(trimethylsilylmethyl)palladium(II), a (2'-amino-1,1'-biphenyl-2-yl)methanesulfonatopalladium(II) dimer, and palladium(II) acetate, with a ligand selected from a ligand represented by a monodentate ligand of the following general formula (L1), (L3), or (L6): 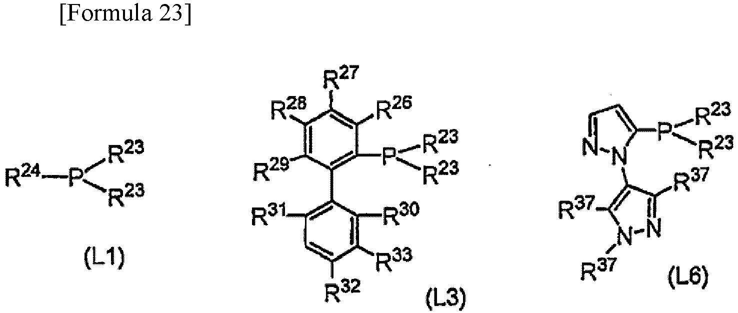
wherein, R²³ is independently tert-butyl, cyclohexyl, or adamantyl;
R²⁴ is C₁₋₆ alkyl, cyclohexyl, N-phenyl-2-indolyl, or adamantyl;
R²⁶, R²⁷, R²⁸, and R²⁹ are each independently a hydrogen atom, C₁₋₆ alkyl, C₁₋₆ alkoxy, or morpholino;
R³⁰, R³¹, and R³² are each independently a hydrogen atom, C₁₋₆ alkyl, C₁₋₆ alkoxy, or dimethylamino;
R³³ is a hydrogen atom or -SO₂-O-M, wherein M is lithium, sodium, or potassium;
R³⁷ is phenyl optionally substituted with C₁₋₆ alkyl, and a ligand being a salt thereof, and a palladium catalyst formed in combination with a compound represented by a salt thereof.

[H-28] The method according to any of [H-1] to [H-22], wherein the catalyst is a nickel catalyst.

[H-29] The method according to any of [H-1] to [H-22] and [H-28], wherein the catalyst is a catalyst containing a nickel complex formed by a combination of a nickel compound selected from the group consisting of bis(1,5-cyclooctadiene)nickel, dichloro(1,2-dimethoxyethane)nickel, dibromo(1,2-dimethoxyethane)nickel, nickel trifluoromethanesulfonate(II), bis(trifluoromethanesulfonimide)nickel(II), nickel(II) acetylacetonate, nickel(II) nitrate, nickel(II) bromide, nickel(II) chloride, and hydrates thereof, with a ligand selected from the group consisting tricyclohexylphosphine, 1,1'-bis(diphenylphosphino)ferocene, and 1,3-bis(diphenylphosphino)propane.

[H-30] The method according to any of [H-1] to [H-22], [H-28] and [H-29], wherein the catalyst is a catalyst containing a nickel complex selected from the group consisting of
dichlorobis(tricyclohexylphosphine)nickel(II),
dichloro[1,1'-bis(diphenylphosphino)ferrocene]nickel(II), and
dichloro[1,3-bis(diphenylphosphino)propane]nickel(II).

[H-31] The method according to any of [H-1] to [H-30], wherein the base includes at least one base selected from the group consisting of an organic base having a conjugate acid pKa of 23 or more in acetonitrile and an inorganic base having a conjugate acid pKa of 9 to 20 in water.

[H-32] The method according to any of [H-1] to [H-31], wherein the base is selected from the group consisting of amidines, guanidines, phosphazenes, carbonates of alkali metals, phosphates of alkali metals, C₁₋₆ alkoxides of alkali metals, and hydroxides of alkali metals.

[H-33] The method according to any of [H-1] to [H-32], wherein the base is selected from the group consisting of 2-tert-butyl-1,1,3,3-tetramethylguanidine (BTMG), 7-methyl-1,5,7-triazabicyclo[4.4.0]dec-5-ene (MTBD), 1-ethyl-2,2,4,4,4-pentakis(dimethylamino)-2l⁵,4l⁵-catenadi(phosphazene) (P2Et), 1-tert-butyl-2,2,4,4,4-pentakis(dimethylamino)-2l⁵,4⁵-catenadi(phosphazene) (P2tBu), tert-butylimino-tris(dimethylamino)phosphorane (P 1 tBu), 2-tert-butylimino-2-diethylamino-1,3-dimethylperhydro-1,3,2-diazaphosphorine (BEMP), tert-butylimino-tri(pyrrolidino)phosphorane (BTPP), carbonates of alkali metals, phosphates of alkali metals, C₁₋₆ alkoxides of alkali metals, and hydroxides of alkali metals.

[H-34] The method according to any of [H-1] to [H-33], wherein the base is selected from the group consisting of 2-tert-butyl-1,1,3,3-tetramethylguanidine (BTMG), 7-methyl-1,5,7-triazabicyclo[4.4.0]dec-5-ene (MTBD), 1-tert-butyl-2,2,4,4,4-pentakis(dimethylamino)-2l⁵,4l⁵-catenadi(phosphazene) (P2tBu), tert-butylimino-tris(dimethylamino)phosphorane (P1tBu), 2-tert-butylimino-2-diethylamino-1,3-dimethylperhydro-1,3,2-diazaphosphorine (BEMP), tert-butylimino-tri(pyrrolidino)phosphorane(BTPP), cesium carbonate, tripotassium phosphate, potassium hydroxide, and sodium tert-butoxide.

[H-35] The method according to any of [H-1] to [H-34], wherein a reaction system contains the base and further contains a salt.

[H-36] The method according to [H-35], wherein the salt is an alkali metal salt of an acid selected from the group consisting of trifluoroacetic acid, trifluoromethanesulfonic acid, trifluoromethanesulfonimide, tetrafluoroboric acid, hexafluorophosphoric acid, and hexafluoroantimonic(V) acid.

[H-37] The method according to [H-35], wherein the salt is sodium trifluoroacetate or potassium trifluoroacetate.

[H-38] The method according to any of [H-1] to [H-37], wherein the molar ratio of the base used to compound 1 or compound 2 is from 0.05 to 100, from 0.2 to 50, or from 1 to 30.

[H-39] The method according to any of [H-1] to [H-38], wherein a mixture containing two or more kinds of compound 1 is reacted.

[H-40] The method according to any of [H-1] to [H-39] for producing a compound constituting a compound library.

[H-41] The method according to any of [H-1] to [H-40], wherein two or more kinds of compound 1 are supported on a resin for solid-phase synthesis via a linker.

[H-42] The method according to any of [H-1] to [H-41], wherein three or more, four or more, five or more, seven or more, or ten or more kinds of compound 1 are supported on a resin for solid-phase synthesis via a linker.

[H-43] A method of producing a compound constituting a compound library, the method comprising producing the compound by the method according to any of [H-1] to [H-42].

### ADVANTAGEOUS EFFECTS OF INVENTION

According to the present invention, there is provided a method of preparing a compound by a cross-coupling reaction using a palladium catalyst, wherein the conversion rate is improved and/or the generation of by-products is suppressed.

### DESCRIPTION OF EMBODIMENTS

In one aspect, the present invention relates to a method of producing a compound by a cross-coupling reaction. Here the cross-coupling reaction is not particularly limited, but examples thereof include a C-O bond formation reaction or a C-N bond formation reaction by substitution with a leaving group.

In one aspect of the present invention, the catalyst added to the reaction system may be an active species that functions as a catalyst, or a catalyst precursor that is converted into an active species in the reaction system, or a metal compound (e.g., a palladium compound or a nickel compound) and a ligand that forms an active species in the reaction system. As used herein, the catalyst encompasses one or more compounds such as an active species having catalytic activity and a catalytic precursor forming an active species in a reaction system.

In one embodiment of the present invention, the cross-coupling reaction is performed in the presence of a palladium catalyst. Examples of the palladium catalyst used can include a palladium compound or a palladium complex described herein. When a palladium catalyst is used, the substrates for the C-O bond formation reaction may be water, or an alcohol, and a compound having a leaving group. As substrates for the C-N bond formation reaction, ammonia, a primary amine, and a secondary amine can be used.

The palladium catalyst is not particularly limited as long as it can be commonly used for cross-coupling reactions. Examples of the palladium catalyst that can be used include palladium acetate, Pd(dba)₂, Pd₂(dba)₃, Pd₂(dba)₃CHCl₃, an allylpalladium chloride dimer, a palladium(p-cinnamyl)chloride dimer, a (1-methylallyl)palladium chloride dimer, (1,5-cyclooctadiene)bis(trimethylsilylmethyl)palladium(II), and a (2'-amino-1,1'-biphenyl-2-yl)methanesulfonatopalladium(II) dimer.

The palladium catalyst may contain a ligand as needed. Examples of the ligand include trialkylphosphine (PCy₃ (tricyclohexylphosphine), P(tBu)₃ (tri-tert-butylphosphine), di(1-adamantyl)-n-butylphosphine (cataCXium A), di-tert-butylmethylphosphine ((tBu)₂PMe), triadamantylphosphine (P(Ad)₃), di-tert-butylneopentylphosphine (Neopentyl(tBu)₂P)), and triarylphosphine (triphenylphosphine, tri(o-tolyl)phosphine (P(o-Tol)₃)).

Further examples of the ligand include dialkylbiarylphosphines such as XPhos (2-dicyclohexylphosphino-2',4',6'-triisopropylbiphenyl), SPhos (2-dicyclohexylphosphino-2',6'-dimethoxybiphenyl), RuPhos (2-dicyclohexylphosphino-2',6'-diisopropoxybiphenyl), BrettPhos (2-(dicyclohexylphosphino)-3,6-dimethoxy-2',4',6'-triisopropyl-1,1'-biphenyl), tBuXPhos (2-di-tert-butylphosphino-2',4',6'-triisopropylbiphenyl), CPhos (2-dicyclohexylphosphino-2',6'-bis(N,N-dimethylamino)biphenyl), DavePhos (2-dicyclohexylphosphino-2'-(N,N-dimethylamino)biphenyl), GPhos (3-(tert-butoxy)-2',6'-diisopropyl-6-methoxy[1,1'-biphenyl]-2-yl)dicyclohexylphosphan), tBuDavePhos (2-di-tert-butylphosphino-2'-(N,N-dimethylamino)biphenyl), VPhos (4,6-di-tert-butyl-2'-dicyclohexylphosphino-2-methoxybiphenyl), EPhos (dicyclohexyl(3-isopropoxy-2',4',6'-triisopropyl-[1,1'-biphenyl]-2-yl)phosphan), CyJohnPhos (2-biphenyldicyclohexylphosphine), RockPhos (2-di(tert-butyl)phosphino-2',4',6'-triisopropyl-3-methoxy-6-methylbiphenyl, di-tert-butyl (2',4',6'-triisopropyl-3-methoxy-6-methyl- [1,1'-biphenyl]-2-yl)phosphine), Me₄tBuXPhos (2-di-tert-butylphosphino-3,4,5,6-tetramethyl-2',4',6'-triisopropyl-1,1'-biphenyl), Me₃(OMe)tBuXPhos (Sigma-Adrich catalog code: 792470), tBuBrettPhos (2-(di-tert-butylphosphino)-2',4',6'-triisopropyl-3,6-dimethoxy-1,1'-biphenyl), AdBrettPhos (2-(di-1-adamantylphosphino)-2',4',6'-triisopropyl-3,6-dimethoxy-1,1'-biphenyl), AlPhos (di-1-adamantyl(4"-butyl-2",3",5",6"-tetrafluoro-2',4',6'-triisopropyl-2-methoxy-meta-terphenyl)phosphine) and the like), monoalkyl monoaryl biaryl phosphine ((tBu) PhCPhos (2-[(tert-butyl)phenylphosphino]-2',6'-bis(N,N-dimethylamino)biphenyl)), diaryl biaryl phosphine (PhCPhos (2-diphenylphosphino-2',6'-bis(N,N-dimethylamino)biphenyl), bis(3,5-bis(trifluoromethyl)phenyl)(2',6'-bis(dimethylamino)-3,6-dimethoxybiphenyl-2-yl)phosphine, bis(3,5-bis(trifluoromethyl)phenyl)(2',6'-bis(isopropoxy)-3,6-dimethoxybinyl-2-ylphosphine, JackiePhos (2-{bis[3,5-bis(trifluoromethyl)phenyl]phosphino}-3,6-dimethoxy-2',4',6'-triisopropyl-1,1'-biphenyl), dialkyl monoarylphosphine (Aphos ((4-(N,N-dimethylamino)phenyl)di-tert-butylphosphine), (tBu)₂PPh, MorDalPhos (2-morpholinophenyldi(1-adamanthyl)phosphine), TrixiePhos (2-(di-tert-butylphosphino)-1,1'-binaphthyl), meCgPPH (1,3,5,7-tetramethyl-6-phenyl-2,4,8-trioxa-6-phosphaadamantane)), QPhos (1,2,3,4,5-pentaphenyl-1'-(di-t-butylphosphino)ferrocene), cataCXium PICy (2-(dicyclohexylphosphino)-1-(2,4,6-trimethylphenyl)-1H-imidazole), CyBippyPhos (5-(dicyclohexylphosphino)-1',3',5'-triphenyl-1' H-1,4'-bipyrazole), BippyPhos (5-(di-tert-butylphosphino)-1',3',5'-triphenyl-1'H-1,4'-bipyrazole), AdBippyPhos (5-[di(1-adamantyl) phosphino]-1',3',5'-triphenyl-1'H-1,4'-bipyrazole), cataCXium PtB (2-(di-tert-butylphosphino)-1-phenyl-1H-pyrrole), cataCXium POMetB (2-(di-tert-butylphosphino)-1-(2-methoxyphenyl)-1H-pyrrole), and cataCXium PIntB (2-(di-tert-butylphosphino)-1-phenyl-indol).

Still further examples of the ligand include bidentate ligands such as dppf (1,1'-bis(phenylphosphino)ferrocene), dtbpf (1,1'-bis(di-tert-butylphosphino)ferrocene), BINAP (2,2'-bis(diphenylphosphino)-1,1'-binaphthyl), Tol-BINAP (2,2'-bis(di-p-tolylphosphino)-1,1'-binaphthyl), XantPhos (4,5-bis(diphenylphosphino)-9,9-dimethylxanthen), N-XantPhos (4,6-bis(diphenylphosphino)phenoxazine), and Josiphos ((R)-1-[(Sp)-2-(dicyclohexylphosphino)ferrocenyl]ethyl di-tert-butylphosphine).

Yet further examples of the ligand include N-heterocyclic carbene (NHC)ligands, such as IPr (1,3-bis(2,6-diisopropylphenyl)imidazole-2-ylidene), SIPr (1,3-bis(2,6-di-i-propylphenyl)imidazolidine-2-ylidene), IMes (1,3-bis(2,4,6-trimethylphenyl)imidazole-2-ylidene), and IPent (1,3-bis(2,6-di-3-pentylphenyl)imidazole-2-ylidene).

The palladium catalyst may also be a palladium complex known as a palladium catalyst precursor. As the palladium catalyst precursor, a Buchwald first-generation catalyst precursor (G1) can be used. Examples thereof include XPhos Pd G1, SPhos Pd G1, RuPhos Pd G1, BrettPhos Pd G1, and tBuXPhos Pd G1.

As the palladium catalyst precursor, a Buchwald second-generation catalyst precursor (G2) can also be used. Examples thereof include XPhos Pd G2, SPhos Pd G2, RuPhos Pd G2, BrettPhos Pd G2, tBuXPhos Pd G2, CPhos Pd G2, DavePhos Pd G2, CyJohnPhs Pd G2, APhos Pd G2, (tBu)₂PPh Pd G2, MorDalPhos Pd G2, PCy₃ Pd G2, P(tBu)₃ Pd G2, cataCXium A Pd G2, (tBu)₂PMe Pd G2, Neopentyl(tBu)₂P Pd G2, P(o-Tol)₃ Pd G2, and XantPhos Pd G2.

As the palladium catalyst precursor, a Buchwald third-generation catalyst precursor (G3) can also be used. Examples include XPhos Pd G3, SPhos Pd G3, RuPhos Pd G3, BrettPhos Pd G3, tBuxPhos Pd G3, CPhos Pd G3, DavePhos Pd G3, GPhos Pd G3, tBuDavePhos Pd G3, VPhos Pd G3, JackiePhos Pd G3, CyJohnPhos Pd G3, RockPhos Pd G3, Me₄tBuXPhos Pd G3, Me₃(OMe)tBuXPhos Pd G3 (Sigma-Aldrich catalog code: 804193), tBuBrettPhos Pd G3, AdBrettPhos Pd G3, (tBu)PhCPhos Pd G3, PhCPhos Pd G3, Aphos Pd G3, (tBu)₂PPh Pd G3, MorDalPhos Pd G3, PCy₃ Pd G3, P(tBu)₃ Pd G3, cataCXium A Pd G3, (tBu)₂Pme Pd G3, Neopentyl(tBu)₂P Pd G3, P(o-Tol)₃ Pd G3, QPhos Pd G3, TrixiePhos Pd G3, meCgPPh Pd G3, dppf Pd G3, dtbpf Pd G3, BINAP Pd G3, Tol-BINAP Pd G3, XantPhos Pd G3, N-XantPhos Pd G3, and Josiphos Pd G3, and each of which may be coordinated with any solvent.

As the palladium catalyst precursor, a Buchwald fourth-generation catalyst precursor (G4) can also be used. Examples thereof include XPhos Pd G4, SPhos Pd G4, RuPhos Pd G4, BrettPhos Pd G4, tBuXPhos Pd G4, CPhos Pd G4, DavePhos Pd G4, GPhos Pd G4, tBuDavePhos Pd G4, VPhos Pd G4, EPhos Pd G4, CyJohnPhos Pd G4, tBuBrettPhos Pd G4, (tBu)PhCPhos Pd G4, methanesulfonato(2-bis(3,5-di(trifluoromethyl)phenylphosphino)-3,6-dimethoxy-2',6'-bis(dimethylamino)-1,1 '-biphenyl)(2'-methylamino-1,1 '-biphenyl-2-yl)palladium(II), APhos Pd G4, (tBu)₂PPh Pd G4, MorDalPhos Pd G4, PCy₃ Pd G4, P(tBu)₃ Pd G4, cataCXium A Pd G4, (tBu)₂PMe Pd G4, Neopentyl(tBu)₂P Pd G4, meCgPPh Pd G4, dppf Pd G4, BINAP Pd G4, Tol-BINAP Pd G4, XantPhos Pd G4, and N-XantPhos Pd G4.

As the palladium catalyst precursor, a Buchwald fifth-generation catalyst precursor (G5) can also be used. Examples thereof include XPhos Pd G5, BrettPhos Pd G5, SPhos Pd G5, and RuPhos Pd G5.

As the palladium catalyst precursor, a Buchwald sixth-generation catalyst precursor (G6) can also be used. Examples thereof include tBuBrettPhos Pd G6 TES, tBuBrettPhos Pd G6 Br, AdBrettPhos Pd G6 Br, Gphos Pd G6 TES, AlPhos Pd G6 Br, and AlPhos Pd G6 OTf.

As the palladium catalyst precursor, a dichloropalladium(II) complex can also be used. Examples thereof include Pd(PPh₃)₂Cl₂, Pd(PCy₃)₂Cl₂, Pd(dppf)Cl₂, Pd(dtbpf)Cl₂, (APhos)₂PdCl₂, and (AdBippyPhos)₂PdCl₂.

As the palladium catalyst precursor, a palladium(0) complex can also be used. Examples thereof include tetrakis(triphenylphosphine)palladium(0), bis(tricyclohexylphosphine)palladium(0), and bis(tri-tert-butylphosphine)palladium(0).

As the palladium catalyst precursor, a palladium(I) halide complex can also be used. Examples thereof include a bromo(tri-tert-butylphosphine)palladium(I) dimer and an iodo (tri-tert-butylphosphine)palladium(I) dimer.

As the palladium catalyst precursor, a phosphine p-allylpalladium catalyst can also be used. Examples thereof include XPhos Pd(crotyl)Cl(chloro(crotyl)(2-dicyclohexylphosphino-2',4',6'-triisopropyl-1,1'-biphenyl)palladium(II)), SPhos Pd(crotyl)Cl(chloro(crotyl)(2-dicyclohexylphosphino-2',6'-dimethoxy-1,1'-biphenyl)palladium(II)), RuPhos Pd(crotyl)Cl(chloro(crotyl)(2-dicyclohexylphosphino-2',6'-diisopropoxy-1,1'-biphenyl)palladium(II)), [BrettPhosPd(crotyl)]OTf(crotyl(2-dicyclohexylphosphino-2',4',6'-triisopropyl-3,6-dimethoxy-1,1'-biphenyl)palladium(II)triflate), [tBuXPhos Pd(allyl)]OTf(allyl(2-di-tert-butylphosphino-2',4',6'-triisopropyl-1,1'-biphenyl)palladium(II)triflate), [tBuBrettPhos Pd(allyl)]OTf(allyl(2-di-tert-butylphosphino-3,6-dimethoxy-2',4',6'-triisopropyl-1,1'-biphenyl)palladium(II) triflate), AmPhos Pd(crotyl)Cl(chloro(crotyl)[(p-dimethylaminophenyl)(di-tert-butylphosphine)]palladium(II)), P(Cy)3Pd(crotyl)Cl(chloro(crotyl)(tricyclohexylphosphine)palladium(II)), [P(tBu)3]Pd(crotyl)Cl(tri-tert-butylphosphine(chloro)(crotyl)palladium(II)), [BINAP Pd(allyl)]Cl((R)-BINAP Pd(allyl)]Cl;allyl[(R)-2,2'-bis(diphenylphosphino)-1,1'-binaphthalene]palladium(II) chloride), and [XantPhosPd(allyl)]Cl(allyl[4,5-bis(diphenylphosphino)-9,9-dimethylxanthene]palladium(II) chloride).

As the palladium catalyst precursor, a PEPPSI catalyst can also be used. Examples thereof include Pd-PEPPSI IPr, Pd-PEPPSI SIPr((1,3-bis(2,6-diisopropylphenyl)imidazolidene)(3-chloropyridyl)palladium(II) dichloride), and Pd-PEPPSI IPent(1,3-bis(2,6-di-3-pentylphenyl)imidazole-2-ylidene).

As the palladium catalyst precursor, a (NHC)Pd(allyl)Cl catalyst can also be used. Examples thereof include allyl[1,3-bis(2,6-diisopropylphenyl)imidazole-2-ylidene] chloropalladium(II), allyl[1,3-bis(2,6-diisopropylphenyl)-2-imidazolidinylidene]chloropalladium(II), and [1,3-bis(2,6-diisopropylphenyl)imidazole-2-ylidene]chloro [3-phenylallyl]palladium(II).

As the palladium catalyst precursor, a NHC-Pd naphthoquinone catalyst can also be used. For example, a 1,3-bis(2,6-diisopropylphenyl)imidazole-2-ylidene(1,4-naphthoquinone)palladium(0) dimer or the like can be used as the palladium catalyst precursor.

In one embodiment of the invention, XPhos Pd G3, SPhos Pd G3, RuPhos Pd G3, BrettPhos Pd G3, tBuXPhos Pd G3, CPhos Pd G3, DavePhos Pd G3, GPhos Pd G3, tBuDavePhos Pd G3, VPhos Pd G3, JackiePhos Pd G3, CyJohnPhos Pd G3, RockPhos Pd G3, Me₄tBuXPhos Pd G3, Me₃(OMe)tBuXPhos Pd G3, tBuBrettPhos Pd G3, AdBrettPhos Pd G3, (tBu)PhCPhos Pd G3, PhCPhos Pd G3, APhos Pd G3, (tBu)₂PPh Pd G3, MorDalPhos Pd G3, PCy₃ Pd G3, P(tBu)₃ Pd G3, cataCXium A Pd G3, (tBu)₂PMe Pd G3, Neopentyl(tBu)₂P Pd G3, P(o-Tol)₃ Pd G3, QPhos Pd G3, TrixiePhos Pd G3, meCgPPh Pd G3, dppf Pd G3, dtbpf Pd G3, XPhos Pd G4, SPhos Pd G4, RuPhos Pd G4, BrettPhos Pd G4, tBuXPhos Pd G4, CPhos Pd G4, DavePhos Pd G4, GPhos Pd G4, tBuDavePhos Pd G4, VPhos Pd G4, EPhos Pd G4, CyJohnPhos Pd G4, tBuBrettPhos Pd G4, (tBu)PhCPhos Pd G4, methanesulfonato(2-bis(3,5-di(trifluoromethyl)phenylphosphino)-3,6-dimethoxy-2',6'-bis(dimethylamino)-1,1'-biphenyl)(2'-methylamino-1,1'-biphenyl-2-yl)palladium(II), APhos Pd G4, (tBu)₂PPh Pd G4, MorDalPhos Pd G4, PCy₃ Pd G4, P(tBu)₃ Pd G4, cataCXium A Pd G4, (tBu)₂PMe Pd G4, Neopentyl(tBu)₂P Pd G4, meCgPPh Pd G4, dppf Pd G4, tBuBrettPhos Pd G6 TES, tBuBrettPhos Pd G6 Br, AdBrettPhos Pd G6 Br, GPhos Pd G6 TES, AlPhos Pd G6 Br, AlPhos Pd G6 OTf, Pd(PCy₃)₂Cl₂, Pd(dppf)Cl₂, Pd(dtbpf)Cl₂, (APhos)₂PdCl₂, bis(tricyclohexylphosphine)palladium(0), bis(tri-tert-butylphosphine)palladium(0), Pd-PEPPSI IPr, Pd-PEPPSI SIPr, Pd-PEPPSI-IPent can be used as the palladium catalyst precursor, and in particular, RuPhos Pd G4, tBuXPhos Pd G4, RockPhos Pd G3, BrettPhos Pd G4, tBuBrettPhos Pd G4, AdBrettPhos Pd G3, AdBrettPhos Pd G6 Br, (tBu)PhCPhos Pd G4 can be preferably used as the palladium catalyst precursor.

In one embodiment of the invention, XPhos, SPhos, RuPhos, BrettPhos, tBuXPhos, CPhos, DavePhos, GPhos, tBuDavePhos, VPhos, JackiePhos, CyJohnPhos, RockPhos, Me₄tBuXPhos, Me₃(OMe)tBuXPhos, tBuBrettPhos, AdBrettPhos, (tBu)PhCPhos, PhCPhos, bis(3,5-bis(trifluoromethyl)phenyl)(2',6'-bis(dimethylamino)-3,6-dimethoxybiphenyl-2-yl)phosphine, bis(3,5-bis(trifluoromethyl)phenyl)(2',6'-bis(isopropoxy)-3,6-dimethoxybiphenyl-2-yl)phosphine, APhos, (tBu)₂PPh, MorDalPhos, PCy₃, P(tBu)₃, cataCXium A, (tBu)₂PMe, Neopentyl(tBu)₂P, QPhos, TrixiePhos, meCgPPh, dppf, dtbpf, cataCXium PICy, CyBippyPhos, BippyPhos, AdBippyPhos, cataCXium PtB, cataCXium POMetB, cataCXium PIntB can be used as the ligand for palladium catalysts, and in particular, RuPhos, tBuXPhos, RockPhos, BrettPhos, tBuBrettPhos, AdBrettPhos, (tBu)PhCPhos, BippyPhos, AdBippyPhos, cataCXiumPIntB, meCgPPh can preferably be used as the ligand for palladium catalysts.

In one embodiment of the invention, palladium acetate, Pd(dba)₂, Pd₂(dba)₃, Pd₂(dba)₃CHCl₃, an allylpalladium chloride dimer, a palladium(p-cinnamyl)chloride dimer, a (1-methylallyl)palladium chloride dimer, (1,5-cyclooctadiene)bis(trimethylsilylmethyl)palladium(II), and a (2'-amino-1,1'-biphenyl-2-yl)methanesulfonatopalladium(II) dimer can be used as the palladium catalyst, and in particular, Pd₂(dba)₃CHCl₃ can be preferably used as the palladium catalyst.

The number of equivalents of the palladium catalyst used can be appropriately set by those skilled in the art. For example, in one embodiment of the invention, the number of equivalents of the palladium catalyst can be from 0.001 to 10 equivalents, from 0.001 to 7.5 equivalents, from 0.001 to 5 equivalents, from 0.01 to 5 equivalents, or from 0.01 to 3 equivalents relative to a compound having a leaving group such as a halogen atom (compound 1 or a compound represented by X¹-Ar² herein), which is one of the substrates of the cross-coupling reaction. The number of equivalents of the ligand used can be appropriately set by one skilled in the art, for example, it can be from 0.001 to 10 equivalents, from 0.001 to 7.5 equivalents, from 0.001 to 5 equivalents, from 0.01 to 5 equivalents, or from 0.01 to 3 equivalents relative to a compound having a leaving group. The number of equivalents of the catalyst precursor used can be appropriately set by those skilled in the art, for example, it can be from 0.001 to 10 equivalents, from 0.001 to 7.5 equivalents, from 0.001 to 5 equivalents, from 0.01 to 5 equivalents, or from 0.01 to 3 equivalents relative to a compound having a leaving group.

For example, in one embodiment of the invention, the number of equivalents of the palladium catalyst can be from 0.001 to 10 equivalents, from 0.001 to 7.5 equivalents, from 0.001 to 5 equivalents, from 0.01 to 5 equivalents, or from 0.01 to 3 equivalents relative to another substrate (compound also referred to as compound 2 herein) that reacts with the compound having the leaving group in the cross-coupling reaction. The number of equivalents of the ligand used can be appropriately set by one skilled in the art, for example, it can be from 0.001 to 10 equivalents, from 0.001 to 7.5 equivalents, from 0.001 to 5 equivalents, from 0.01 to 5 equivalents, or from 0.01 to 3 equivalents relative to the compound. The number of equivalents of the catalyst precursor used can be appropriately set by one skilled in the art, for example, it can be from 0.001 to 10 equivalents, from 0.001 to 7.5 equivalents, from 0.001 to 5 equivalents, from 0.01 to 5 equivalents, or from 0.01 to 3 equivalents relative to the compound.

In one embodiment of the present invention, the cross-coupling reaction is performed in the presence of a nickel catalyst. The nickel catalyst can be, for example, a nickel complex described herein. When a nickel catalyst is used, water, or an alcohol, and a compound having a leaving group can be used as substrates for the C-O bond formation reaction. As substrates for the C-N bond formation reaction, ammonia, a primary amine, and a secondary amine can be used.

The nickel catalyst is not particularly limited as long as it can be commonly used for cross-coupling reactions. Examples of the nickel catalyst include bis(1,5-cyclooctadiene)nickel, dichloro(1,2-dimethoxyethane)nickel, dibromo(1,2-dimethoxyethane)nickel, nickel(II) chloride, nickel(II) bromide, nickel(II) trifluoromethanesulfonate, bis(trifluoromethanesulfonimide)nickel(II), nickel(II) acetylacetonate, and nickel(II) nitrate, and each of which may be coordinated with water. In one embodiment of the invention, a nickel catalyst formed by a combination of at least one selected from the group consisting of bis(1,5-cyclooctadiene)nickel and nickel(II) chloride and at least one selected from the group consisting of tricyclohexylphosphine, 1,1'-bis(diphenylphosphino)ferrocene, and 1,3-bis(diphenylphosphino)propane can be used as the catalyst for cross-coupling.

The number of equivalents of the nickel catalyst used can be appropriately set by those skilled in the art. For example, in one embodiment of the invention, the number of equivalents of the nickel catalyst can be from 0.001 to 10 equivalents, from 0.001 to 7.5 equivalents, from 0.001 to 5 equivalents, from 0.01 to 5 equivalents, or from 0.01 to 3 equivalents relative to a compound having a leaving group such as a halogen atom (compound 1 or a compound represented by X¹-Ar² herein), which is one of the substrates of the cross-coupling reactions. The number of equivalents of the ligand used can be appropriately set by one skilled in the art, for example, it can be from 0.001 to 10 equivalents, from 0.001 to 7.5 equivalents, from 0.001 to 5 equivalents, from 0.0 to 5 equivalents, or from 0.01 to 3 equivalents relative to a compound having a leaving group. The number of equivalents of the catalyst precursor used can be appropriately set by those skilled in the art, for example, it can be from 0.001 to 10 equivalents, from 0.001 to 7.5 equivalents, from 0.001 to 5 equivalents, from 0.01 to 5 equivalents, or from 0.01 to 3 equivalents relative to a compound having a leaving group.

For example, in one embodiment of the invention, the number of equivalents of the nickel catalyst can be from 0.001 to 10 equivalents, from 0.001 to 7.5 equivalents, from 0.001 to 5 equivalents, from 0.01 to 5 equivalents, or from 0.01 to 3 equivalents relative to another substrate (compound also referred to herein as compound 2) that reacts with the compound having the leaving group in the cross-coupling reaction. The number of equivalents of the ligand used can be appropriately set by one skilled in the art, for example, it can be from 0.001 to 10 equivalents, from 0.001 to 7.5 equivalents, from 0.001 to 5 equivalents, from 0.01 to 5 equivalents, or from 0.01 to 3 equivalents relative to the compound. The number of equivalents of the catalyst precursor used can be appropriately set by one skilled in the art, for example, it can be from 0.001 to 10 equivalents, from 0.001 to 7.5 equivalents, from 0.001 to 5 equivalents, from 0.01 to 5 equivalents, or from 0.01 to 3 equivalents relative to the compound.

In one aspect of the invention, compound 1 having a leaving group X¹ on a carbon atom of the aromatic ring can be used as one substrate in the cross-coupling reaction. The aromatic ring herein is not particularly limited as long as it is an aromatic cyclic chemical structure, and examples thereof include a C₆₋₁₀ aromatic carbocycle, or a 5- to 10-membered aromatic heterocycle containing one or more ring heteroatoms independently selected from O, N, and S. The number of the leaving group X¹ contained in compound 1 is at least one. For example, compound 1 may have 1 to 10, 1 to 5, or 1 to 3 leaving groups. When compound 1 has more than one leaving group X¹, the leaving groups X¹ may be the same or different. One aromatic ring may contain two or more carbon atoms each having a leaving group X¹, or compound 1 may contain one or more aromatic rings containing one or more leaving groups X¹. In one embodiment, the number of the leaving group X¹ in compound 1 is one.

In one embodiment of the invention, the leaving group X¹ is a halogen atom, or -O-SO₂-R⁴. In a preferred embodiment, the leaving group X¹ is a bromine atom or a chlorine atom, and in a more preferred embodiment, the leaving group X¹ is a bromine atom.

As the compound used for cross-coupling with compound 1, compound 2 having a hydroxy capable of forming a C-O bond or an H-N group capable of forming a C-N bond can be used. In one aspect of the invention, compound 2 has one or more reactive groups selected from a hydroxy capable of forming a C-O bond and a H-N group capable of forming a C-N bond. When compound 2 has a plurality of reactive groups, the reactive groups may be the same or different. In one embodiment, the number of the reactive group in compound 1 is one.

In one aspect of the invention, compound 2 is a compound having a hydroxy capable of forming a C-O bond, and examples thereof include water and an alcohol. Examples of the alcohol include a compound represented by HO-R⁵. R⁵ is C₁₋₆ alkyl, C₂₋₆ alkenyl, C₂₋₆ alkynyl, C₃₋₈ cycloalkyl, C₇₋₁₄ aralkyl, C₆₋₁₀ aryl, or 5- to 10-membered heteroaryl containing one or more ring heteroatoms independently selected from O, N and S, each of which is optionally substituted with one or more groups independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, aminocarbonyl, (C₁₋₆ alkyl)aminocarbonyl, di(C₁₋₆ alkyl)aminocarbonyl, and 4- to 8-membered cyclic aminocarbonyl.

In one embodiment of the invention, a compound represented by HO-R⁵ can be used as the substrate for cross-coupling. In another embodiment of the invention, a compound containing a chemical structure of the compound represented by HO-R⁵ in a molecule can be used as the substrate for cross-coupling. In one embodiment, the compound containing a chemical structure of the compound represented by HO-R⁵ in a molecule does not contain a reactive group other than a reactive group derived from HO-R⁵.

In one embodiment of the invention, a resin for solid phase synthesis in which a compound represented by one or more HO-R⁵ or a compound containing a chemical structure of the compound represented by HO-R⁵ in a molecule is supported via a linker can be used as the substrate for cross-coupling. Here, "supported" means that the compound binds via a linker. Thus, in the above embodiment, a resin for solid-phase synthesis having a side chain to which the compound is bound via a linker can be used as the substrate for cross-coupling.

In one aspect of the invention, compound 2 is a compound having an H-N group capable of forming a C-N bond, and examples thereof include ammonia and amines. Examples of the amines include a compound represented by HNR⁶R⁷. In the compound, R⁶ and R⁷ together with the nitrogen atom to which they are bonded form a 5- to 7-membered saturated heterocycle, wherein the heterocycle is optionally substituted with one or more substituents independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl, or

R⁶ and R⁷ are each independently a hydrogen atom, C₁₋₆ alkyl, C₂₋₆ alkenyl, C₂₋₆ alkynyl, C₃₋₈ cycloalkyl, (C₁₋₆ alkyl)carbonyl, (C₆₋₁₀ aryl)carbonyl, 5- to 10-membered heteroarylcarbonyl containing one or more ring heteroatoms independently selected from O, N and S, C₇₋₁₄ aralkyl, C₆₋₁₀ aryl, or 5- to 10-membered heteroaryl containing one or more ring heteroatoms independently selected from O, N and S, each of which is optionally substituted with one or more substituents independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl.

In one embodiment of the invention, a compound represented by HNR⁶R⁷ can be used as the substrate for cross-coupling. In another embodiment of the invention, a compound containing a chemical structure of the compound represented by HNR⁶R⁷ in a molecule can be used as the substrate for cross-coupling. In one embodiment, the compound containing a chemical structure of the compound represented by HNR⁶R⁷ in a molecule does not contain a reactive group other than a reactive group derived from HNR⁶R⁷.

In one embodiment of the invention, a resin for solid phase synthesis in which a compound represented by one or more HNR⁶R⁷ or a compound containing a chemical structure of the compound represented by HNR⁶R⁷ in a molecule is supported via a linker can be used as the substrate for cross-coupling. Here, "supported" means that the compound binds via a linker. Thus, in the above embodiment, a resin for solid-phase synthesis having a side chain to which the compound is bound via a linker can be used as the substrate for cross-coupling.

As used herein, "R⁶ and R⁷ together with the nitrogen atom to which they are bonded form a 5- to 7-membered saturated heterocycle" is not particularly limited as long as it is a nitrogen-containing 5- to 7-membered saturated heterocycle, and examples thereof may include pyrrolidine, piperidine, piperazine, morpholine, thiomorpholine, azacycloheptane, diazacycloheptane, and triazacycloheptane. The heterocycle may have a substituent.

In one aspect of the invention, the cross-coupling reaction is performed in a solvent containing an amide-based solvent represented by formula A: 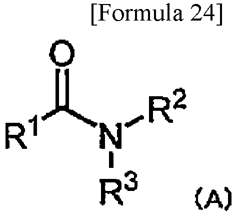 wherein R¹, R² and R³ are each independently C₁₋₄ alkyl provided that the sum of carbon atoms of R¹, R² and R³ is 4 or more and 6 or less. Specific examples of the solvent include N-ethyl-N-methylacetamide, N,N-diethylacetamide, N-methyl-N-propylacetamide, N-ethyl-N-propylacetamide, N-methyl-N-butylacetamide, N,N-dimethylpropionamide, N,N-ethyl-N-methylpropionamide, N,N-diethylpropionamide, N-methyl-N-propionamide, N-ethyl-N-methylbutanoic acid amide, N,N-dimethylbutanoic acid amide, N,N-diethylbutanoic acid amide, N,N-dimethylisobutanoic acid amide, N-ethyl-N-methylisobutanoic acid amide, or N,N-diethylisobutanoic acid amide.

In a preferred embodiment of the invention, the amide-based solvent is N,N-dimethylpropionamide, N,N-diethylacetamide, or N,N-diethylpropionamide. In one embodiment, a mixed solvent containing two or more amide-based solvents represented by formula A is used as the solvent for the cross-coupling reaction. In one embodiment, one solvent selected from the amide-based solvent represented by formula A is used as the solvent for the cross-coupling reaction. In one embodiment, a solvent containing an amide-based solvent represented by formula A at 30%v/v or more, 40%v/v or more, 50%v/v or more, 60%v/v or more, 70%v/v or more, 80%v/v or more, 85%v/v or more, 90%v/v or more, or 95%v/v or more is used as the solvent for the cross-coupling reaction.

The reaction temperature can be appropriately set by one skilled in the art, for example, it can be from 0 to 200°C, from 0 to 150°C, from 0 to 100°C, from 10 to 80°C, or from 25 to 80°C.

In one aspect of the invention, a base can be used for the cross-coupling reaction. The base can be a base that is commonly used for cross-coupling reactions, and a mixture of a plurality of bases can be used. In one embodiment of the invention, the base is selected from the group consisting of an organic base having a conjugate acid pKa of 23 or more in acetonitrile and an inorganic base having a conjugate acid pKa of 9 to 20 in water. Examples of the base include pyridine, 2,6-lutidine, 2,4,6-collidine, DTBP, DTBMP, triethylamine, DIPEA, 4-methylmorpholine, DBU, DBN, MTBD, BTMG, TMG, 1,8-bis(dimethylamino)naphthalene, N,N-dimethylaniline, P1-tBu, and P2tBu. In one embodiment, the base is selected from 2-tert-butyl-1,1,3,3-tetramethylguanidine (BTMG), 7-methyl-1,5,7-triazabicyclo[4.4.0]dec-5-ene (MTBD), 1-ethyl-2,2,4,4,4-pentakis(dimethylamino)-2l⁵,4l⁵-catenadi(phosphazene) (P2Et), 1-tert-butyl-2,2,4,4,4-pentakis(dimethylamino)-2l⁵,4l-catenadi(phosphazene) (P2tBu), tert-butylimino-tris(dimethylamino)phosphorane (P1tBu), 2-tert-butylimino-2-diethylamino-1,3-dimethylperhydro-1,3,2-diazaphosphorine (BEMP), tert-butylimino-tri(pyrrolidino)phosphorane (BTPP), carbonates of alkali metals, phosphates of alkali metals, C₁₋₆ alkoxides of alkali metals, and hydroxides of alkali metals. The number of equivalents of the base used can be appropriately set by one skilled in the art, for example, it can be from 1 to 100 equivalents, from 1 to 75 equivalents, from 1 to 50 equivalents, from 1 to 30 equivalents, or from 1 to 10 equivalents relative to the carboxylic acid containing A.

The reaction time of the cross-coupling reaction can be appropriately set by one skilled in the art, for example, it can be in the range of 1 minute to 96 hours, 5 minutes to 72 hours, 10 minutes to 48 hours, 15 minutes to 48 hours, or 30 minutes to 24 hours.

As used herein, the aromatic ring includes, for example, a 5- to 10-membered monocyclic or fused aromatic ring, a C₆₋₁₀ aromatic ring, or a 5- to 10-membered heteroaromatic ring containing one or more ring heteroatoms independently selected from O, N and S, and examples thereof include a benzene ring, a naphthalene ring, a pyrrole ring, a thiophene ring, a furan ring, a pyridine ring, a thiazole ring, an isothiazole ring, a pyrazole ring, an oxazole ring, an isoxazole ring, an imidazole ring, a triazole ring, a pyrimidine ring, a uridine ring, a pyrazine ring, a pyridazine ring, a quinoline ring, an isoquinoline ring, a 4H-quinolizine ring, a phthalazine ring, a naphthyridine ring, a quinoxaline ring, a quinazoline ring, a cinnoline ring, a pteridine ring, an indole ring, an indoline ring, a benzothiophene ring, a 1-methyl-1H-indole ring, a benzofuran ring, a benzisothiazole ring, a benzisoxazole ring, an indazole ring, a benzimidazole ring, a benzotriazole ring, an azaindole ring, and an imidazopyridine ring.

Examples of the C₆₋₁₀ aryl herein include phenyl and naphthyl. Examples of the 5-to 10-membered heteroaryl containing one or more ring heteroatoms independently selected from O, N and S include pyrrolyl, thienyl, furyl, pyridyl, thiazolyl, isothiazolyl, pyrazolyl, oxazolyl, isoxazolyl, imidazolyl, triallyl, pyrimidyl, pyrazinyl, pyridazinyl, quinolinyl, isoquinolinyl, 4H-quinolidinyl, phthalazinyl, naphthyridinyl, quinoxalinyl, quinazolinyl, cinnolinyl, pteridinyl, indolyl, indolinyl, benzothiophenyl, benzofuranyl, benzisothiazolyl, benzisoxazolyl, indazolyl, benzimidazolyl, benzotriazolyl, azaindolyl, and imidazopyridyl.

As used herein, C₁₋₆ alkyl is a linear and branched monovalent saturated aliphatic group having 1 to 6 carbon atoms. Specific examples thereof include methyl, ethyl, n-propyl, isopropyl, n-butyl, isobutyl, sec-butyl, t-butyl, 1-methylpropyl, n-pentyl, isopentyl, 2-methylbutyl, 1,1-dimethylpropyl, 1-ethylpropyl, hexyl, 4-methylpentyl, and 2-ethylbutyl.

As used herein, C₁₋₄ alkyl is a linear and branched monovalent saturated aliphatic group having 1 to 4 carbon atoms. Specific examples thereof include methyl, ethyl, n-propyl, isopropyl, n-butyl, isobutyl, sec-butyl, t-butyl, and 1-methylpropyl.

As used herein, C₂₋₆ alkenyl is a linear and branched monovalent group of 2 to 6 carbon atoms having one or more double bonds. Examples thereof include ethenyl (vinyl), 1-propenyl, 2-propenyl (allyl), propen-2-yl, and 3-butenyl (homoallyl).

As used herein, C₂₋₆ alkynyl refers to a linear or branched monovalent group of 2 to 6 carbon atoms having one or more triple bonds, and examples thereof include ethynyl, 1-propynyl, 2-propynyl, 1-butynyl, 2-butynyl, and 3-butynyl.

As used herein, C₃₋₈ cycloalkyl refers to a cyclic saturated aliphatic hydrocarbon group of 3 to 8 carbon atoms. Specific examples thereof include cyclopropyl, cyclobutyl, cyclopentyl, and cyclohexyl.

As used herein, C₁₋₆ alkoxy refers to a C₁₋₆ alkyl-O- group, wherein the C₁₋₆ alkyl is as already defined. Specific examples thereof include methoxy, ethoxy, 1-propoxy, 2-propoxy, n-butoxy, i-butoxy, sec-butoxy, and t-butoxy.

As used herein, (C₁₋₆ alkoxy)carbonyl refers to a C₁₋₆ alkoxy-C(=O)- group, wherein the C₁₋₆ alkoxy is as already defined.

As used herein, (C₁₋₆ alkyl)carbonyl refers to a C₁₋₆ alkyl-C(=O)- group, wherein the C₁₋₆ alkyl is as already defined.

As used herein, "(C₁₋₆ alkoxy)carbonyl" of (C₁₋₆ alkoxy)carbonylamino is as already defined.

As used herein, "(C₁₋₆ alkyl)" of (C₁₋₆ alkyl)amino is as already defined.

As used herein, "(C₁₋₆ alkyl)" of di(C₁₋₆ alkyl)amino is as already defined and may be the same or different.

As used herein, 4- to 8-membered cyclic amino includes groups such as aziridinyl, azetidinyl, pyrrolidinyl, piperidinyl, piperazinyl, morpholinyl, and the like, which bind with a nitrogen atom.

As used herein, "(C₆₋₁₀ aryl)" of (C₆₋₁₀ aryl)carbonylamino is as already defined.

As defined herein, "5- to 10-membered heteroaryl containing one or more ring heteroatoms independently selected from O, N, and S" of 5- to 10-membered heteroarylcarbonylamino containing one or more ring heteroatoms independently selected from O, N, and S.

As used herein, aminocarbonyl means -CONH₂.

"(C₁₋₆ alkyl)" of (C₁₋₆ alkyl)aminocarbonyl is as already defined.

As used herein, "(C₁₋₆ alkyl)" of di(C₁₋₆ alkyl)aminocarbonyl is as already defined and may be the same or different.

As used herein, 4- to 8-membered cyclic amino of 4- to 8-membered cyclic aminocarbonyl is as already defined, in which the nitrogen atom is bound to the carbonyl.

As used herein, "(C₆₋₁₀ aryl)" of (C₆₋₁₀ aryl)carbonyl is as already defined.

As defined herein, "5- to 10-membered heteroaryl containing one or more ring heteroatoms independently selected from O, N, and S" of 5- to 10-membered heteroarylcarbonyl containing one or more ring heteroatoms independently selected from O, N, and S.

As used herein, "(C₁₋₆ alkyl)" of tri(C₁₋₆ alkyl)silyl is as already defined and may be the same or different. Examples thereof include trimethylsilyl, triethylsilyl, and t-butyldimethylsilyl.

As used herein, the "halogen atom" refers to a fluorine atom, a chlorine atom, a bromine atom, an iodine atom, and the like. In the present invention, when a halogen atom is a substituent for an aryl, a heteroaryl, and the like, examples of the preferred halogen atom include a fluorine atom, a chlorine atom, and a bromine atom. In the present invention, when a halogen atom is a substituent for an alkyl or a group containing an alkyl as a part thereof (alkoxy, alkenyl, alkylthio, and the like), examples of the preferred halogen atom include a fluorine atom. Specific examples of the group having a halogen atom as a substituent include trifluoromethyl, pentafluoroethyl, trifluoromethoxy, pentafluoroethoxy, trifluoromethylthio, and pentafluoroethylthio. The preferred halogen atom as X¹ is a chlorine atom, a bromine atom, and an iodine atom.

Examples of C₁₋₆ alkyl optionally substituted with one or more fluorine atoms include trifluoromethyl.

As used herein, C₇₋₁₄ aralkyl refers to aryl-substituted alkyl having a total 7 to 14 carbon atoms. Examples thereof include benzyl, 1-phenethyl, 2-phenethyl, 1-naphthylmethyl, and 2-naphthylmethyl.

As used herein, the alkali metal means lithium, sodium, potassium, rubidium, cesium, and francium. Examples of the alkali metal that forms a salt such as an alkoxide and a hydroxide include lithium, sodium, and potassium.

In one aspect of the invention, the cross-coupling reaction is performed in a reaction system containing the base and further containing a salt. The salt is not particularly limited and examples thereof include organic acid metal salts such as, in particular, fluorine-substituted carboxylic acid metal salts, fluorine-substituted sulfonic acid metal salts, or fluorine-substituted sulfonimide metal salts, and inorganic acid metal salts such as, in particular, fluorine atom-containing borate metal salts, fluorine atom-containing phosphoric acid metal salts, and fluorine atom-containing antimonate metal salts. Examples of the metal salts include alkali metal salts. In one embodiment of the invention, the salt is, for example, an alkali metal salt of an acid selected from the group consisting of trifluoroacetic acid, trifluoromethanesulfonic acid, trifluoromethanesulfonimide, tetrafluoroboric acid, hexafluorophosphoric acid, and hexafluoroantimonic(V) acid.

In one aspect of the invention, the cross-coupling reaction can be performed using a resin for solid-phase synthesis on which compound 1 or 2 is supported as a substrate. In another aspect of the invention, the cross-coupling reaction can be performed using a resin for solid-phase synthesis on which two or more, three or more, four or more, five or more, seven or more, or ten or more kinds of compound 1 or 2 are supported via a linker as a substrate. The resin for solid-phase synthesis used as the solid-phase support is not particularly limited as long as it is commonly used. Examples thereof include a Carboxylic resin, a CTC resin, a Trt resin, a SASRIN resin, a Rink amide resin, a PAL AM resin, a Seiber amide resin, a Merrifield resin, a Wang resin, 2-(4-bromomethylphenoxy)ethyl polystyrene, and a solid phase support having any functional group such as a carboxy group, an amino group, an aminomethyl group, a hydroxy group, or a hydroxymethyl group on polystyrene. Furthermore, the resin for solid-phase synthesis may have a design in which any linker covalently connecting the support and compound 1 or 2 can be used to make cleaving between the linker and the compound. The support is also not particularly limited, and examples thereof include polystyrene and polyethylene glycol (PEG).

Methods and reaction conditions for having compound 1 or 2 supported on a resin for solid-phase synthesis can be appropriately set by one skilled in the art based on the methods described in known literatures and the like. The reaction conditions for cleaving the compound from the resin for solid-phase synthesis can be appropriately set by a person skilled in the art based on the chemical structure of the resin for solid-phase synthesis used. Examples of the reagents used for cleavage can include hydrochloric acid, carboxylic acids such as trifluoroacetic acid (TFA), fluoroalcohols such as 2,2,2-trifluoroethanol (TFE) or 1,1,1,3,3,3-hexafluoroisopropyl alcohol (HFIP), Brensted acid with pKa of 10 or less in water, or any Lewis acid. In one embodiment, a compound cut out from a resin for solid-phase synthesis can be used as a compound for screening for pharmaceutical exploration.

In one aspect of the present invention, the cross-coupling reaction can be performed with a resin for solid-phase synthesis on which either a resin for solid-phase synthesis having a leaving group X¹ on a carbon atom of an aromatic ring in a side chain as compound 1, or a resin for solid-phase synthesis having a reactive group capable of a C-O bond formation reaction or a C-N bond formation reaction by substitution of a side chain with the leaving group as compound 2 is supported as a substrate. In another aspect of the invention, the cross-coupling reaction can be performed using a resin for solid-phase synthesis to which two or more, three or more, four or more, five or more, seven or more, or ten or more kinds of different compounds bind as a substrate. The resin for solid-phase synthesis used as the solid-phase support is not particularly limited as long as it is commonly used. Examples thereof include a Carboxylic resin, a CTC resin, a Trt resin, a SASRIN resin, an Rink amide resin, a PAL AM resin, a Seiber amide resin, a Merrifield resin, a Wang resin, 2-(4-bromomethylphenoxy)ethyl polystyrene, and a solid phase support having any functional group such as a carboxyl group, an amino group, an aminomethyl group, a hydroxy group, or a hydroxymethyl group on polystyrene. Furthermore, the resin for solid-phase synthesis may have a design in which cleavage can be made between the linker and compound 1 or 2 by using the linker that covalently connects the support and the compound. The support is also not particularly limited, and examples thereof include polystyrene and polyethylene glycol (PEG).

Methods and reaction conditions for preparing a resin for solid-phase synthesis having a side chain to which a predetermined compound is bound via a linker can be appropriately set by one skilled in the art based on the methods described in known literatures and the like. The reaction conditions for cleaving the compound from the resin for solid-phase synthesis can be appropriately set by a person skilled in the art based on the chemical structure of the resin for solid-phase synthesis used. Examples of the reagents used for cleavage can include hydrochloric acid, carboxylic acids such as trifluoroacetic acid (TFA), fluoroalcohols such as 2,2,2-trifluoroethanol (TFE) or 1,1,1,3,3,3-hexafluoroisopropyl alcohol (HFIP), Brensted acid with pKa of 10 or less in water, or any Lewis acid. In one embodiment, a compound cleaved from a resin for solid-phase synthesis can be used as a compound for screening for pharmaceutical exploration.

Hereinafter, the present invention will be described in more detail using Reference Examples and Examples, but the present invention is not limited to these Examples.

### Examples

All starting materials, reagents, and solvents were obtained from commercial suppliers, or synthesized by known methods. The reagents and solvents were of reagent quality or better and were used as obtained from various commercial sources, unless otherwise noted. For palladium catalysts, information such as commercial source and catalog code is shown below.

**[Table 1]**

| **Palladium catalyst** | **Commercial source, catalog code, and the like** | |
|---|---|---|
| Pd₂dba₃CHCl₃ | Wako | catalog code: 209-19844 |
| tBuXPhos Pd G4 | Strem Chemicals | catalog code: 46-0330 |
| RockPhos Pd G3 | Strem Chemicals | catalog code: 46-0335 |
| BrettPhos Pd G4 | Sigma-Aldrich | catalog code: 804355 |
| tBuBrettPhos Pd G4 | LEAP Chem | CAS No.: 1980785-05-4 |
| AdBrettPhos Pd G3 | Sigma-Aldrich | catalog code: 776106 |
| AdBrettPhos Pd G6 Br | Sigma-Aldrich | catalog code: 915378 |
| RuPhos Pd G4 | Sigma-Aldrich | catalog code: 804290 |
| (tBu)PhCPhos Pd G4 | Sigma-Aldrich | catalog code: 900533 |
| MorDalPhos Pd G4 | Sigma-Aldrich | catalog code: 900276 |
| tBu3P Pd G4 | Sigma-Aldrich | catalog code: 900701 |
| meCgPPh Pd G4 | Sigma-Aldrich | catalog code: 900697 |
| PEPPSI-IPent | Sigma-Aldrich | catalog code: 732117 |

For silica gel of the column chromatography, Biotage(R) SNAP MLtra, Biotage(R) Sfaer D (Duo) (60 mm), or Biotage(R) Sfaer HC D (Duo) (20 mm), or the like was appropriately used.

For amino silica gel of the column chromatography, Biotage(R) SNAP Isolute NH2 (50 mm) or Biotage(R) SNAP Cartridge KP-NH, or the like was appropriately used.

For reverse phase silica gel of the column chromatography, Biotage(R) SNAP Mltra C18 (25 mm), Biotage(R) Sfaer C18 (30 mm), or the like was appropriately used.

¹H-NMR and ¹³C-NMR spectra were analyzed, with or without Me₄Si as the internal standard substance, using ECP-400 (manufactured by JEOL Ltd.), Agilent 400-MR (manufactured by Agilent Technologies), AVANCE3 Cryo-TCI, AVANCE3 400, AVANCE3 HD 400, AVANCE NEO 400, AVANCE3 HD 300, AVANCE3 300, AVANCE2 300, AVANCE NEO 300 (manufactured by Bruker) or the like as appropriate (s = singlet, brs = broad singlet, d = doublet, t = triplet, q = quartet, dd = double doublet, ddd = double double doublet, dt = double triplet, td = triple doublet, and m = multiplet).

The reaction tracking and purity analysis were carried out, unless otherwise noted, by performing retention time measurement and mass spectrometry using 2020 (manufactured by Shimadzu Corporation), under the analysis conditions shown in the following tables.

In Examples, the following abbreviations were used.

**[Table 2]**

| Abbreviations | |
|---|---|
| TFA | trifluoroacetic acid |
| DCM | dichloromethane |
| DEAc | N,N-diethylacetamide |
| DEPr | N,N-diethylpropionamide |
| DMAc | N,N-dimethylacetamide |
| DMF | N,N-dimethylformamide |
| DMI | 1,3-dimethyl-2-imidazolidinone |
| DMOc | N,N-dimethyloctaneamide |
| DMPr | N,N-dimethylpropionamide |
| DMPU | N,N'-dimethylpropyleneurea |
| DMSO | dimethyl sulfoxide |
| MeCN | acetonitrile |
| MeOH | methanol |
| NMP | N-methylpyrrolidone |
| tAmylOH | 2-methyl-2-butanol |
| THF | tetrahydrofuran |
| Pd₂dba₃CHCl₃ | tris(dibenzylideneacetone)dipalladium(0)-chloroform adduct (CAS: 52522-40-4) |
| Pd G3 | Buchwald third-generation catalyst precursor (see, https://www.sigmaaldrich.com/content/dam/sigma-aldrich/docs/SAJ/Brochure/1/saj1975.pdf and https://www.strem.com/uploads/resources/documents/ buchwaldligprecat.pdf) |
| Pd G4 | Buchwald fourth-generation catalyst precursor (see, https://www.sigmaaldrich.com/content/dam/sigma-aldrich/docs/SAJ/Brochure/1/saj1975.pdf and https://www.strem.com/uploads/resources/documents/ buchwaldligprecat.pdf) |
| BippyPhos | 5-(di-tert-butylphosphino)-1',3',5'-triphenyl-1'H-1,4'-bipyrazole (CAS: 894086-00-1) |
| AdBippyPhos | 5-[di(1-adamantyl)phosphino]-1',3',5'-triphenyl-1'H-1,4'-bipyrazole (CAS: 1239478-87-5) |

**[Table 3]**

| | |
|---|---|
| cataCXium PIntB | 2-(di-tert-butylphosphino)-1-phenylindole (CAS: 740815-37-6) |
| RuPhos | 2-dicyclohexylphosphino-2',6'-diisopropoxylbiphenyl (CAS: 787618-22-8) |
| tBuXPhos | 2-di-tert-butylphosphino-2',4',6'-triisopropylbiphenyl (CAS: 564483-19-8) |
| RockPhos | 2-(di-tert-butylphosphino)-3-methoxy-6-methyl-2',4',6'-triisopropylbiphenyl (CAS: 1262046-34-3) |
| BrettPhos | 2-(dicyclohexylphosphino)-3,6-dimethoxy-2',4',6'-triisopropylbiphenyl (CAS: 1070663-78-3) |
| tBuBrettPhos | 2-(di-tert-butylphosphino)-3,6-dimethoxy-2',4',6'-triisopropylbiphenyl (CAS: 1160861-53-9) |
| AdBrettPhos | 2-[di(1-adamantyl)phosphino]-3,6-dimethoxy-2',4',6'-triisopropylbiphenyl (CAS: 1160861-59-5) |
| AdBrettPhos Pd G6 Br | See, HP of Sigma-Aldrich (catalog code: 915378) |
| (tBu)PhCPhos | 2-[(tert-butyl)phenylphosphino]-2',6'-(N,N-dimethylamino)biphenyl (CAS: 1660153-91-2) |
| MorDalPhos | di(1-adamantyl)-2-morpholinophenylphosphine (CAS: 1237588-12-3) |
| tBu3P | tri-tert-butylphosphine |
| meCgPPh | 1,3,5,7-tetramethyl-6-phenyl-2,4,8-trioxa-6-phosphaadamantane (CAS: 97739-46-3) |
| PEPPSI-IPent | [1,3-bis(2,6-di-3-pentylphenyl)imidazol-2-ylidene](3-chloropyridyl)dichloropalladium(II) (CAS: 1158652-41-5) |
| Cs2CO3 | cesium carbonate |
| K3PO4 | tri-potassium phosphate |
| KOH | potassium hydroxide |
| NaOtBu | sodium tert-butoxide |
| LiHMDS | lithium hexamethyldisilazide |
| BTMG | 2-tert-butyl-1,1,3,3-tetramethylguanidine |
| MTBD | 7-methyl-1,5,7-triazabicyclo[4.4.0]dec-5-ene |
| P1tBu | tert-butylimino-tris(dimethylamino)phosphorane (CAS: 81675-81-2) |
| BEMP | 2-tert-butylimino-2-diethylamino-1,3-dimethylperhydro-1,3,2-diazaphosphorine (CAS: 98015-45-3) |
| BTPP | tert-butylimino-tri(pyrrolidino)phosphorane (CAS: 161118-67-8) |

**[Table 4]**

| | |
|---|---|
| P2Et | 1-ethyl-2,2,4,4,4-pentakis(dimethylamino)-215,415-catenadi(phosphazene) (CAS: 165535-45-5) |
| P2tBu | 1-tert-butyl-2,2,4,4,4-pentakis(dimethylamino)-215,415-catenadi(phosphazene) (CAS: 111324-03-9) |
| NaTFA | sodium trifluoroacetate |
| KTFA | potassium trifluoroacetate |
| DIC | N,N'-diisopropylcarbodiimide |
| HOAt | 1-hydroxy-7-azabenzotriazole |
| PyClU | 1-(chloro-1-pyrrolidinylmethylene)pyrrolidinium hexafluorophosphate (CAS: 135540-11-3) |
| PipClU | chlorodipiperidinocarbenium hexafluorophosphate (CAS: 161308-40-3) |
| NMI | 1-methylimidazol |

The analysis conditions of LCMS are shown in Table LC01.

**[Table 5]**

| [Table LC01] | | | | | |
|---|---|---|---|---|---|
| Analysis method | Column | Mobile phase A | Mobile phase B | Gradient | Flow rate mL/min |
| FA05-1 | Ascentis Express C18 | 0.1%FA H₂O | 0.1 %FA MeCN | A/B=95/5 → 0/100(1.5 min) → 0/100(0.5 min) | 1 |
| | 2.1mmI.D.x50mm, 2.7um | | | | |
| FA05-2 | Ascentis Express C18 | 0.1%FA H₂O | 0.1 %FA MeCN | A/B=95/5 → 0/100(1.5 min) → 0/100(1.5 min) | 1 |
| | 2.1mmI.D.x50mm, 2.7um | | | | |
| FA05-long | Ascentis Express C18 | 0.1%FA H₂O | 0.1 %FA MeCN | A/B=95/5 → 0/100(4.5 min) → 0/100(0.5 min) | 1 |
| | 2.1mmI.D.x50mm, 2.7um | | | | |
| RPAmideTFA05-long | Ascentis Express RP-Amide | 0.05%TFA H₂O | 0.05%TFA MeCN | A/B=95/5 → 0/100(4.5 min) → 0/100(0.5 min) | 1 |
| | 2.1mmI.D.x50mm, 2.7um | | | | |

The descriptions of m/z [M+H]⁺ and (M+H)⁺ noted in the LCMS analysis results in Examples all indicate values detected in positive mode, unless otherwise noted. Also, the UV area% in LCMS indicated values with PDA (190 to 400 nm or 210 to 400 nm), unless otherwise noted. When a specific wavelength (for example, 299 nm) is described, the UV area% at wavelengths up to +/- 4 nm centered on the described wavelength was shown. Note that the blank in the table indicates that it was below the detection limit.

The expression "concentration under reduced pressure" refers to the evaporation and removal of a solvent under reduced pressure with a rotary evaporator, a mechanical oil vacuum pump, or a mechanical oil-free vacuum pump.

The expression "drying overnight under reduced pressure" refers to the evaporation and removal of a solvent under reduced pressure with a rotary evaporator, a mechanical oil vacuum pump, or a mechanical oil-free vacuum pump.

The expressions "overnight" and "all night" refer to, but are not limited to, about 8 to 14 hours, unless otherwise noted.

The solid phase reaction can be performed in an appropriate container, such as a glass vial that can be tightly sealed by a cap equipped with a Teflon (R) packing or a column having a frit filter and an appropriate stopper. The container size is selected as appropriate such that there is sufficient space for the solvent and sufficient room for the resin to be effectively stirred, taking into account that certain resins may be significantly swollen when treated with organic solvents.

Stirring in the solid phase reaction was performed at 50 to 200 rpm using an appropriate shaker (for example, Tokyo Rikakikai Co., Ltd., EYELA, MMS-320, MMS-220H, or Asone, MyBL-100CS, or TAITEC, M×BR-104) or an agitator (combination of Asahi Glassplant inc., separable flask, and NAKAMURA SCIENTIFIC INSTRUMENT CO., LTD., sealing mixer UZU, and AQUATECHS Co., Ltd., centrifugal agitator C-Mix) as appropriate in order to ensure adequate mixing, which is a factor generally accepted as important for successful reaction on the resin.

In order to monitor the progress of the reaction on the solid phase, it is necessary to collect the resin from the reaction vessel. At that time, using a micropipette fitted with a pipette tip cut at the appropriate length from the end, the resin was collected by sucking approximately 10 mL to ensure that the resin was included and was transferred onto the filter of a pipette tip equipped with a filter (for example, Thermo Scientific, tip with ART filter, ART20P, 2149P-05). Thereafter, the compound supported on the resin was cleaved from the resin by the following representative procedures for the resin on the filter. The resin was washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and the progress of the reaction was analyzed by LCMS analysis.

The expression "cleavage" from the solid phase indicates the removal of the compound supported on the resin from the resin, such as a treatment of the resin with a 10% TFA/DCM solution containing 0.02 M pentamethylbenzene to recover the supported compound into the solution.

The compound No. used in Examples was indicated by the combination of arbitrary alphabets, numbers, and symbols. For compounds in the state wherein they are supported on the solid phase, "R" was added at the end, for example, "A02-1R". In contrast, the compound cleaved from the solid phase was indicated as "A02" without "-1R".

The notation 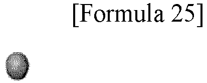 used in the chemical structure representations in Examples refer to polystyrene resin and indicate the state wherein the compound is supported on the solid phase.

In "-1R" used in Examples to indicate that the compound is supported on the solid phase, the number indicates the lot of resin used.

### Notation example of "-1R"

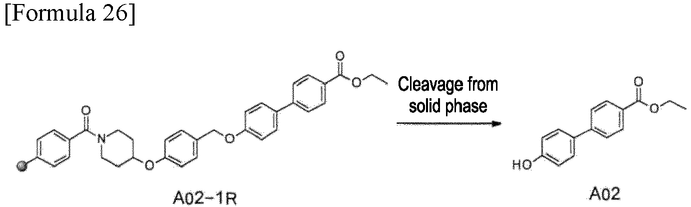

For the solid phase-supported compound used in the solid phase synthesis, the loading rate (mmol/g) is shown, which indicates the loading rate calculated when the cleaved compound is assumed to be 100% supported on the solid phase.

Even when the solid phase-supported compound is the same, the loading rate may vary from lot to lot, but the same compound No. may be used for the compound No.

### Example 1: Synthesis of compounds used in the present specification

### Example 1-1-1: Synthesis of tert-butyl 4-[4-[[4-(4-ethoxycarbonylphenyl)phenoxy]methyl]phenoxy]piperidine-1 -carboxylate (Compound a04)

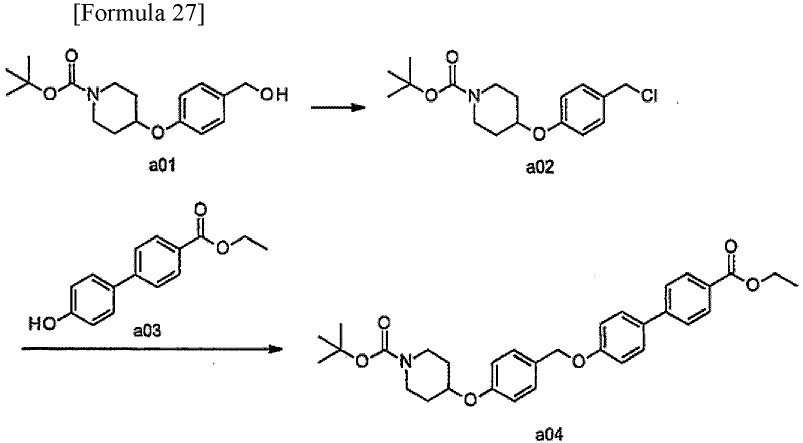

Under a nitrogen atmosphere, to a 100 mL three-neck flask, tert-butyl 4-[4-(hydroxymethyl)phenoxy]piperidine-1-carboxylate (a01, 1.00 g, 3.25 mmol), triethylamine (0.499 mL, 3.58 mmol) and DCM (16.3 mL) were added, and the reaction vessel was cooled to 0°C. Methanesulfonyl chloride (0.266 mL, 3.42 mmol) was added, and the mixture was stirred at 0°C for 3 hours. To the resulting mixture, a saturated aqueous sodium bicarbonate solution (4.9 mL) was added. The organic layer was extracted three times with dichloromethane (24 mL), dried over sodium sulfate, and then concentrated under reduced pressure. Under a nitrogen atmosphere, the resulting residue (a02), ethyl 4-(4-hydroxyphenyl)benzoate (a03, 0.866 g, 3.58 mmol), cesium carbonate (2.12 g, 6.50 mmol) and NMP (12.0 mL) were mixed in a 100 mL three-neck flask, and the mixture was stirred at room temperature for 24 hours. To the resulting mixture, a saturated aqueous ammonium chloride solution (6 mL) and ethyl acetate (12 mL) were added. The organic layer was extracted three times with ethyl acetate (10 mL), and then the resulting organic layers were mixed, and hexane (20 mL) was added. The organic layer was washed three times with water (15 mL) and once with a saturated aqueous sodium chloride solution (15 mL) and concentrated under reduced pressure. The resulting residue was purified by silica gel column chromatography (NH-silica gel, 0-25% ethyl acetate/hexane), and then the resulting crude product was purified by silica gel column chromatography (NH-silica gel, 0-100% dichloromethane/hexane). The resulting crude product was dissolved in ethyl acetate (100 mL) and hexane (200 mL), and then washed three times with water (200 mL) and once with saturated aqueous sodium chloride solution (100 mL), and concentrated under reduced pressure to afford the title compound a04 (1.44 g, 2.71 mmol, 83%) as a white solid.

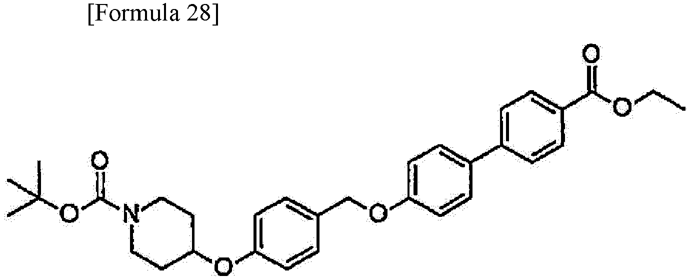

### Compound a04

¹H-NMR (400 MHz, CDCl₃) d 8.08 (d, J = 8.4 Hz, 2H), 7.61 (d, J = 8.4 Hz, 2H), 7.57 (d, J = 8.8 Hz, 2H), 7.37 (d, J = 8.4 Hz, 2H), 7.06 (d, J = 8.8 Hz, 2H), 6.94 (d, J = 8.4 Hz, 2H), 5.04 (s, 1H), 4.50-4.45 (m, 1H), 4.40 (q, J = 7.2 Hz, 2H), 3.73-3.67 (m, 2H), 3.38-3.31 (m, 2H), 1.96-1.88 (m, 2H), 1.80-1.71 (m, 2H), 1.47 (s, 9H), 1.41 (t, J = 7.2 Hz, 3H).
LRMS: m/z 554 [M+Na]⁺
Retention time: 1.684 min (analysis condition FA05-1, 290 nm)

### Example 1-1-2: Synthesis of ethyl 4-[4-[(4-piperidin-4-yloxyphenyl)methoxy]phenyl]benzoate (compound a05)

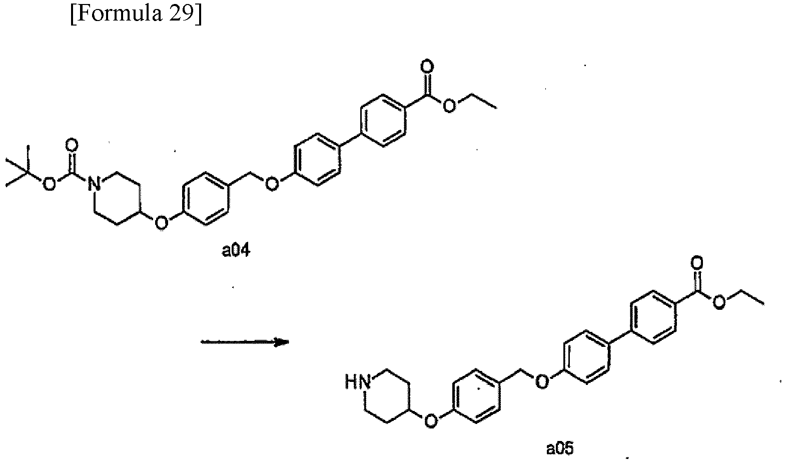

Under a nitrogen atmosphere, to a 5 mL screw cap vial, tert-butyl 4-[4-[[4-(4-ethoxycarbonylphenyl)phenoxy]methyl]phenoxy]piperidine-1-carboxylate (a04, 50.0 mg, 94.0 mmol), N,N-diisopropylethylamine (29.5 mL, 0.169 mmol) and THF (1.88 mL) were added, and the reaction vessel was cooled to 0°C. Trimethylsilyl trifluoromethanesulfonate (20.4 mL, 0.113 mmol) was added, and the mixture was stirred at 0°C for 3 hours. N,N-diisopropylethylamine (2.95 mL, 16.9 mmol) and trimethylsilyl trifluoromethanesulfonate (2.0 mL, 11 mmol) were added, and the mixture was stirred at 0°C for 1.5 hours. To the resulting mixture, triethylamine (26.2 mL) was added at 0°C, and the mixture was stirred at room temperature for 30 minutes. Water (847 mL), DMSO (1 mL), and formic acid (12.1 mL, 0.282 mmol) were added, and the mixture was purified by reverse phase column chromatography (C18, 0-60% 0.1% acetonitrile formate solution/0.1% aqueous formic acid solution). The resulting product was dissolved in dichloromethane (100 mL), and then washed three times with a saturated aqueous sodium bicarbonate solution (50 mL) and once with a saturated aqueous sodium chloride solution (50 mL), and concentrated under reduced pressure to afford the title compound a05 (19.8 mg, 45.9 mmol, 49%) as a white solid.

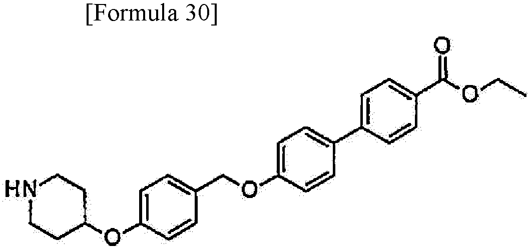

### Compound a05

¹H-NMR (400 MHz, CDCl₃) d 8.08 (d, J = 8.4 Hz, 2H), 7.61 (d, J = 8.8 Hz, 2H), 7.57 (d, J = 8.8 Hz, 2H), 7.36 (d, J = 8.8 Hz, 2H), 7.06 (d, J = 8.8 Hz, 2H), 6.94 (d, J = 8.8 Hz, 2H), 5.03 (s, 1H), 4.42-4.36 (m, 3H), 3.15 (ddd, J = 13.6, 4.8, 4.8 Hz, 2H), 2.74 (ddd, J = 13.6, 9.2, 3.2 Hz, 2H), 2.06-1.99 (m, 2H), 173-1.64 (m, 2H), 1.41 (t, J = 7.2 Hz, 3H).
LRMS: m/z 432 [M+H]⁺
Retention time: 0.904 min (analysis condition FA05-1, 290 nm)

### Example 1-2-1: Synthesis of compound A02-1R

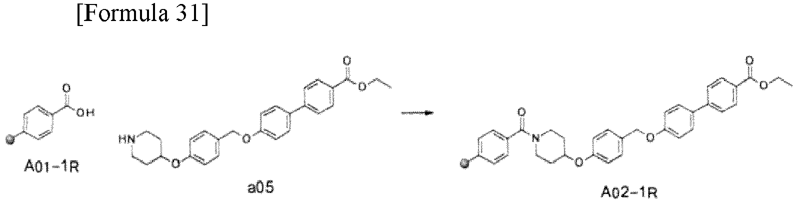

Under a nitrogen atmosphere, to a 20 mL glass vial, Carboxylic Resin (A01-1R) (Chem-Impex, loading rate 2.19 mmol/g, 1.00 g, 2.19 mmol) and NMP (15 mL) were added, and the mixture was shaken at room temperature for 1 hour. Ethyl 4-[4-[(4-piperidin-4-yloxyphenyl)methoxy]phenyl]benzoate (a05) (0.106 g, 0.246 mmol) and piperidine (0.033 mL, 0.329 mmol), HOAt (0.298 g, 2.19 mmol), DIC (0.341 mL, 2.19 mmol) were added, and the mixture was shaken at room temperature for 4 hours. Piperidine (1.30 mL, 13.1 mmol), HOAt (1.79 g, 13.1 mmol), and DIC (2.05 mL, 13.1 mmol) were added, and the mixture was shaken at room temperature all night.

### Solid-phase purification

The whole of the suspension of the reaction solution and the solid phase was transferred onto a filter, and washed 3 times with NMP (20 mL), 3 times with MeOH (20 mL), and 3 times with DCM (20 mL), and the resulting solid phase was dried overnight under reduced pressure to afford compound A02-1R (loading rate 0.200 mmol/g, 1.25 g, 0.250 mmol).

### Reaction tracking

The suspension (10 mL) of the reaction solution and the solid phase was transferred onto a filter, and washed 3 times with NMP (0.1 mL), 3 times with MeOH (0.1 mL), and 3 times with DCM (0.1 mL), and then immersed in a 10% TFA/DCM solution containing 0.1 M pentamethylbenzene (0.05 mL) for 2 minutes. After filtration, the solid phase was washed with NMP (0.05 mL). MeCN (0.25 mL) was then added to the filtrate to prepare an LC sample, and the progress of the reaction was analyzed by LCMS analysis. As a result, the target material A02 was observed as 100%.

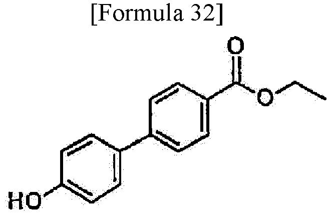

### Compound A02

LRMS: m/z 243 [M+H]⁺
Retention time: 1.085 min (analysis condition FA05-1, 299 nm)

### Example 1-2-2: Synthesis of compound A03-1R

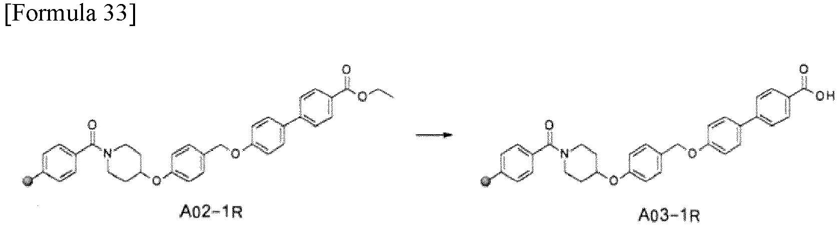

Under a nitrogen atmosphere, to a 10 mL glass vial, compound A02-1R (loading rate 0.200 mmol/g, 1.25 g, 0.250 mmol), THF (7.2 mL), MeOH (0.8 mL), and an aqueous sodium hydroxide solution (5 M, 0.8 mL, 4.0 mmol) were added, and the mixture was shaken at 60°C for 6 hours.

### Solid-phase purification

The whole of the suspension of the reaction solution and the solid phase was transferred onto a filter, and washed 3 times with NMP (20 mL), 3 times with water (20 mL), 3 times with an HOAt/NMP solution (0.2 M, 20 mL), 3 times with NMP (20 mL), 3 times with MeOH (20 mL), and 3 times with DCM (20 mL), and the resulting solid phase was dried overnight under reduced pressure to afford compound A03-1R (loading rate 0.201 mmol/g, 1.16 g, 0.232 mmol).

### Reaction tracking

The suspension (10 mL) of the reaction solution and the solid phase was transferred onto a filter, and washed 3 times with NMP (0.1 mL), 3 times with MeOH (0.1 mL), and 3 times with DCM (0.1 mL), and then immersed in a 10% TFA/DCM solution containing 0.1 M pentamethylbenzene (0.05 mL) for 2 minutes. After filtration, the solid phase was washed with NMP (0.05 mL). MeCN (0.25 mL) was then added to the filtrate to prepare an LC sample, and the progress of the reaction was analyzed by LCMS analysis. As a result, the target material A03 was observed as 100%.

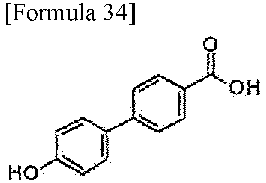

### Compound A03

Maximum wavelength: 293 nm
Retention time: 0.775 min (analysis condition FA05-1, 299 nm)

### Example 1-2-3: Synthesis of compound A02-2R

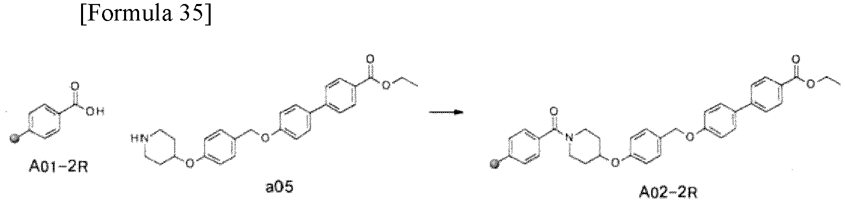

Under a nitrogen atmosphere, to a 200 mL empty column with a filter, Carboxylic Resin (A01-2R) (Chem-Impex, loading rate 1.70 mmol/g, 10.5 g, 17.9 mmol), ethyl 4-[4-[(4-piperidin-4-yloxyphenyl)methoxy]phenyl]benzoate (a05) (1.08 g, 2.51 mmol), HOAt (2.43 g, 17.9 mmol) and NMP (158 mL) were added, and the mixture was shaken at room temperature for 1 hour. Piperidine (0.884 mL, 8.93 mmol) and DIC (2.78 mL, 17.9 mmol) were added, and the mixture was shaken at room temperature for 20 hours. HOAt (4.86 g, 35.7 mmol), DIC (5.56 mL, 35.7 mmol) and piperidine (3.53 mL, 35.7 mmol) were added, and the mixture was shaken at room temperature all night.

### Solid-phase purification

The solid phase was washed 3 times with NMP (150 mL), 3 times with MeOH (150 mL), 3 times with DCM (150 mL) and 3 times with heptane (150 mL), and the resulting solid phase was dried overnight under reduced pressure to afford compound A02-2R (loading rate 0.200 mmol/g, 12.8 g, 2.56 mmol).

A02-2R was washed 3 times with DCM (0.1 mL), and then immersed in a 10% TFA/DCM solution containing 0.05 M pentamethylbenzene (0.1 mL) for 5 minutes. After filtration, NMP (0.1 mL) was added to the filtrate. To 0.1 mL of which, MeCN (0.25 mL) was added to dilute, and LCMS analysis was performed on the diluted solution. As a result, the target material A02 was observed as 100%.

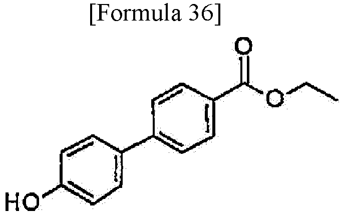

### Compound A02

LRMS: m/z 243 [M+H]⁺
Retention time: 1.081 min (analysis condition FA05-1, 299 nm).

### Example 1-2-4: Synthesis of compound A03-2R

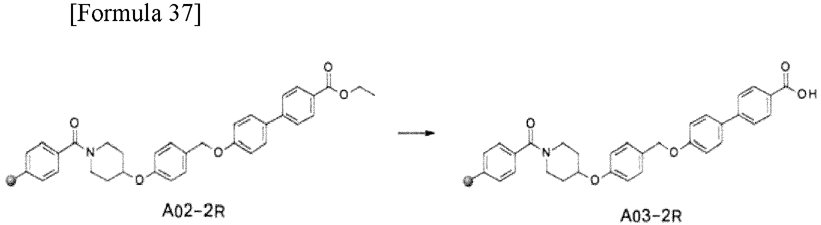

Under a nitrogen atmosphere, to a 200 mL empty column with a filter, compound A02-2R (loading rate 0.200 mmol/g, 12.8 g, 2.56 mmol), NMP (134 mL), and 2-methyl-2-butanol (38.4 mL) were added, and the mixture was shaken at room temperature for 1 hour. To this, an aqueous normal tetrabutyl ammonium hydroxide solution (1 M, 6.40 mL, 6.40 mmol) was added, and the mixture was shaken at room temperature for 14 hours.

### Solid-phase purification

The solid phase was washed once with NMP (190 mL), 3 times with a HOAt/NMP solution (0.1 M, 190 mL), 3 times with NMP (190 mL), 3 times with MeOH (190 mL), 3 times with DCM (190 mL), and 3 times with heptane (190 mL), and the resulting solid phase was dried overnight under reduced pressure to afford compound A03-2R (loading rate 0.201 mmol/g, 12.5 g, 2.50 mmol).

### Reaction tracking

The suspension (12 mL) of the reaction solution and the solid phase was transferred onto a filter, and washed 3 times with NMP (0.1 mL), 3 times with MeOH (0.1 mL), and 3 times with DCM (0.1 mL), and then immersed in a 10% TFA/DCM solution containing 0.02 M pentamethylbenzene (0.05 mL) for 5 minutes. After filtration, the solid phase was washed with DMF (0.05 mL). MeCN (0.25 mL) was then added to the filtrate to prepare an LC sample, and the progress of the reaction was analyzed by LCMS analysis. As a result, the target material A03 was observed as 100%.

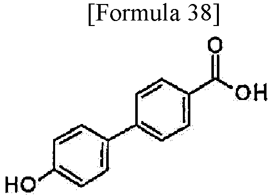

### Compound A03

Maximum wavelength: 294 nm
Retention time: 0.767 min (analysis condition FA05-1, 299 nm).

### Example 1-2-5: Synthesis of compound A02-3R

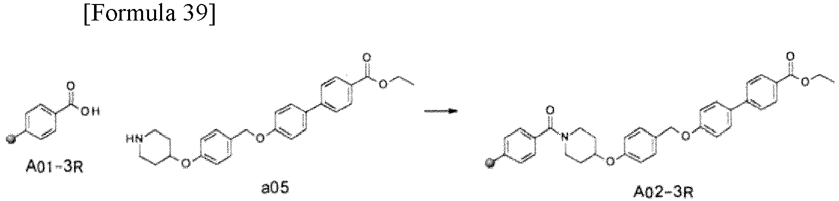

Under a nitrogen atmosphere, to an 800 mL empty column with a filter, Carboxylic Resin (A01-3R) (Rapp Polymer, loading rate 1.70 mmol/g, 40.0 g, 68.0 mmol) and NMP (600 mL) were added, and the mixture was shaken at room temperature for 1 hour. HOAt (9.26 g, 68.0 mmol) and DIC (10.6 mL, 68.0 mmol), ethyl 4-[4-[(4-piperidin-4-yloxyphenyl)methoxy]phenyl]benzoate (a05) (4.13 g, 9.58 mmol) were added, and the mixture was shaken at room temperature for 3.5 hours. HOAt (18.5 g, 136 mmol), DIC (21.2 mL, 136 mmol) and piperidine (16.8 mL, 170 mmol) were added, and the mixture was shaken at room temperature all night.

### Solid-phase purification

The whole of the suspension of the reaction solution and the solid phase was transferred to a 2 L separable flask, and washed 3 times with NMP (800 mL), 3 times with MeOH (800 mL), 3 times with DCM (800 mL), and 3 times with heptane (800 mL), and the resulting solid phase was dried overnight under reduced pressure to afford compound A02-3R (loading rate 0.200 mmol/g, 47.9 g, 9.58 mmol).

### Reaction tracking

The suspension (10 mL) of the reaction solution and the solid phase was transferred onto a filter, and washed 3 times with NMP (0.1 mL), 3 times with MeOH (0.1 mL), and 3 times with DCM (0.1 mL), and then immersed in a 10% TFA/DCM solution containing 0.05 M pentamethylbenzene (0.1 mL) for 5 minutes. After filtration, the solid phase was washed with DMF (0.05 mL). MeCN (0.25 mL) was then added to the filtrate to prepare an LC sample, and the progress of the reaction was analyzed by LCMS analysis. As a result, the target material A02 was observed as 100%.

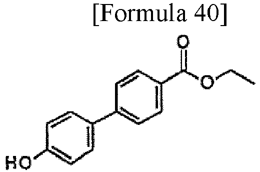

### Compound A02

LRMS: m/z 243 [M+H]⁺
Retention time: 1.081 min (analysis condition FA05-1, 299 nm).

### Example 1-2-6: Synthesis of compound A03-3R

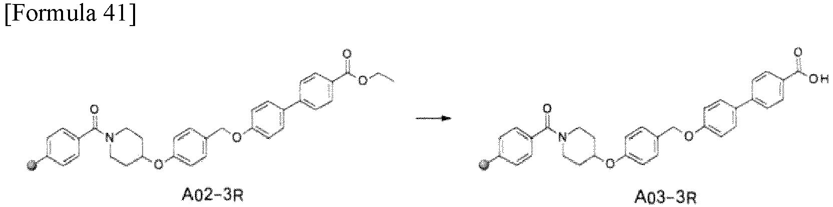

Under a nitrogen atmosphere, to a 2 L separable flask, compound A02-3R (loading rate 0.200 mmol/g, 47.9 g, 9.58 mmol), NMP (503 mL), and 2-methyl-butanol (144 mL) were added, and the mixture was shaken at room temperature for 1 hour. After the suspension was cooled to 5°C, an aqueous normal tetrabutyl ammonium hydroxide solution (1 M, 14.4 mL, 144 mmol) was added dropwise, and the mixture was stirred at 25°C for 3.5 hours. To this, an aqueous normal tetrabutyl ammonium hydroxide solution (1 M, 2.39 mL, 2.39 mmol) was added dropwise, and stirring of the mixture at room temperature for 1 hour was repeated twice.

### Solid-phase purification

The suspension of the reaction solution and the solid phase was washed 3 times with an HOAt/NMP solution (0.1 M, 800 mL), 3 times with NMP (800 mL), 3 times with MeOH (800 mL), 3 times with DCM (800 mL), and 3 times with heptane (800 mL), and the resulting solid phase was dried for 6 days under reduced pressure to afford compound A03-3R (loading rate 0.201 mmol/g, 49.8 g, 10.0 mmol).

A03-3R was washed 3 times with DCM (0.05 mL), and then immersed in a 10% TFA/DCM solution containing 0.02 M pentamethylbenzene (0.05 mL) for 5 minutes. After filtration, DMF (0.05 mL) and MeCN (0.25 mL) were added to dilute, and LCMS analysis was performed on the diluted solution. As a result, the target material A03 was observed as 100%.

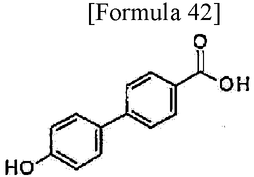

### Compound A03

Maximum wavelength: 294 nm
Retention time: 0.761 min (analysis condition FA05-1, 299 nm).

### Example 1-2-7: Synthesis of compound A02-4R

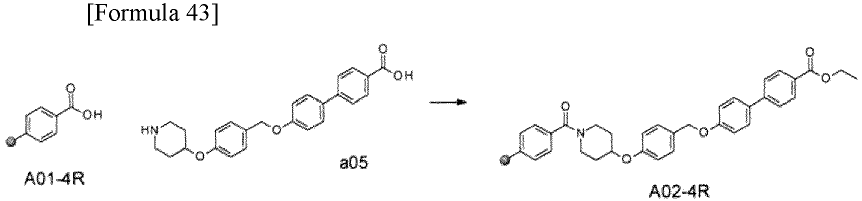

Under a nitrogen atmosphere, to an 800 mL empty column with a filter, Carboxylic Resin (A01-4R) (WATANABE CHEMICAL INDUSTRIES, LTD., loading rate 1.70 mmol/g, 24.7 g, 42.0 mmol) and NMP (371 mL) were added, and the mixture was shaken at room temperature for 45 minutes. HOAt (5.72 g, 42.0 mmol) and DIC (6.55 mL, 42.0 mmol), ethyl 4-[4-[(4-piperidin-4-yloxyphenyl)methoxy]phenyl]benzoate (a05) (2.56 g, 5.93 mmol) were added, and the mixture was shaken at room temperature for 4.5 hours. HOAt (11.5 g, 84.0 mmol), DIC (13.1 mL, 84.0 mmol) and piperidine (10.4 mL, 105 mmol) were added, and the mixture was shaken at room temperature for 21 hours.

### Solid-phase purification

The reaction solution was discharged from the column, and the solid phase was washed 3 times with NMP (500 mL), 3 times with MeOH (500 mL), 3 times with DCM (500 mL) and 3 times with heptane (500 mL), and the resulting solid phase was dried overnight under reduced pressure to afford compound A02-4R (loading rate 0.200 mmol/g, 30.5 g, 6.1 mmol).

A02-4R was washed 3 times with DCM (0.10 mL), and then immersed in a 10% TFA/DCM solution containing 0.2 M pentamethylbenzene (0.05 mL) for 5 minutes. After filtration, DMF (0.05 mL) and MeCN (0.20 mL) were added to dilute, and LCMS analysis was performed on the diluted solution. As a result, the target material A03 was observed as 100%.

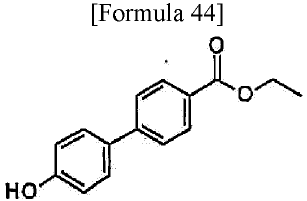

### Compound A02

LRMS: m/z 243 [M+H]⁺
Retention time: 1.071 min (analysis condition FA05-1, 299 nm).

### Example 1-2-8: Synthesis of compound A03-4R

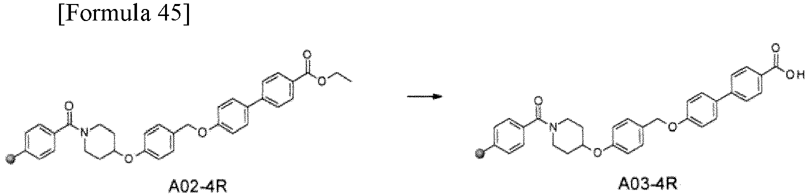

Under a nitrogen atmosphere, to an 800 mL empty column with a filter, compound A02-4R (loading rate 0.200 mmol/g, 29.6 g, 5.92 mmol), NMP (311 mL), and 2-methyl-2-butanol (89 mL) were added, and the mixture was stirred at room temperature for 1 hour. After the suspension was cooled to 5°C, an aqueous normal tetrabutyl ammonium hydroxide solution (1 M, 11.8 mL, 118 mmol) was added dropwise, and the mixture was stirred at 25°C for 7.0 hours.

### Solid-phase purification

The suspension of the reaction solution and the solid phase was washed 3 times with an HOAt/NMP solution (0.1 M, 500 mL), 3 times with NMP (500 mL), 3 times with MeOH (500 mL), 3 times with DCM (500 mL), and 3 times with heptane (500 mL), and the resulting solid phase was dried for 4 days under reduced pressure to afford compound A03-4R (loading rate 0.201 mmol/g, 30.6 g, 6.2 mmol).

A03-4R was washed 3 times with DCM (0.10 mL), and then immersed in a 10% TFA/DCM solution containing 0.2 M pentamethylbenzene (0.05 mL) for 5 minutes. After filtration, DMF (0.05 mL) and MeCN (0.2 mL) were added to dilute, and LCMS analysis was performed on the diluted solution. As a result, the target material A03 was observed as 99.9%.

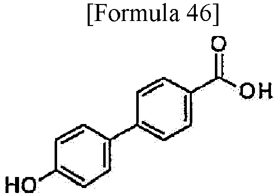

### Compound A03

Maximum wavelength: 294 nm
Retention time: 0.775 min (analysis condition FA05-1, 299 nm)

### Example 1-3-1: Synthesis of compound B01-1R

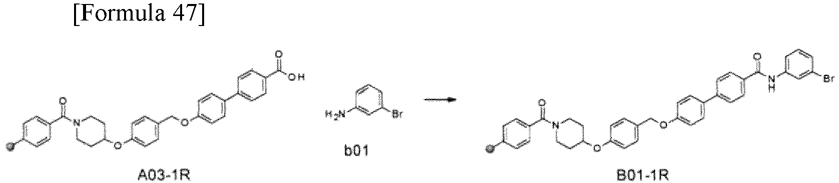

Under a nitrogen atmosphere, to a 5 mL glass vial, compound A03-1R (loading rate 0.201 mmol/, 250 mg, 0.0502 mmol), NMP (3.75 mL), 3-bromoaniline (b01) (20 mL, 0.19 mmol), HOAt (26 mg, 0.19 mmol), and DIC (29 mL, 0.19 mmol) were added, and the mixture was shaken at 60°C for 15 hours. 3-Bromoaniline (b01) (41 mL, 0.38 mmol), HOAt (51 mg, 0.38 mmol), and DIC (58 mL, 0.38 mmol) were added, and the mixture was shaken at 60°C for 4 hours.

### Solid-phase purification

The whole of the suspension of the reaction solution and the solid phase was transferred onto a filter, and washed 3 times with NMP (5 mL), once with NMP/H₂O = 1 : 1 (v/v, 5 mL), 3 times with MeOH (5 mL), and 3 times with DCM (5 mL), and the resulting solid phase was dried overnight under reduced pressure to afford compound B01-1R (loading rate 0.195 mmol/g, 248 mg, 0.0484 mmol).

### Reaction tracking

The suspension (20 mL) of the reaction solution and the solid phase was transferred onto a filter, and washed 3 times with NMP (0.1 mL), 3 times with MeOH (0.1 mL), and 3 times with DCM (0.1 mL), and then immersed in a 10% TFA/DCM solution containing 0.1 M pentamethylbenzene (0.05 mL) for 2 minutes. After filtration, the solid phase was washed with NMP (0.05 mL). MeCN (0.25 mL) was then added to the filtrate to prepare an LC sample, and the progress of the reaction was analyzed by LCMS analysis. As a result, the target material B01 was observed as 100%.

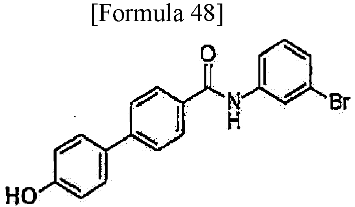

### Compound B01

LRMS: m/z 368, 370 [M+H]⁺
Retention time: 1.166 min (analysis condition FA05-1, 299 nm).

### Example 1-3-2: Synthesis of compound B02-1R

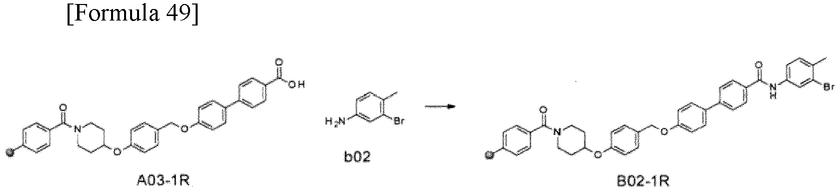

By the same method as compound B01-1R, compound B02-1R (loading rate 0.194 mmol/g) can be synthesized using compound A03-1R (loading rate 0.201 mmol/g) and 3-bromo-4-methylaniline (b02).

B02-1R was immersed in a 10% TFA/DCM solution (0.05 mL) containing 0.1 M pentamethylbenzene for 2 minutes, and NMP (0.05 mL) and MeCN (0.25 mL) were added. The mixture was filtered, and then LCMS analysis of the filtered solution was performed. As a result, the target material B02 was observed as 100%.

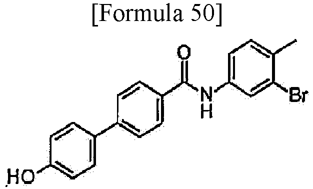

### Compound B02

LRMS: m/z 382, 384 [M+H]⁺
Retention time: 1.221 min (analysis condition FA05-1, 299 nm).

### Example 1-3-3: Synthesis of compound B03-1R

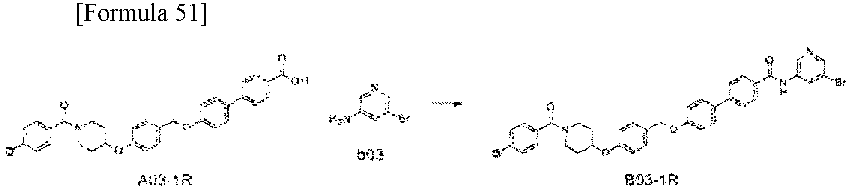

Under a nitrogen atmosphere, to a 20 mL empty column with a filter, compound A03-1R (loading rate 0.201 mmol/g, 1.00 g, 0.201 mmol) and NMP (15 mL) were added, and the mixture was shaken at room temperature for 1 hour. To this, 3-amino-5-bromopyridine (b03) (70 mg, 0.402 mmol), 1-methylimidazole (64 mL, 0.804 mmol), and PyClU (134 mg, 0.402 mmol) were added, and the mixture was shaken at room temperature for 1 hour. To this, 3-amino-5-bromopyridine (b03) (35 mg, 0.201 mmol), 1-methylimidazole (32 mL, 0.402 mmol), and PyClU (67 mg, 0.201 mmol) were added, and the mixture was shaken at room temperature for 20 minutes.

### Solid-phase purification

The solid phase was washed 2 times with NMP (15 mL), 3 times with MeOH (15 mL), and 3 times with DCM (15 mL), and the resulting solid phase was dried overnight under reduced pressure to afford compound B03-1R (loading rate 0.195 mmol/g, 1.04 g, 0.203 mmol).

### Reaction tracking

The suspension (10 mL) of the reaction solution and the solid phase was transferred onto a filter, and washed 3 times with NMP (0.1 mL), 3 times with MeOH (0.1 mL), and 3 times with DCM (0.1 mL), and then immersed in a 10% TFA/DCM solution containing 0.1 M pentamethylbenzene (0.05 mL) for 2 minutes. After filtration, the solid phase was washed with NMP (0.1 mL). To the filtrate (50 mL), MeCN (0.25 mL) was added to prepare an LC sample, and the progress of the reaction was analyzed by LCMS analysis. As a result, the target material B03 was observed as 100%.

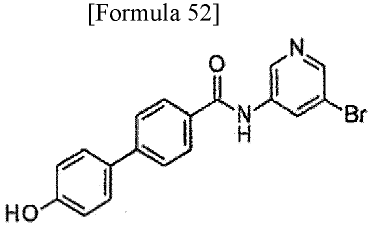

### Compound B03

LRMS: m/z 369, 371 [M+H]⁺
Retention time: 1.014 min (analysis condition FA05-1, 299 nm).

### Example 1-3-4: Synthesis of compound B01-2R

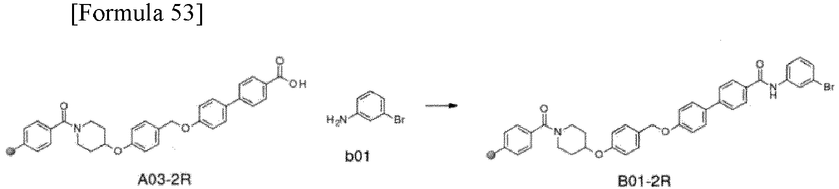

By the same method as compound B03-1R, compound B01-2R (loading rate 0.195 mmol/g) can be synthesized using compound A03-2R (loading rate 0.201 mmol/g) and 3-bromoaniline (b01).

B01-2R was washed 3 times with DCM (0.1 mL), and then immersed in a 10% TFA/DCM solution containing 0.05 M pentamethylbenzene (0.05 mL) for 5 minutes. After filtration, DMF (0.05 mL) and MeCN (0.25 mL) were added to dilute, and LCMS analysis was performed on the diluted solution. As a result, the target material B01 was observed as 99.6%.

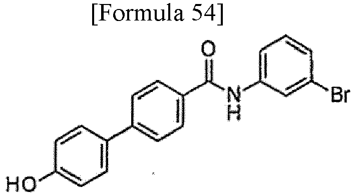

### Compound B01

LRMS: m/z 368, 370 [M+H]⁺
Retention time: 1.161 min (analysis condition FA05-1, 299 nm).

### Example 1-3-5: Synthesis of compound B02-2R

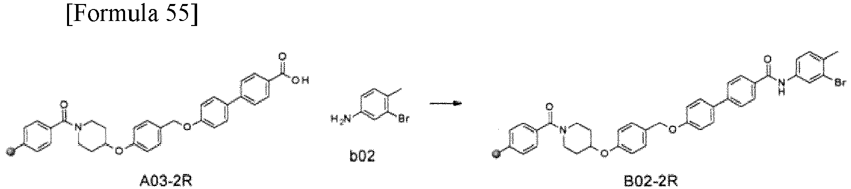

By the same method as compound B03-1R, compound B02-2R (loading rate 0.194 mmol/g) can be synthesized using compound A03-2R (loading rate 0.201 mmol/g) and 3-bromo-4-methylaniline (b02).

B02-2R was washed 3 times with DCM (0.1 mL), and then immersed in a 10% TFA/DCM solution containing 0.05 M pentamethylbenzene (0.05 mL) for 5 minutes. After filtration, DMF (0.05 mL) and MeCN (0.25 mL) were added to dilute, and LCMS analysis was performed on the diluted solution. As a result, the target material B02 was observed as 100%.

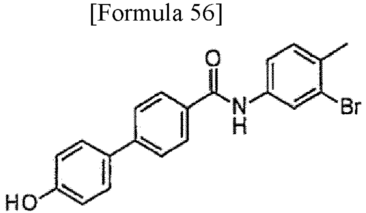

### Compound B02

LRMS: m/z 382, 384 [M+H]⁺
Retention time: 1.220 min (analysis condition FA05-1, 299 nm).

### Example 1-3-6: Synthesis of compound B04-3R

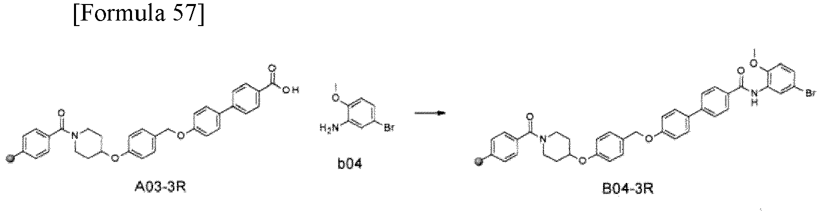

By the same method as compound B03-1R, compound B04-3R (loading rate 0.201 mmol/g) can be synthesized using compound A03-3R (loading rate 0.194 mmol/g) and 5-bromo-2-methoxy-aniline (b04).

B04-3R was washed 3 times with DCM (0.1 mL), and then immersed in a 10% TFA/DCM solution containing 0.05 M pentamethylbenzene (0.05 mL) for 5 minutes. After filtration, DMF (0.05 mL) and MeCN (0.25 mL) were added to dilute, and LCMS analysis was performed on the diluted solution. As a result, the target material B04 was observed as 99.5%.

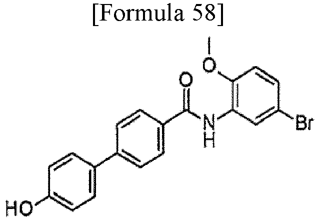

### Compound B04

LRMS: m/z 398, 400 [M+H]⁺
Retention time: 1.228 min (analysis condition FA05-1, 299 nm).

### Example 1-3-7: Synthesis of compound B05-2R

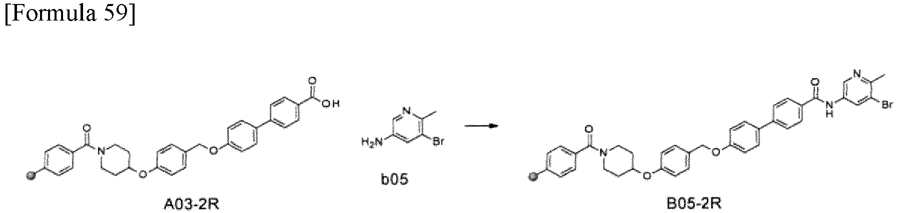

By the same method as compound B03-1R, compound B05-2R (loading rate 0.194 mmol/g) can be synthesized using compound A03-2R (loading rate 0.201 mmol/g) and 5-bromo-6-methylpyridin-3-amine (b05).

B05-2R was washed 3 times with DCM (0.1 mL), and then immersed in a 10% TFA/DCM solution containing 0.05 M pentamethylbenzene (0.05 mL) for 5 minutes. After filtration, DMF (0.05 mL) and MeCN (0.25 mL) were added to dilute, and LCMS analysis was performed on the diluted solution. As a result, the target material B05 was observed as 99.2%.

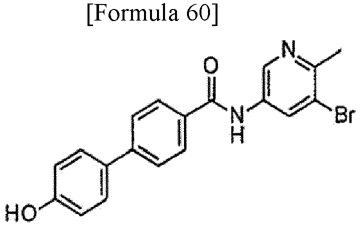

### Compound B05

LRMS: m/z 383, 385 [M+H]⁺
Retention time: 1.029 min (analysis condition FA05-1, 299 nm).

### Example 1-3-8: Synthesis of compound B06-2R

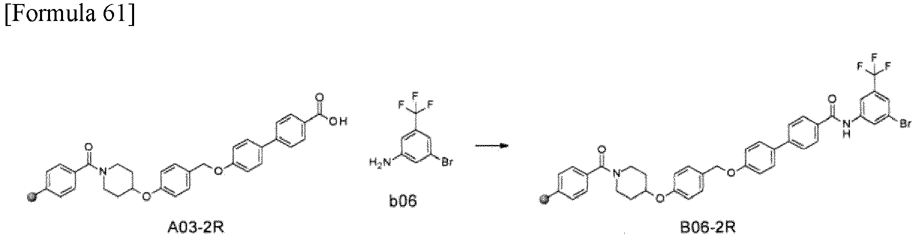

By the same method as compound B03-1R, compound B06-2R (loading rate 0.192 mmol/g) can be synthesized using compound A03-2R (loading rate 0.201 mmol/g) and 5-bromo-2-methoxy-aniline (b06).

B06-2R was washed 3 times with DCM (0.1 mL), and then immersed in a 10% TFA/DCM solution containing 0.05 M pentamethylbenzene (0.05 mL) for 5 minutes. After filtration, DMF (0.05 mL) and MeCN (0.25 mL) were added to dilute, and LCMS analysis was performed on the diluted solution. As a result, the target material B06 was observed as 99.6%.

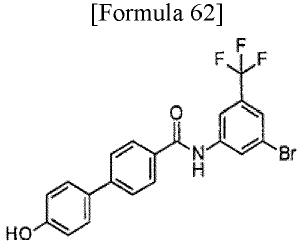

### Compound B06

LRMS: m/z 436, 438 [M+H]⁺
Retention time: 1.335 min (analysis condition FA05-1, 299 nm).

### Example 1-3-9: Synthesis of compound B07-1R

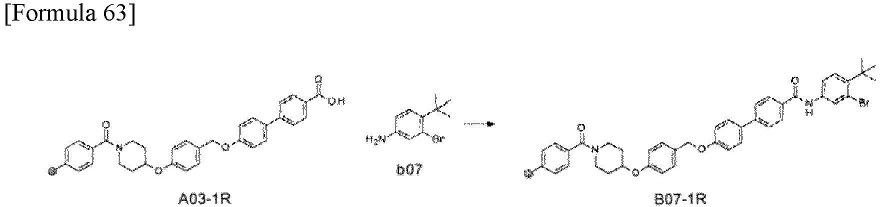

By the same method as compound B03-1R, compound B07-1R (loading rate 0.193 mmol/g) can be synthesized using compound A03-1R (loading rate 0.201 mmol/g) and 3-bromo-4-(tert-butyl)aniline (b07).

### Reaction tracking

The suspension (10 mL) of the reaction solution and the solid phase was transferred onto a filter, and washed 3 times with NMP (0.1 mL), 3 times with MeOH (0.1 mL), and 3 times with DCM (0.1 mL), and then immersed in a 10% TFA/DCM solution containing 0.1 M pentamethylbenzene (0.05 mL) for 2 minutes. After filtration, the solid phase was washed with NMP (0.1 mL). To the filtrate (0.05 mL), MeCN (0.25 mL) was added to dilute, and LCMS analysis was performed on the diluted solution. As a result, the target material B07 was observed as 96%.

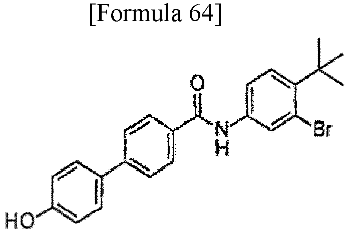

### Compound B07

LRMS: m/z 424, 426 [M+H]⁺
Retention time: 1.396 min (analysis condition FA05-1, 299 nm).

### Example 1-3-10: Synthesis of compound B08-3R

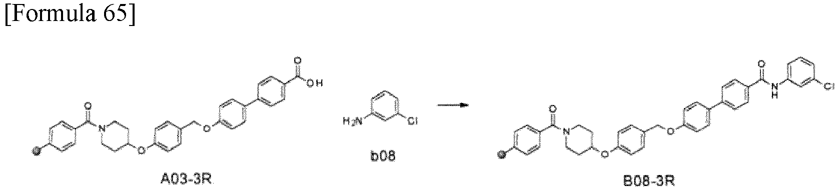

By the same method as compound B03-1R, compound B08-3R (loading rate 0.197 mmol/g) can be synthesized using compound A03-3R (loading rate 0.201 mmol/g) and 3-chloroaniline (b08).

B08-3R was washed 3 times with DCM (0.1 mL), and then immersed in a 10% TFA/DCM solution containing 0.05 M pentamethylbenzene (0.05 mL) for 5 minutes. After filtration, DMF (0.05 mL) and MeCN (0.25 mL) were added to dilute, and LCMS analysis was performed on the diluted solution. As a result, the target material B08 was observed as 100%.

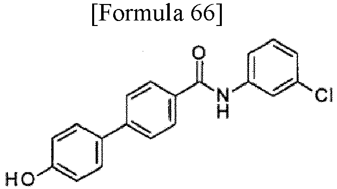

### Compound B08

LRMS: m/z 324 [M+H]⁺
Retention time: 1.141 min (analysis condition FA05-1, 299 nm).

### Example 1-3-11: Synthesis of compound B09-3R

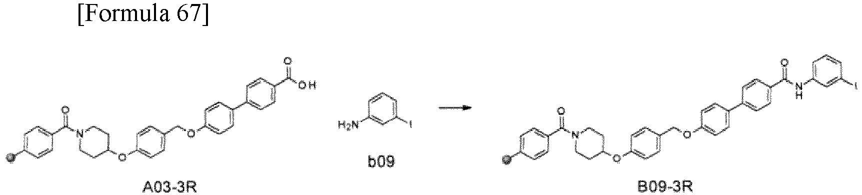

By the same method as compound B03-1R, compound B09-3R (loading rate 0.193 mmol/g) can be synthesized using compound A03-3R (loading rate 0.201 mmol/g) and 3-iodoaniline (b09).

B09-3R was washed 3 times with DCM (0.1 mL), and then immersed in a 10% TFA/DCM solution containing 0.05 M pentamethylbenzene (0.05 mL) for 5 minutes. After filtration, DMF (0.05 mL) and MeCN (0.25 mL) were added to dilute, and LCMS analysis was performed on the diluted solution. As a result, the target material B09 was observed as 100%.

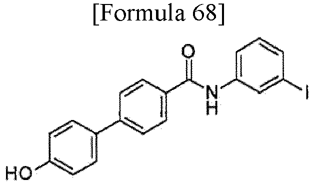

### Compound B09

LRMS: m/z 416 [M+H]⁺
Retention time: 1.192 min (analysis condition FA05-1, 299 nm).

### Example 1-3-12: Synthesis of compound B10-3R

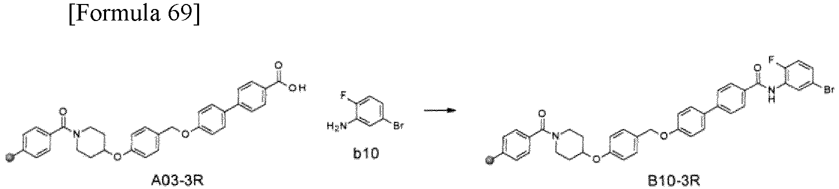

By the same method as compound B03-1R, compound B10-3R (loading rate 0.194 mmol/g) can be synthesized using compound A03-3R (loading rate 0.201 mmol/g) and 5-bromo-2-fluoroaniline (b10).

B10-3R was washed 3 times with DCM (0.1 mL), and then immersed in a 10% TFA/DCM solution containing 0.05 M pentamethylbenzene (0.05 mL) for 5 minutes. After filtration, DMF (0.05 mL) and MeCN (0.25 mL) were added to dilute, and LCMS analysis was performed on the diluted solution. As a result, the target material B10 was observed as 97.2%.

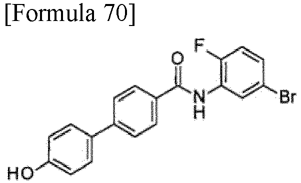

### Compound B10

LRMS: m/z 386, 388 [M+H]⁺
Retention time: 1.164 min (analysis condition FA05-1, 299 nm)

### Example 1-3-13: Synthesis of compound B11-3R

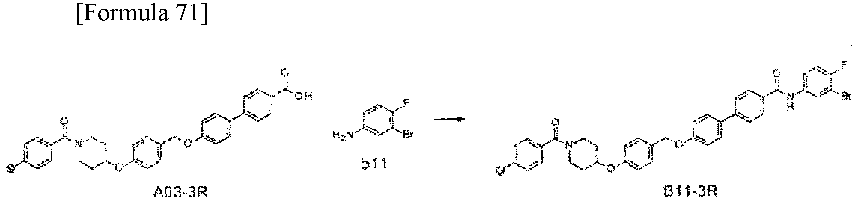

By the same method as compound B03-1R, compound B11-3R (loading rate 0.194 mmol/g) can be synthesized using compound A03-3R (loading rate 0.201 mmol/g) and 5-bromo-4-fluoroaniline (b11).

B 11-3R was washed 3 times with DCM (0.1 mL), and then immersed in a 10% TFA/DCM solution containing 0.05 M pentamethylbenzene (0.05 mL) for 5 minutes. After filtration, DMF (0.05 mL) and MeCN (0.25 mL) were added to dilute, and LCMS analysis was performed on the diluted solution. As a result, the target material B11 was observed as 99.1%.

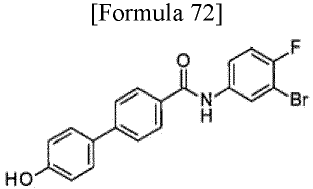

### Compound B11

LRMS: m/z 386, 388 [M+H]⁺
Retention time: 1.165 min (analysis condition FA05-1, 299 nm).

### Example 1-3-14: Synthesis of compound B12-3R

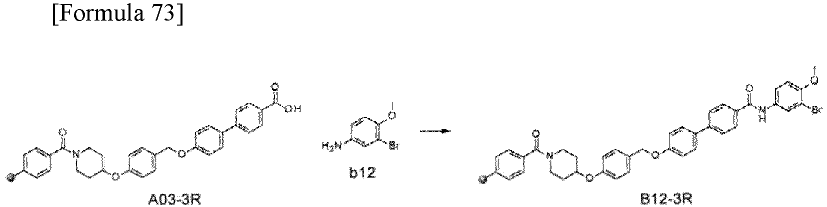

By the same method as compound B03-1R, compound B12-3R (loading rate 0.194 mmol/g) can be synthesized using compound A03-3R (loading rate 0.201 mmol/g) and 5-bromo-4-methoxy-aniline (b12).

B12-3R was washed 3 times with DCM (0.1 mL), and then immersed in a 10% TFA/DCM solution containing 0.05 M pentamethylbenzene (0.05 mL) for 5 minutes. After filtration, DMF (0.05 mL) and MeCN (0.25 mL) were added to dilute, and LCMS analysis was performed on the diluted solution. As a result, the target material B12 was observed as 100%.

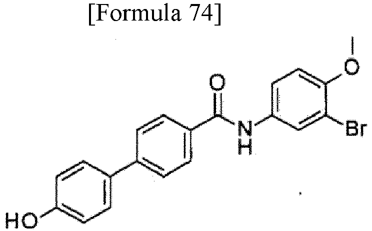

### Compound B12

LRMS: m/z 398, 400 [M+H]⁺
Retention time: 1.093 min (analysis condition FA05-1, 299 nm).

### Example 1-3-15: Synthesis of compound B13-3R

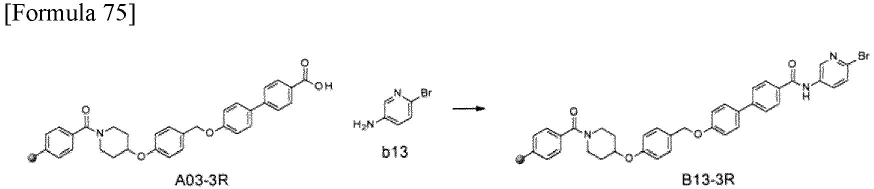

By the same method as compound B03-1R, compound B13-3R (loading rate 0.195 mmol/g) can be synthesized using compound A03-3R (loading rate 0.201 mmol/g) and 6-bromopyridin-3-amine (b13).

B13-3R was washed 3 times with DCM (0.1 mL), and then immersed in a 10% TFA/DCM solution containing 0.05 M pentamethylbenzene (0.05 mL) for 5 minutes. After filtration, DMF (0.05 mL) and MeCN (0.25 mL) were added to dilute, and LCMS analysis was performed on the diluted solution. As a result, the target material B13 was observed as 99.6%.

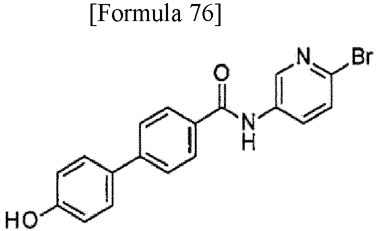

### Compound B13

LRMS: m/z 369, 371 [M+H]⁺
Retention time: 1.011 min (analysis condition FA05-1, 299 nm).

### Example 1-3-16: Synthesis of compound B14-3R

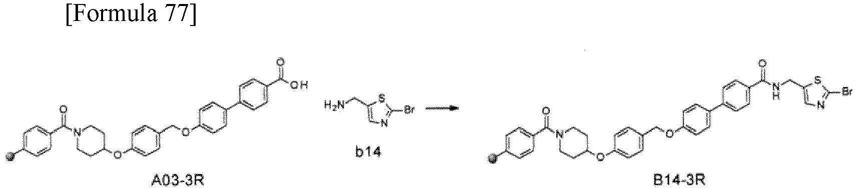

By the same method as compound B03-1R, compound B 14-3R (loading rate 0.194 mmol/g) can be synthesized using compound A03-3R (loading rate 0.201 mmol/g) and (2-bromothiazol-5-yl)methanamine (b14).

B14-3R was washed 3 times with DCM (0.1 mL), and then immersed in a 10% TFA/DCM solution containing 0.05 M pentamethylbenzene (0.05 mL) for 5 minutes. After filtration, DMF (0.05 mL) and MeCN (0.25 mL) were added to dilute, and LCMS analysis was performed on the diluted solution. As a result, the target material B14 was observed as 97.8%.

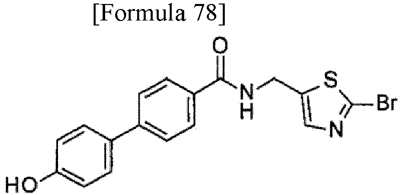

### Compound B14

LRMS: m/z 389, 391 [M+H]⁺
Retention time: 0.925 min (analysis condition FA05-1, 299 nm).

### Example 1-3-17: Synthesis of compound B15-1R

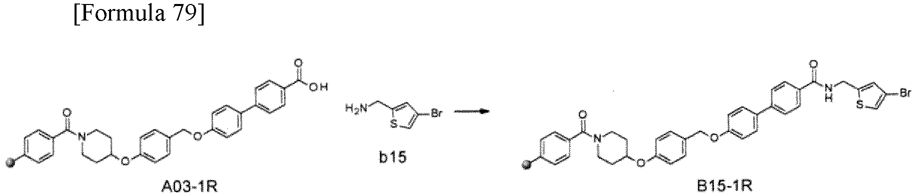

By the same method as compound B03-1R, compound B15-1R (loading rate 0.194 mmol/g) can be synthesized using compound A03-3R (loading rate 0.201 mmol/g) and (4-bromothiophen-2-yl)methanamine (b 15).

The suspension (10 mL) of the reaction solution and the solid phase was transferred onto a filter, and washed 3 times with NMP (0.1 mL), 3 times with MeOH (0.1 mL), and 3 times with DCM (0.1 mL), and then immersed in a 10% TFA/DCM solution containing 0.1 M pentamethylbenzene (0.05 mL) for 2 minutes. After filtration, the solid phase was washed with NMP (0.1 mL). To the filtrate (0.05 mL), MeCN (0.25 mL) was added to dilute, and LCMS analysis was performed on the diluted solution. As a result, the target material B15 was observed as 100%.

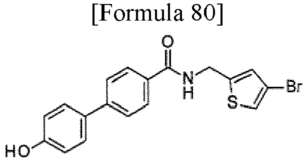

### Compound B15

LRMS: m/z 388, 390 [M+H]⁺
Retention time: 1.061 min (analysis condition FA05-1, 299 nm).

### Example 1-3-18: Synthesis of compound H04-3R

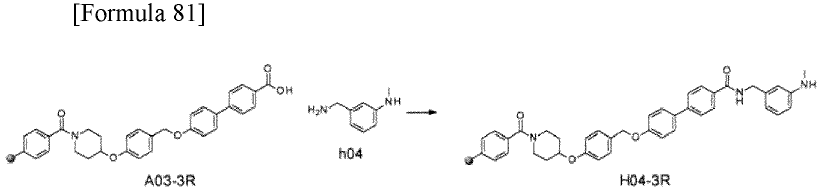

Under a nitrogen atmosphere, to a 100 mL glass vial, compound A03-3R (loading rate 0.201 mmol/g, 5.00 g, 1.01 mmol) and NMP (60 mL) were added, and the mixture was shaken at room temperature for 1 hour. To this, 3-(aminomethyl)-N-methylaniline (h04) (0.411 g, 3.02 mmol), HOAt (0.410 g, 3.02 mmol), and DIC (0.467 mL, 3.02 mmol) were added, and the mixture was shaken at room temperature for 96 hours.

The whole of the suspension of the reaction solution and the solid phase was transferred onto a filter, and washed 3 times with NMP (100 mL), 3 times with MeOH (100 mL), 3 times with DCM (100 mL), and 3 times with heptane (100 mL) to afford compound H04-3R (loading rate 0.196 mmol/g, 5.45 g).

H04-3R was washed 3 times with DCM (0.1 mL), and then immersed in a 10% TFA/DCM solution containing 0.02 M pentamethylbenzene (0.05 mL) for 5 minutes. After filtration, DMF (0.05 mL) and MeCN (0.25 mL) were added to dilute, and LCMS analysis was performed on the diluted solution. As a result, the target material H04 was observed as 97.2%.

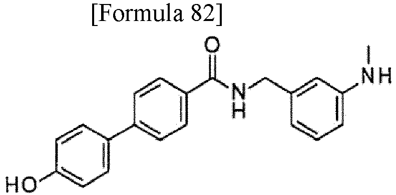

### Compound H04

LRMS: m/z 333 [M+H]⁺
Retention time: 0.675 min (analysis condition FA05-1, 299 nm).

### Example 1-3-19: Synthesis of compound H01-4R

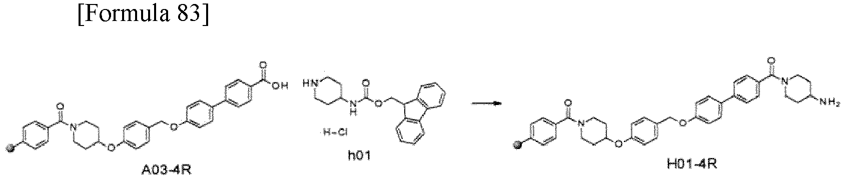

Under a nitrogen atmosphere, to a 50 mL empty column with a filter, compound A03-4R (loading rate 0.201 mmol/g, 2.5 g, 0.50 mmol) and DCM (37.5 mL) were added, and the mixture was shaken at room temperature for 1 hour. To this, 4-(Fmoc-amino)piperidine hydrochloride (h01, CAS No: 221352-86-9) (0.361 g, 1.00 mmol), DIPEA (0.175 mL, 1.00 mmol), NMI (0.160 mL, 2.01 mmol) and PipClU (0.361 g, 1.00 mmol) were added, and the mixture was shaken at room temperature for 6 hours.

The solid phase was washed 3 times with NMP (50 mL), 3 times with NMP/H₂O = 1/1 (50 mL), and 3 times with NMP (50 mL). A 20% piperidine/DMF solution (50 mL) was added to the column, the mixture was shaken at room temperature for 2 hours. After discharge of the solution, the residue was washed 3 times with NMP (50 mL), 3 times with MeOH (50 mL), 3 times with DCM (50 mL) and 3 times with heptane (50 mL) to afford compound H01-4R (loading rate 0.198 mmol/g, 3.0 g).

H01-4R was washed 3 times with DCM (0.1 mL), and then immersed in a 10% TFA/DCM solution containing 0.2 M pentamethylbenzene (0.05 mL) for 5 minutes. After filtration, DMF (0.05 mL) and MeCN (0.20 mL) were added to dilute, and LCMS analysis was performed on the diluted solution. As a result, the target material H01 was observed as 98.0%.

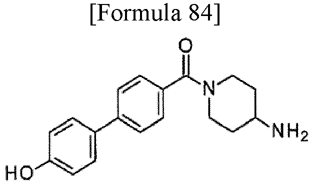

### Compound H01

LRMS: m/z 297 [M+H]⁺
Retention time: 0.551 min (analysis condition FA05-1, 299 nm).

### Example 1-3-20: Synthesis of compound H02-4R

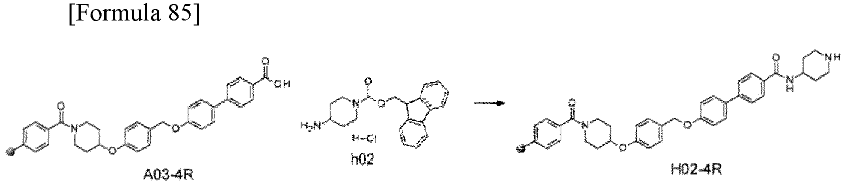

Under a nitrogen atmosphere, to a 100 mL empty column with a filter, compound A03-4R (loading rate 0.201 mmol/g, 6.5 g, 1.31 mmol) and DCM (98.0 mL) were added, and the mixture was shaken at room temperature for 1 hour. To this, 4-amino-1-N-Fmoc-piperidine hydrochloride (h02, CAS No: 811841-89-1) (0.938 g, 2.61 mmol), DIEA (0.455 mL, 2.61 mmol), NMI (0.417 mL, 5.23 mmol) and PipClU (0.943 g, 2.61 mmol) were added, and the mixture was shaken at room temperature for 2.5 hours.

The solid phase was washed 3 times with NMP (100 mL), 3 times with NMP/H₂O = 1/1 (100 mL), and 3 times with NMP (100 mL). A 20% piperidine/DMF solution (100 mL) was added to the column, and the mixture was shaken at room temperature for 1 hour. After discharge of the solution, the residue was washed 3 times with NMP (100 mL), 3 times with MeOH (100 mL), 3 times with DCM (100 mL) and 3 times with heptane (100 mL) to afford compound H02-4R (loading rate 0.198 mmol/g, 7.3 g).

H02-4R was washed 3 times with DCM (0.1 mL), and then immersed in a 10% TFA/DCM solution containing 0.2 M pentamethylbenzene (0.05 mL) for 5 minutes. After filtration, DMF (0.05 mL) and MeCN (0.20 mL) were added to dilute, and LCMS analysis was performed on the diluted solution. As a result, the target material H01 was observed as 97.4%.

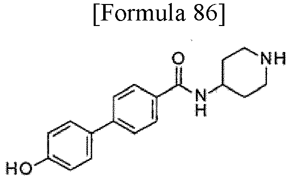

### Compound H02

LRMS: m/z 297 [M+H]⁺
Retention time: 0.561 min (analysis condition FA05-1, 299 nm).

### Example 1-3-21: Synthesis of compound H03-4R

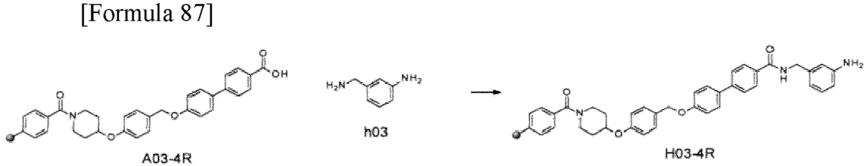

Under a nitrogen atmosphere, to a 50 mL empty column with a filter, compound A03-4R (loading rate 0.201 mmol/g, 2.5 g, 0.50 mmol) and DCM (37.5 mL) were added, and the mixture was shaken at room temperature for 1 hour. To this, 3-(aminomethyl)aniline (h03) (0.307 g, 2.51 mmol), DIC (0.389 mL, 2.51 mmol) and HOAt (0.342 g, 2.51 mmol) were added and the mixture was shaken at room temperature for 18 hours. To this, 3-(aminomethyl)aniline (h03) (0.123 g, 1.00 mmol), DIC (0.156 mL, 1.00 mmol) and HOAt (0.137 g, 1.00 mmol) were added and the mixture was shaken at room temperature for 4 hours.

The solid phase was washed 3 times with NMP (50 mL), 3 times with NMP/H₂O =/1 (50 mL), 3 times with NMP (50 mL), 3 times with MeOH (50 mL), 3 times with DCM (50 mL), and 3 times with heptane (50 mL) to afford compound H03-4R (loading rate 0.197 mmol/g, 2.9 g).

H03-4R was washed 3 times with DCM (0.1 mL), and then immersed in a 10% TFA/DCM solution containing 0.2 M pentamethylbenzene (0.05 mL) for 5 minutes. After filtration, DMF (0.05 mL) and MeCN (0.20 mL) were added to dilute, and LCMS analysis was performed on the diluted solution. As a result, the target material H03 was observed as 98.6%.

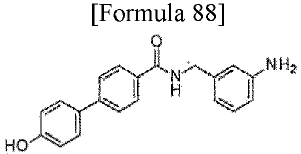

### Compound H03

LRMS: m/z 319 [M+H]⁺
Retention time: 0.641 min (analysis condition FA05-1, 299 nm).

### Example 2

### Example 2-1: Study of various solvents in hydroxylation reaction of aryl bromide supported on solid phase

### Example 2-1-1: Experiment of comparing solvents using AdBrettPhos Pd G6 Br as palladium catalyst and BTMG as base in hydroxylation reaction of compound B01-1R

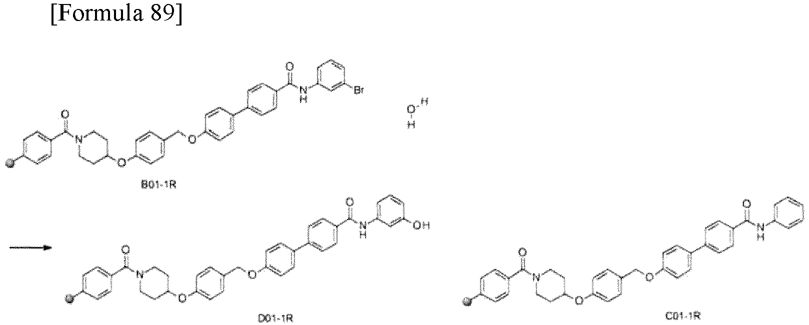

### Experimental procedures

Under a nitrogen atmosphere, to a 0.6 mL glass vial, compound B01-1R (0.195 mmol/g, 20 mg, 0.0039 mmol), a solvent (0.4 mL) and water (0.010 mL) were added, and the mixture was shaken at room temperature for 1 hour. AdBrettPhos Pd G6 Br (0.8 mg, 0.00078 mmol) and BTMG (11.7 mL, 0.059 mmol) were added and the mixture was shaken at 80°C for 0.5 to 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and the progress of the reaction was analyzed by LCMS analysis. The results (UV area%) are as shown in Table 2-1-1.

**[Table 6]**

| [Table 2-1-1] | | | | | | | |
|---|---|---|---|---|---|---|---|
| Solvent | Reaction time (hour) | Target material D01 | Starting material B01 | H-product C01 | Impurity P01 | Impurity Q01 | Total of other impurities |
| DMPr | 0.5 | 94.1 | | 3.7 | | | 2.2 |
| DEAc | 0.5 | 92.9 | 5.1 | 2.1 | | | |
| | 2 | 95.6 | | 2.5 | | | 1.9 |
| DEPr | 0.5 | 69.5 | 27.3 | 3.2 | | | |
| | 2 | 92.6 | 3.1 | 4.4 | | | |
| DMAc/Toluene =1/1 (v/v) | 0.5 | 88.5 | | 10.3 | | | 1.3 |
| DMAc/Toluene =7/1 (v/v) | 0.5 | 88.9 | | 9.6 | | | 1.5 |
| DMAc | 0.5 | 87.9 | | 9.5 | | | 2.6 |
| DMF | 0.5 | 87.3 | | 4.9 | | 3.2 | 4.6 |
| DMI | 0.5 | 66.9 | | 10.9 | | | 22.1 |
| DMPU | 0.5 | 35.3 | | 59.2 | | | 5.6 |
| NMP | 0.5 | 93.0 | | 3.2 | 3.8 | | 0.0 |
| DMOc | 2 | 37.6 | | 58.1 | | | 4.4 |
| THF | 0.5 | 36.6 | 57.4 | 2.7 | | | 3.3 |
| | 2 | 64.4 | 29.2 | 3.1 | | | 3.2 |
| | 24 | 88.4 | | 4.1 | | | 7.5 |
| Toluene | 0.5 | 58.1 | 27.9 | 4.0 | | | 10.0 |
| | 2 | 82.7 | 2.2 | 5.7 | | | 9.3 |
| | 24 | 76.0 | | 7.0 | | | 17.0 |

From the above results, it was confirmed that, when DMPr, DEAc, and DEPr are used, compared to the use of conventionally used solvents, the conversion rate is consistently higher and the generation of a by-product (H-product), which is generated by the substitution of the leaving group of the substrate with a hydrogen atom, and other impurities are reduced, giving a high yield. When DMAc, which is conventionally used as an amide-based solvent, or DMI and DMPU, which are urea-based solvents having excellent substrate solubility similar to the amide-based solvents, was used, H-product (C01) was often produced, and the yield of the target material substance was low. When NMP, which is conventionally used as an amide-based solvent, was used, impurity P01 with 83 m/z higher than the H-product was observed, although the yield of the target material was high. When NMP is used, even in the case of other substrates, the impurity with 83 m/z higher than the H-product is generated, and the yield of the target material is reduced because this impurity is more generated depending on the substrate (Example 2-1-2, and Example 21-3 described below). When DMF was used, an impurity Q01 having 57 higher m/z than the H-product was observed, making separation from the target material D01 difficult and the yield of the target material low. In addition, when Toluene was added to DMAc to confirm the effect of improving the fat solubility of the entire solvent, there was no tendency to improve compared to DMAc. When THF and Toluene were used, the reaction was slow, the generation of impurities was high, and the yield of the target material was low. In addition, when DMOc, which is an amide-based solvent and has been reported as an additive to solvents in flow reaction of C-N coupling reactions (Angew. Chem. Int. Ed. 2016, 55, 2531-2535.) was used, the production of H-product (C01) was high and the yield was low.

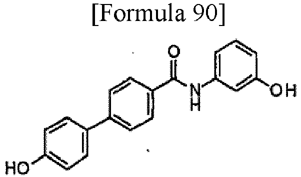

### Compound D01

LRMS: m/z 306 [M+H]⁺
Retention time: 0.864 min (analysis condition FA05-1, 299 nm)
Retention time: 1.660 min (analysis condition FA05-long, 299 nm).

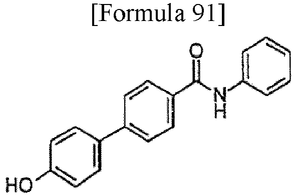

### Compound C01

LRMS: m/z 290 [M+H]⁺
Retention time: 1.017 min (analysis condition FA05-1, 299 nm).
Retention time: 2.081 min (analysis condition FA05-long, 299 nm).

### Compound P01 (structure undetermined)

LRMS: m/z 373 [M+H]⁺
Retention time: 0.923 min (analysis condition FA05-1, 299 nm).

### Compound Q01 (structure undetermined)

LRMS: m/z 347 [M+H]⁺
Retention time: 1.744 min (analysis condition FA05-long, 299 nm).

### Example 2-1-2: Experiment of comparing solvents using AdBrettPhos Pd G6 Br as palladium catalyst and BTMG as base in hydroxylation reaction of compound B03-1R

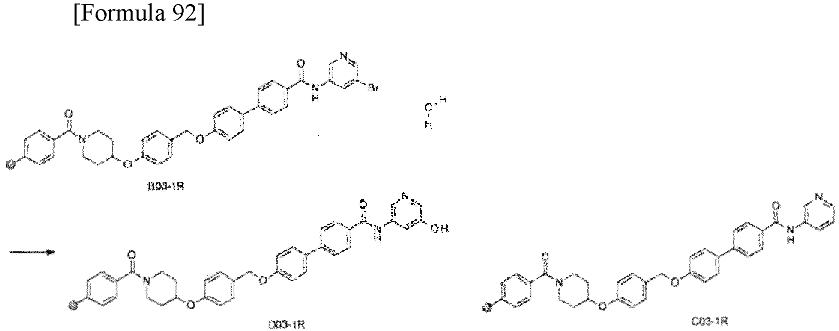

### Experimental procedures

Under a nitrogen atmosphere, to a 0.6 mL glass vial, compound B03-1R (0.195 mmol/g, 20 mg, 0.0039 mmol), a solvent (0.4 mL) and water (0.010 mL) were added, and the mixture was shaken at room temperature for 1 hour. AdBrettPhos Pd G6 Br (0.8 mg, 0.00078 mmol) and BTMG (11.7 mL, 0.059 mmol) were added and the mixture was shaken at 80°C for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and the progress of the reaction was analyzed by LCMS analysis. The results (UV area%) are as shown in Table 2-1-2.

**[Table 7]**

| [Table 2-1-2] | | | | | | |
|---|---|---|---|---|---|---|
| Solvent | Reaction time (hour) | Target material D03 | Starting material B03 | H-product C03 | Impurity P03 | Total of other impurities |
| DMPr | 24 | 88.7 | | 4.9 | | 6.4 |
| DEAc | 24 | 93.8 | | 2.7 | | 3.5 |
| DMAc | 24 | 78.0 | | 14.9 | | 7.0 |
| NMP | 24 | 77.4 | | 7.0 | 10.8 | 4.8 |

From the above results, it was confirmed that, when DMPr and DEAc are used, the conversion rate is consistently high, and the generation of H-product and other impurities, which has been problems, are reduced, and high yields are provided. Meanwhile, when DMAc was used, the production of H-product (C03) was high, and when NMP was used, impurity P03 with 83 m/z higher than the H-product was observed, and the yield of the target material was low.

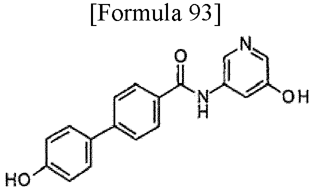

### Compound D03

LRMS: m/z 307 [M+H]⁺
Retention time: 1.296 min (analysis condition RPAmideTFA05-long, 299 nm).

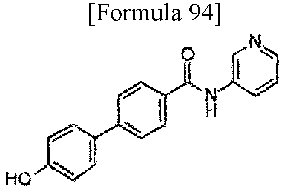

### Compound C03

LRMS: m/z 291 [M+H]⁺
Retention time: 1.211 min (analysis condition RPAmideTFA05-long, 299 nm).

### Compound P03 (structure undetermined)

LRMS: m/z 374 [M+H]⁺
Retention time: 1.425 min (analysis condition RPAmideTFA05-long, 299 nm).

### Example 2-1-3: Experiment of comparing solvents using AdBrettPhos Pd G6 Br as palladium catalyst and P1tBu as base in hydroxylation reaction of compound B04-3R

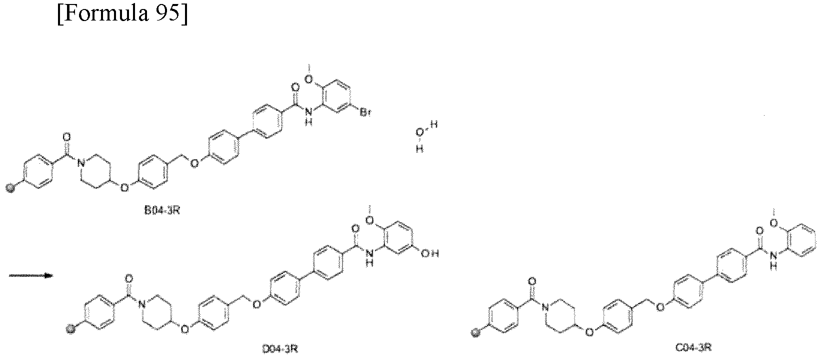

### Experimental procedures

Under a nitrogen atmosphere, to a 0.6 mL glass vial, compound B04-3R (0.194 mmol/g, 20 mg, 0.0039 mmol), a solvent (0.32 mL) and water (0.080 mL) were added, and the mixture was shaken at room temperature for 1 hour. AdBrettPhos Pd G6 Br (0.8 mg, 0.00078 mmol) and P1tBu (14.8 mL, 0.058 mmol) were added and the mixture was shaken at 80°C for 0.5 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and the progress of the reaction was analyzed by LCMS analysis. The results (UV area%) are as shown in Table 2-1-3.

**[Table 8]**

| [Table 2-1-3] | | | | | | |
|---|---|---|---|---|---|---|
| Solvent | Reaction time (hour) | Target material D04 | Starting material B04 | H-product C04 | Impurity P04 | Total of other impurities |
| DMPr | 0.5 | 93.5 | | 2.3 | | 4.3 |
| DEAc | 0.5 | 94.6 | | 2.6 | | 2.8 |
| DMAc | 0.5 | 76.6 | 17.1 | 1.6 | | 4.7 |
| DMF | 0.5 | 32.5 | 53.0 | 10.4 | | 4.2 |
| NMP | 0.5 | 89.0 | | 3.5 | 5.1 | 2.4 |

From the above results, it was confirmed that, when DMPr and DEAc are used, the conversion rate is consistently high, and the generation of H-product and other impurities, which has been problems, are reduced, and high yields are provided. Meanwhile, when DMAc was used, the progress of the reaction was slow. When DMF was used, H-product (C04) production was high as the reaction progressed slowly, and the yield of the target material was low. When NMP was used, the H-product and impurity P04 with 83 m/z higher than H-product (C04) were observed, and the yield of the target material was low.

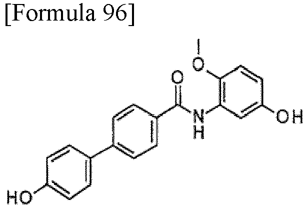

### Compound D04

LRMS: m/z 336 [M+H]⁺
Retention time: 0.905 min (analysis condition FA05-2, 299 nm).

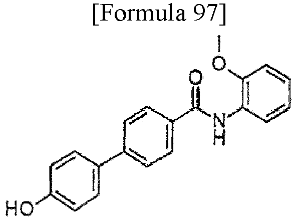

### Compound C04

LRMS: m/z 320 [M+H]⁺
Retention time: 1.077 min (analysis condition FA05-2, 299 nm).

### Compound P04 (structure undetermined)

LRMS: m/z 403 [M+H]⁺
Retention time: 0.935 min (analysis condition FA05-2, 299 nm).

### Example 2-2: Study of various palladium catalysts in hydroxylation reaction of aryl bromide supported on solid phase

### Example 2-2-1: Experiment of studying palladium catalysts using P2tBu as base and DMPr as solvent in hydroxylation reaction of compound B02-2R

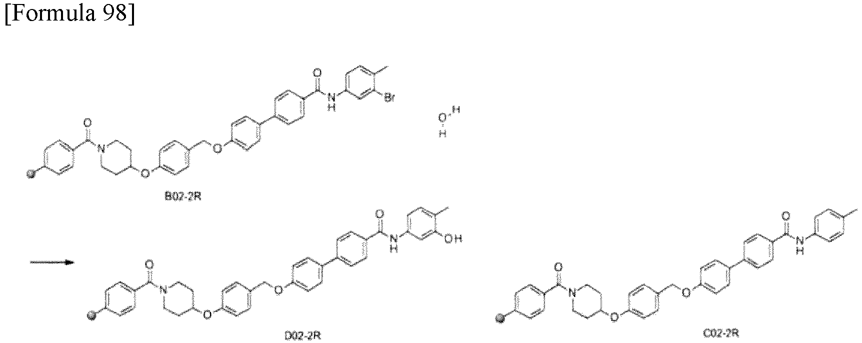

### Experimental procedures

Under a nitrogen atmosphere, to a 0.6 mL glass vial, compound B02-2R (0.194 mmol/g, 20 mg, 0.0039 mmol), DMPr (0.4 mL) and water (0.010 mL) were added, and the mixture was shaken at room temperature for 1 hour. The catalyst described in Table 2-2-1 and a P2tBu/THF solution (2M, 29.1 mL, 0.058 mmol) were added, and the mixture was shaken at 80°C for 0.5 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and the progress of the reaction was analyzed by LCMS analysis. The results (UV area%) are as shown in Table 2-2-1.

**[Table 9]**

| [Table 2-2-1] | | | | |
|---|---|---|---|---|
| Catalyst | Reaction time (hour) | Target material D02 | Starting material B02 | H-product C02 |
| tBuXPhos Pd G4 (0.6 mg, 0.00078 mmol) | 0.5 | 92.7 | | 3.3 |
| RockPhos Pd G3 (0.7 mg, 0.00078 mmol) | 0.5 | 96.0 | | 1.1 |
| BrettPhos Pd G4 (0.7 mg, 0.00078 mmol) | 0.5 | 91.5 | | 3.8 |
| tBuBrettPhos Pd G4 (0.7 mg, 0.00078 mmol) | 0.5 | 95.7 | | 1.7 |
| AdBrettPhos Pd G6 Br (0.8 mg, 0.00078 mmol) | 0.5 | 89.7 | | 0.9 |
| AdBippyPhos (1.0 mg, 0.0016 mmol) | 0.5 | 96.3 | | 2.0 |
| Pd₂dba₃CHCl₃ (0.4 mg, 0.00039 mmol) | | | | |

From the above results, it was shown that various palladium catalysts are available in the hydroxylation reaction using DMPr as the solvent. In particular, high yields were obtained when the combination of RockPhos Pd G3, tBuBrettPhos Pd G4, or AdBippyPhos and Pd₂dba₃CHCl₃ was used as the catalyst.

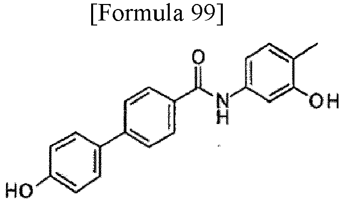

### Compound D02

LRMS: m/z 320 [M+H]⁺
Retention time: 0.931 min (analysis condition FA05-1, 299 nm).

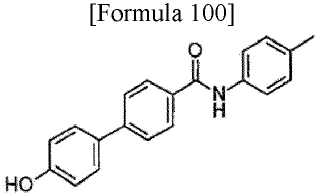

### Compound C02

LRMS: m/z 304 [M+H]⁺
Retention time: 1.075 min (analysis condition FA05-1, 299 nm).

### Example 2-3: Study of various bases in hydroxylation reaction of aryl bromide supported on solid phase

### Example 2-3-1: Experiment of studying bases using AdBrettPhos Pd G3 as palladium catalyst and DMPr as solvent in hydroxylation reaction of compound B02-2R

### Experimental procedures

Under a nitrogen atmosphere, to a 0.6 mL glass vial, compound B02-2R (0.194 mmol/g, 20 mg, 0.0039 mmol), DMPr (0.4 mL) and water (0.010 mL) were added, and the mixture was shaken at room temperature for 1 hour. AdBrettPhos Pd G3 (0.8 mg, 0.00078 mmol) and the base described in Table 2-3-1 (0.058 mmol) were added and the mixture was shaken at 80°C for the time described in Table 2-3-1.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and the progress of the reaction was analyzed by LCMS analysis. The results (UV area%) are as shown in Table 2-3-1.

**[Table 10]**

| [Table 2-3-1] | | | | |
|---|---|---|---|---|
| Base | Reaction time (hour) | Target material D02 | Starting material B02 | H-product C02 |
| Cs₂CO₃ (19.0 mg, 0.058 mmol) | 24 | 82.9 | 7.8 | 6.2 |
| K₃PO₄ (12.4 mg, 0.058 mmol) | 24 | 89.7 | | 6.7 |
| BTMG (11.6 mL, 0.058 mmol) | 2 | 87.9 | 7.3 | 2.6 |
| MTBD (8.4 mL, 0.058 mmol) | 24 | 73.2 | 14.6 | 6.7 |
| PltBu (14.8 mL, 0.058 mmol) | 0.5 | 95.3 | | 1.9 |
| BEMP (16.9 mL, 0.058 mmol) | 24 | 73.7 | 11.7 | 7.6 |
| BTPP (17.8 mL, 0.058 mmol) | 2 | 91.6 | 3.9 | 2.5 |
| P2tBu (2 M in THF, 29.1 mL, 0.058 mmol) | 0.5 | 91.9 | 2.0 | 1.8 |
| KOH (8 M aqueous solution, 7.3 mL, 0.058 mmol) | 24 | 81.6 | | 5.1 |

From the above results, it was shown that a wide range of organic bases such as BTMG, MTBD, P1tBu, BEMP, BTPP, and P2tBu, and further a wide range of inorganic bases such as Cs₂CO₃, K₃PO₄, and KOH, are available in the hydroxylation reaction using DMPr as a solvent. Preferred bases were phosphazene bases such as P1tBu and P2tBu, which have a fast reaction rate and less H-product generation.

### Example 2-4: Substrate generality

### Example 2-4-1: Synthesis of D06-2R by hydroxylation reaction of compound B06-2R

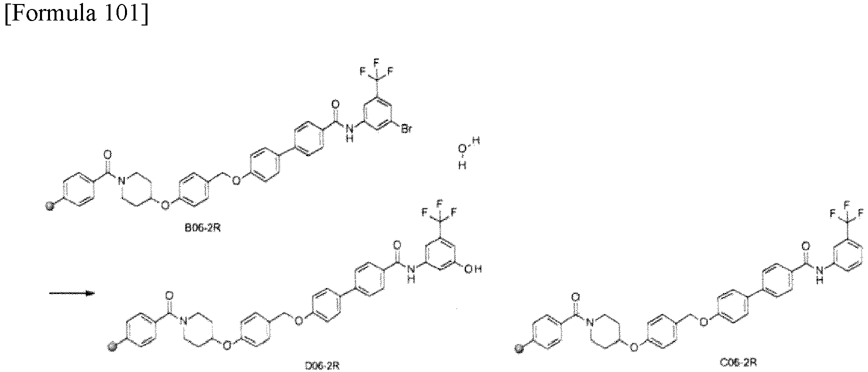

Under a nitrogen atmosphere, to a 0.6 mL glass vial, compound B06-2R (0.193 mmol/g, 20 mg, 0.0038 mmol), DMPr (0.4 mL) and water (0.010 mL) were added, and the mixture was shaken at room temperature for 1 hour. AdBrettPhos Pd G6 Br (0.7 mg, 0.00077 mmol) and BTMG (11.5 mL, 0.058 mmol) were added and the mixture was shaken at 80°C for 2 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and LCMS analysis was performed. As a result, the production of H-product (C06) was low as 2.7%, and the yield of target material D06 was high as 97.3%.

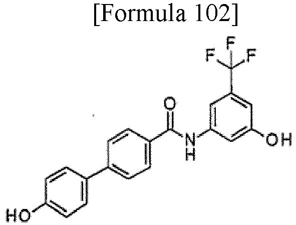

### Compound D06

LRMS: m/z 374 [M+H]⁺
Retention time: 1.040 min (analysis condition FA05-1, 299 nm).

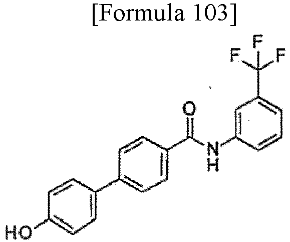

### Compound C06

LRMS: m/z 358 [M+H]⁺
Retention time: 1.183 min (analysis condition FA05-1, 299 nm).

### Example 2-5: Study of various solvents in C-O coupling reaction of aryl bromide supported on solid phase

### Example 2-5-1: Experiment of comparing solvents using AdBippyPhos as ligand for palladium catalyst and BTMG as base in C-O coupling reaction of compound B02-2R with 3-phenyl-1-propanol (g01)

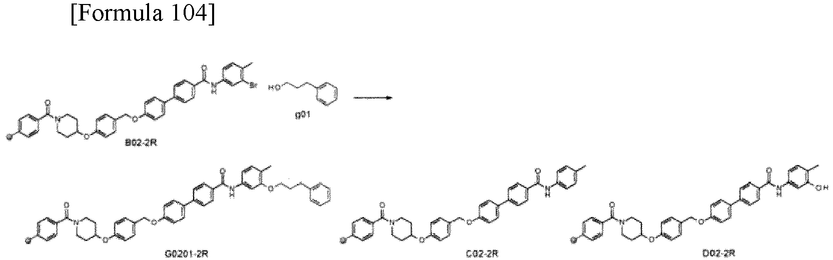

### Experimental procedures

Under a nitrogen atmosphere, to a 0.6 mL glass vial, compound B02-2R (0.194 mmol/g, 15 mg, 0.0029 mmol) and a solution of potassium trifluoromethanesulfonate (5.5 mg, 0.029 mmol) dissolved in the solvent (0.3 mL) described in Table 2-5-1. were added. To this, 3-phenyl-1-propanol (39.6 mL, 0.291 mmol) was added and the mixture was shaken at room temperature for 1 hour. A mixed solution of Pd₂dba₃CHCl₃ (0.025 M, 0.00029 mmol) and AdBippyPhos (0.1 M, 0.0016 mmol) (prepared by adding two compounds to the corresponding solvent and heating with a dryer for about 1 minute) (11.6 mL) was added. BTMG (8.7 mL, 0.044 mmol) was added and the mixture was shaken at 80°C for 24 hours.

The suspension (12 mL) of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 1 minute. After filtration, the solid phase was washed with DMF (0.05 mL). MeCN (0.25 mL) was then added to the filtrate to prepare an LC sample, and the progress of the reaction was analyzed by LCMS analysis. The results (UV area%) are as shown in Table 2-5-1.

**[Table 11]**

| [Table 2-5-1] | | | | | |
|---|---|---|---|---|---|
| | Reaction time (hour) | Target material G0201 | Starting material B02 | H-product C02 | OH-product D02 |
| DMAc | 0.5 | 31.1 | 46.4 | 12.3 | 6.1 |
| DMPr | 0.5 | 80.4 | 0 | 12.1 | 4.8 |
| NMP | 0.5 | 22.7 | 33.7 | 25.9 | 12.6 |
| DMAc | 24 | 57.8 | 27.8 | 12.3 | 7.7 |
| DMPr | 24 | 80.1 | 0 | 11.2 | 4 |
| NMP | 24 | 35.2 | 0 | 46.6 | 12.1 |

From the above results, it was confirmed that, when DMPr is used, compared to the use of conventionally used solvents, the conversion rate is higher and the generation of H-product and a by-product (OH-product) that is thought to be generated by reaction with water are reduced, giving a high yield. Meanwhile, when DMAc was used, the progress of the reaction was slow and the yield of the target material was low even after 24 hours. When NMP was used, H-product (C02) and OH-product (D02) were often generated, and the yield of the target material was low.

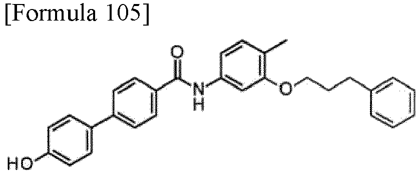

### Compound G0201

LRMS: m/z 438 [M+H]⁺
Retention time: 1.381 min (analysis condition FA05-1, 299 nm).

### Example 2-5-2: Experiment of comparing solvents using AdBippyPhos as ligand for palladium catalyst and BTMG as base in C-O coupling reaction of compound B02-2R with 2-methylphenol (g02)

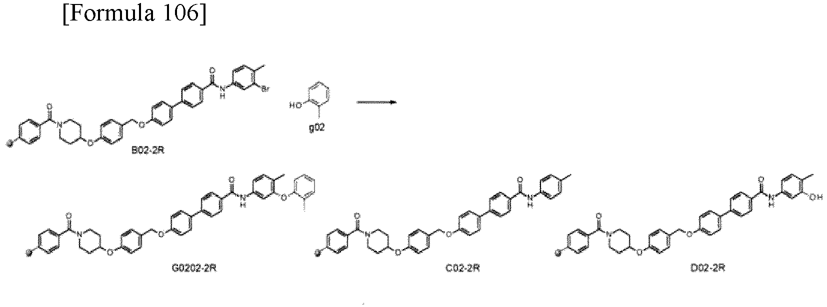

### Experimental procedures

Under a nitrogen atmosphere, to a 0.6 mL glass vial, compound B02-2R (0.194 mml/g, 15 mg, 0.0029 mmol) and a solution of potassium trifluoromethanesulfonate (5.5 mg, 0.029 mmol) dissolved in the solvent (0.3 mL) described in Table 2-5-2 were added. 2-Methylphenol (30.0 mL, 0.291 mmol) was added and the mixture was shaken at room temperature for 1 hour. A mixed solution of Pd₂dba₃CHCl₃ (0.025 M, 0.00029 mmol) and AdBippyPhos (0.1 M, 0.0016 mmol) (prepared by adding two compounds to the corresponding solvent and heating with a dryer for about 1 minute) (11.6 mL) was added. BTMG (8.7 mL, 0.044 mmol) was added and the mixture was shaken at 80°C for 24 hours.

The suspension (12 mL) of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 1 minute. After filtration, the solid phase was washed with DMF (0.05 mL). MeCN (0.25 mL) was then added to the filtrate to prepare an LC sample, and the progress of the reaction was analyzed by LCMS analysis. The results (UV area%) are as shown in Table 2-5-2.

**[Table 12]**

| [Table 2-5-2] | | | | | |
|---|---|---|---|---|---|
| | Reaction time (hour) | Target material G0202 | Starting material B02 | H-product C02 | OH-product D02 |
| DMAc | 24 | 17.8 | 73.7 | 6.4 | 0.7 |
| DMPr | 24 | 71.7 | 5.6 | 12.6 | 2.9 |
| NMP | 24 | 24.6 | 44 | 25.1 | 1.4 |

From the above results, it was confirmed that, when DMPr is used, compared to the use of conventionally used solvents, the conversion rate is higher and the generation of H-product and a by-product (OH-product) that is thought to be generated by reaction with water are reduced, giving a high yield. Meanwhile, when DMAc was used, the progress of the reaction was slow and the yield of the target material was low. When NMP was used, H-product (C02) was often generated, and the yield of the target material was low.

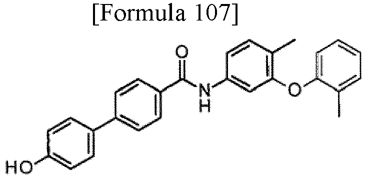

### Compound G0202

LRMS: m/z 410 [M+H]⁺
Retention time: 1.327 min (analysis condition FA05-1, 299 nm).

### Example 3

### Example 3-1: Study of various solvents in C-N coupling reaction of aryl bromide supported on solid phase

### Example 3-1-1: Experiment of studying solvents using (tBu)PhCPhos Pd G4 as palladium catalyst and P2tBu as base in C-N coupling reaction of compound B02-1R with 4-phenylpiperidine (e01)

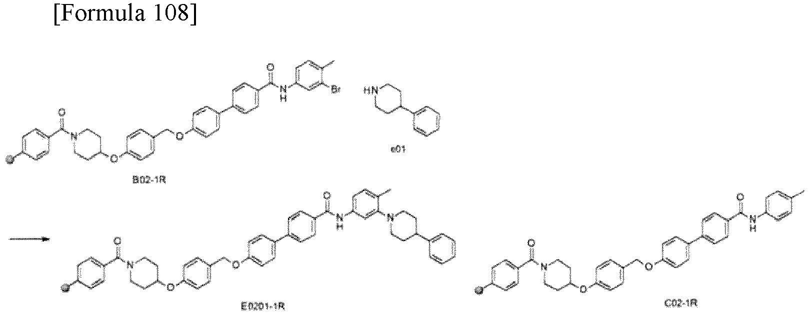

### Experimental procedures

Under a nitrogen atmosphere, to a 0.6 mL glass vial, compound B02-1R (0.194 mmol/g, 20 mg, 0.0039 mmol) and a solvent (0.4 mL) were added, and the mixture was shaken at room temperature for 1 hour. 4-Phenylpiperidine (e01) (12.5 mg, 0.078 mmol) and (tBu)PhCPhos Pd G4 (0.6 mg, 0.00078 mmol), and a P2tBu/THF solution (2 M, 58.2 mL, 0.116 mmol) were added and the mixture was shaken at room temperature for 2 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and the progress of the reaction was analyzed by LCMS analysis. The results (UV area%) are as shown in Table 3-1-1.

**[Table 13]**

| [Table 3-1-1] | | | | | | |
|---|---|---|---|---|---|---|
| Solvent | Reaction time (hour) | Target material E0201 | Starting material B02 | H-product C02 | Impurity P02 | Total of other impurities |
| DMPr | 2 | 98.3 | | 0.8 | | 0.9 |
| DMAc | 2 | 53.8 | 45.0 | 0.8 | | 0.4 |
| DMF | 2 | 32.1 | 64.8 | 0.9 | | 2.2 |
| DMI | 2 | 5.7 | 84.7 | 1.2 | | 8.4 |
| DMPU | 2 | 2.5 | 95.9 | 1.2 | | 0.4 |
| NMP | 2 | 79.2 | | 17.2 | 0.9 | 2.7 |
| THF | 2 | 22.8 | 75.5 | 0.7 | | 1.0 |
| Toluene | 2 | 9.0 | 87.3 | 0.6 | | 3.2 |

From the above results, it was confirmed that, when DMPr is used, compared to the use of conventionally used solvents, the conversion rate is higher and the generation of H-product and other impurities are reduced, giving a high yield. Meanwhile, it was confirmed that the progress of the reaction is slow when DMAc, DMF, DMI, or DMPU is used, and the reaction rate is slow even when THF and Toluene, which have been reported to inhibit H-product generation, (Reference: Organometallics 2013, 32, 5428-5434.) are used. When NMP was used, although the loss of starting materials was rapid, H-product (C02) was often produced, impurity P02 with 83 m/z higher than H-product was observed, and the yield of the target material was low.

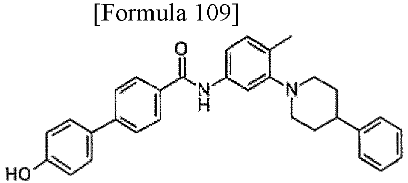

### Compound E0201

LRMS: m/z 463 [M+H]⁺
Retention time: 1.340 min (analysis condition FA05-1, 299 nm).

### Compound P02 (structure undetermined)

LRMS: m/z 387 [M+H]⁺
Retention time: 0.905 min (analysis condition FA05-1, 299 nm).

### Example 3-1-2: Experiment of studying solvents using (tBu)PhCPhos Pd G4 as palladium catalyst and P2tBu as base in C-N coupling reaction of compound B02-1R with N-methyl-3-phenylpropylamine (e02)

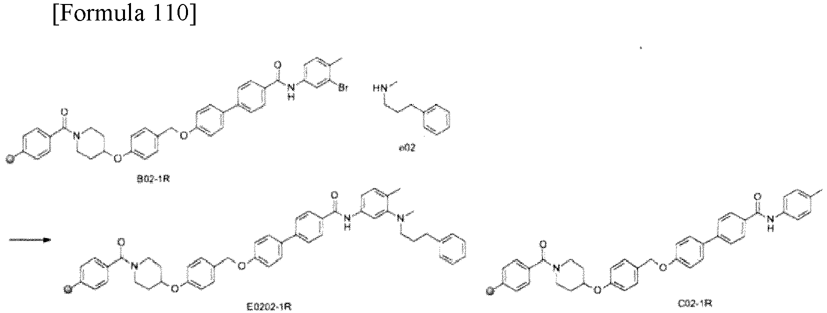

### Experimental procedures

Under a nitrogen atmosphere, to a 0.6 mL glass vial, compound B02-1R (0.194 mmol/g, 20 mg, 0.0039 mmol) and a solvent (0.4 mL) were added, and the mixture was shaken at room temperature for 1 hour. N-methyl-3-phenylpropylamine (e02) (11.6 mg, 0.078 mmol) and (tBu)PhCPhos Pd G4 (0.6 mg, 0.00078 mmol), and a P2tBu/THF solution (2 M, 58.2 mL, 0.116 mmol) were added and the mixture was shaken at room temperature for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the phase was washed with DMF (0.05 mL). MeCN (0.25 mL) was then added to the filtrate to prepare an LC sample, and the progress of the reaction was analyzed by LCMS analysis. The results are as shown in Table 3-1-2.

**[Table 14]**

| [Table 3-1-2] | | | | | | |
|---|---|---|---|---|---|---|
| Solvent | Reaction time (hour) | Target material E0202 | Starting material B02 | H-product C02 | Impurity P02 | Total of other impurities |
| DMPr | 24 | 87.0 | 7.5 | 3.4 | | 2.1 |
| DMAc | 24 | 17.4 | 77.4 | 2.7 | | 2.4 |
| DMF | 24 | 27.9 | 61.1 | 5.9 | | 5.1 |
| DMI | 24 | 4.5 | 75.3 | 3.5 | | 16.6 |
| DMPU | 24 | 2.1 | 90.6 | 5.2 | | 2.2 |
| NMP | 24 | 47.1 | | 46.2 | 3.8 | 2.9 |
| THF | 24 | 6.4 | 88.0 | 1.7 | | 3.9 |
| Toluene | 24 | 3.7 | 91.6 | 1.1 | | 3.6 |
| DMOc | 24 | 26.5 | 60.9 | 2.7 | | 9.9 |
| DMSO | 24 | 14.9 | 74.7 | 2.5 | | 7.9 |
| tAmylOH | 24 | 5.2 | 90.0 | 1.1 | | 3.6 |

From the above results, it was confirmed that, when DMPr is used, compared to the use of conventionally used solvents, the conversion rate is higher and the generation of H-product and other impurities are reduced, giving a high yield. Meanwhile, it was confirmed that the reaction rate is slow when DMAc, DMF, DMI, DMPU, THF, and Toluene are used. When NMP was used, the production of H-product (C02) was extremely high and impurity P02 with 83 m/z higher than H-product was observed, and the yield was low. Although it was studied using DMSO and tAmylOH, which are high-polarity solvents similar to amide-based solvents and have been reported as solvents for C-N coupling reactions (Organic Letters 2015, 17, 3370-3373.), the solid phase did not swell and the reaction had almost no progress. In addition, when DMOc, an amide-based solvent, which has been reported as an additive to solvents in flow reaction of C-N coupling reactions (Angew. Chem. Int. Ed. 2016, 55, 2531-2535.) was used, the progress of the reaction was slow and the yield was low.

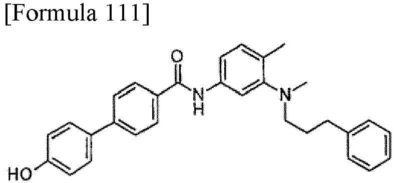

### Compound E0202

LRMS: m/z 451 [M+H]⁺
Retention time: 1.027 min (analysis condition FA05-1, 299 nm)
Retention time: 2.120 min (analysis condition FA05-long, 299 nm).

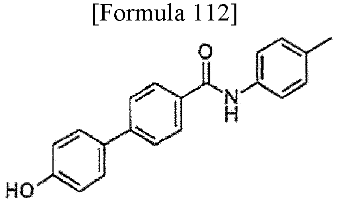

### Compound C02

LRMS: m/z 304 [M+H]⁺
Retention time: 2.257 min (analysis condition FA05-long, 299 nm).

### Compound P02 (structure undetermined)

LRMS: m/z 387 [M+H]⁺
Retention time: 1.807 min (analysis condition FA05-long, 299 nm).

### Example 3-1-3: Experiment of studying solvents using (tBu)PhCPhos Pd G4 as palladium catalyst and P2tBu as base in C-N coupling reaction of compound B02-1R with 1-methyl-3-phenylpropylamine (e06)

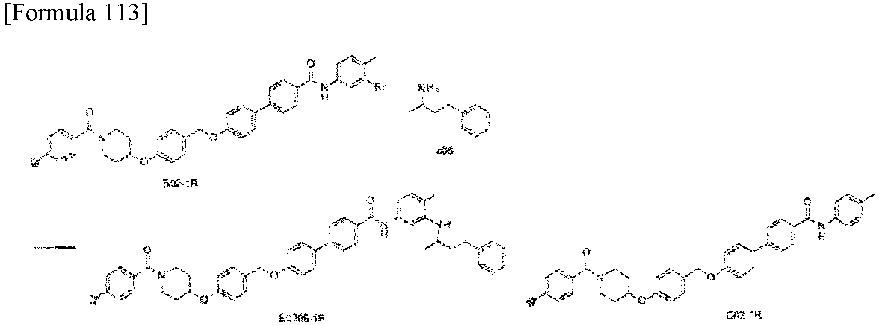

### Experimental procedures

Under a nitrogen atmosphere, to a 0.6 mL glass vial, compound B02-1R (0.194 mmol/g, 20 mg, 0.0039 mmol) and a solvent (0.4 mL) were added, and the mixture was shaken at room temperature for 1 hour. 1-Methyl-3-phenylpropylamine (e06) (12.5 mL, 0.078 mmol) and (tBu)PhCPhos Pd G4 (0.6 mg, 0.00078 mmol), and a P2tBu/THF solution (2 M, 58.2 mL, 0.116 mmol) were added and the mixture was shaken at room temperature for 2 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and the progress of the reaction was analyzed by LCMS analysis. The results (UV area%) are as shown in Table 3-1-3.

**[Table 15]**

| [Table 3-1-3] | | | | | | |
|---|---|---|---|---|---|---|
| Solvent | Reaction time (hour) | Target material E0206 | Starting material B02 | H-product C02 | Impurity P02 | Total of other impurities |
| DMPr | 2 | 97.8 | | 0.6 | | 1.7 |
| DMAc | 2 | 61.7 | 36.0 | 0.7 | | 1.6 |
| NMP | 2 | 59.1 | 20.0 | 11.5 | 0.9 | 8.5 |

From the above results, it was confirmed that, when DMPr is used, compared to the use of conventionally used solvents, the conversion rate is higher and the generation of H-product and other impurities are reduced, giving a high yield. Meanwhile, it was confirmed that, when DMAc is used, the reaction rate is slow. When NMP was used, H-product (C02) was often generated, and the reaction rate was confirmed to be slow, and the yield was low.

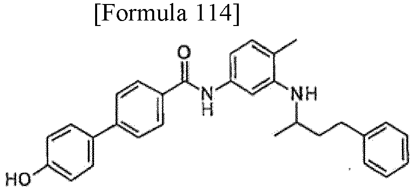

### Compound E0206

LRMS: m/z 451 [M+H]⁺
Retention time: 1.276 min (analysis condition FA05-1, 299 nm).

### Example 3-1-4: Experiment of studying solvents using (tBu)PhCPhos Pd G4 as palladium catalyst and P2tBu as base in C-N coupling reaction of compound B01-1R with dipropylamine (e03)

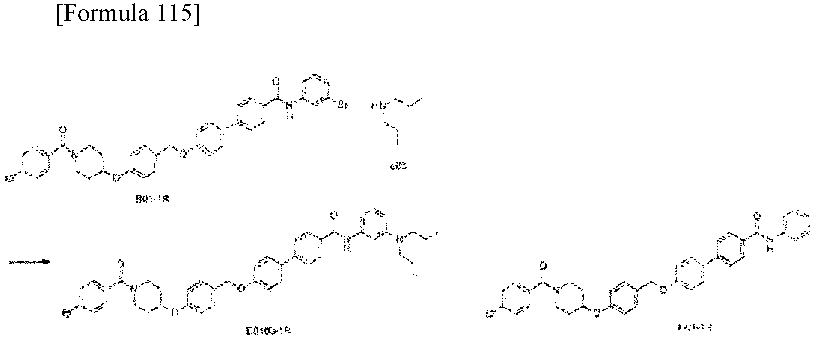

### Experimental procedures

Under a nitrogen atmosphere, to a 0.6 mL glass vial, compound B01-1R (0.195 mmol/g, 20 mg, 0.0039 mmol) and a solvent (0.4 mL) were added, and the mixture was shaken at room temperature for 1 hour. Dipropylamine (e03) (10.7 mL, 0.078 mmol) and (tBu)PhCPhos Pd G4 (0.6 mg, 0.00078 mmol), and a P2tBu/THF solution (2 M, 58.5 mL, 0.117 mmol) were added and the mixture was shaken at room temperature for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and the progress of the reaction was analyzed by LCMS analysis. The results (UV area%) are as shown in Table 3-1-4.

**[Table 16]**

| [Table 3-1-4] | | | | | | | |
|---|---|---|---|---|---|---|---|
| Solvent | Reaction time (hour) | Target material E0103 | Starting material B01 | H-product C01 | Impurity P01 | Impurity R01 | Total of other impurities |
| DMPr | 24 | 87.5 | 0.6 | 3.7 | | | 8.2 |
| DMAc | 24 | 44.0 | 46.0 | 3.3 | | 2.7 | 4.1 |
| NMP | 24 | 11.3 | | 73.4 | 12.4 | | 2.9 |

From the above results, it was confirmed that, when DMPr is used, compared to the use of conventionally used solvents, the conversion rate is higher and the generation of H-product and other impurities are reduced, giving a high yield. Meanwhile, when DMAc was used, the reaction rate was slow, and impurity R01 with 57 m/z higher than H-product was observed. When NMP was used, the production of H-product (C01) was extremely high and impurity P01 with 83 m/z higher than H-product was observed, and the yield was low.

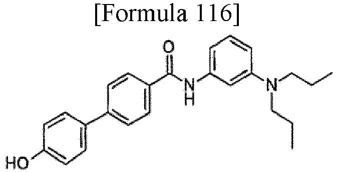

### Compound E0103

LRMS: m/z 389 [M+H]⁺
Retention time: 1.989 min (analysis condition FA05-long, 299 nm).

### Compound P01 (structure undetermined)

LRMS: m/z 373 [M+H]⁺
Retention time: 1.823 min (analysis condition FA05-long, 299 nm).

### Compound R01 (structure undetermined)

LRMS: m/z 347 [M+H]⁺
Retention time: 1.720 min (analysis condition FA05-long, 299 nm).

### Example 3-1-5: Experiment of studying solvents using (tBu)PhCPhos Pd G4 as palladium catalyst and P2tBu as base in C-N coupling reaction of compound B01-1R with 2-methylpiperidine (e04)

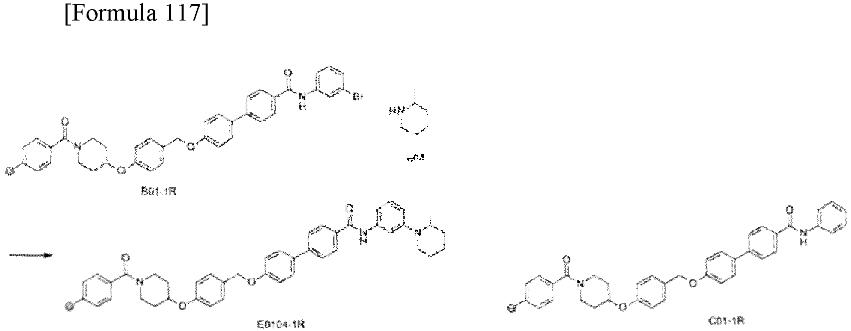

### Experimental procedures

Under a nitrogen atmosphere, to a 0.6 mL glass vial, compound B01-1R (0.195 mmol/g, 20 mg, 0.0039 mmol) and a solvent (0.4 mL) were added, and the mixture was shaken at room temperature for 1 hour. 2-Methylpiperidine (e04) (9.21 mL, 0.078 mmol) and (tBu)PhCPhos Pd G4 (0.6 mg, 0.00078 mmol), and a P2tBu/THF solution (2 M, 58.5 mL, 0.117 mmol) were added and the mixture was shaken at room temperature for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and the progress of the reaction was analyzed by LCMS analysis. The results (UV area%) are as shown in Table 3-1-5.

**[Table 17]**

| [Table 3-1-5] | | | | | | | |
|---|---|---|---|---|---|---|---|
| Solvent | Reaction time (hour) | Target material E0104 | Starting material B01 | H-product C01 | Impurity P01 | Impurity R01 | Total of other impurities |
| DMPr | 24 | 90.6 | | 2.5 | | | 6.9 |
| DMAc | 24 | 46.5 | 42.3 | 2.0 | | 1.4 | 7.8 |
| NMP | 24 | 13.8 | | 70.7 | 12.3 | | 3.2 |

From the above results, it was confirmed that, when DMPr is used, compared to the use of conventionally used solvents, the conversion rate is higher and the generation of H-product and other impurities are reduced, giving a high yield. Meanwhile, when DMAc was used, the reaction rate was slow, and impurity R01 with 57 m/z higher than H-product was observed. When NMP was used, the production of H-product (C01) was extremely high and impurity P01 with 83 m/z higher than H-product was observed, and the yield was low.

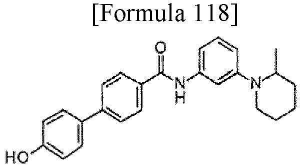

### Compound E0104

LRMS: m/z 387 [M+H]⁺
Retention time: 0.705 min (analysis condition FA05-01, 299 nm).

### Compound R01 (structure undetermined)

LRMS: m/z 347 [M+H]⁺
Retention time: 0.879 min (analysis condition FA05-01, 299 nm).

### Example 3-1-6: Experiment of studying solvents using (tBu)PhCPhos Pd G4 as palladium catalyst and P2tBu as base in C-N coupling reaction of compound B02-1R with 2,6-dimethylaniline (e13)

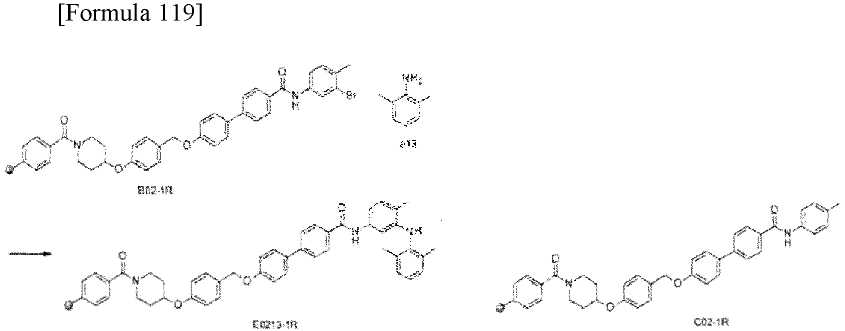

Under a nitrogen atmosphere, to a 0.6 mL glass vial, compound B02-1R (0.194 mmol/g, 20 mg, 0.0039 mmol) and a solvent (0.4 mL) were added, and the mixture was shaken at room temperature for 1 hour. 2,6-Dimethylaniline (e13) (9.6 mL, 0.078 mmol) and (tBu)PhCPhos Pd G4 (0.6 mg, 0.00078 mmol) were added. A P2tBu/THF solution (2M, 58.2 mL, 0.116 mmol) was added and the mixture was shaken at room temperature for 24 hours.

The suspension (12 mL) of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and the progress of the reaction was analyzed by LCMS analysis. The results (UV area%) are as shown in Table 3-1-6.

**[Table 18]**

| [Table 3-1-6] | | | | | | |
|---|---|---|---|---|---|---|
| Solvent | Reaction time (hour) | Target material E0213 | Starting material B02 | H-product C02 | Impurity P02 | Total of other impurities |
| DMAc | 24 | 12.9 | 84.7 | 1.0 | 0.0 | 1.4 |
| DMPr | 24 | 87.5 | 5.4 | 3.5 | 0.0 | 3.6 |
| NMP | 24 | 19.7 | 0.0 | 72.2 | 5.9 | 2.2 |

From the above results, it was confirmed that, when DMPr is used, compared to the use of conventionally used solvents, the conversion rate is higher and the generation of H-product and other impurities are reduced, giving a high yield. Meanwhile, it was confirmed that, when DMAc is used, the reaction rate is slow. When NMP was used, the production of H-product (C02) was extremely high and impurity P02 with 83 m/z higher than H-product was observed, and the yield was low.

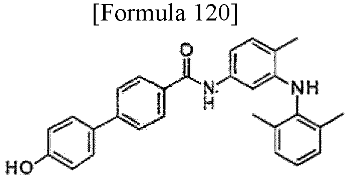

### Compound E0213

LRMS: m/z 423 [M+H]⁺
Retention time: 1.275 min (analysis condition FA05-1, 299 nm).

### Example 3-1-7: Experiment of studying solvents using (tBu)PhCPhos Pd G4 as palladium catalyst and P2tBu as base in C-N coupling reaction of compound B01-1R with N-ethylaniline (e16)

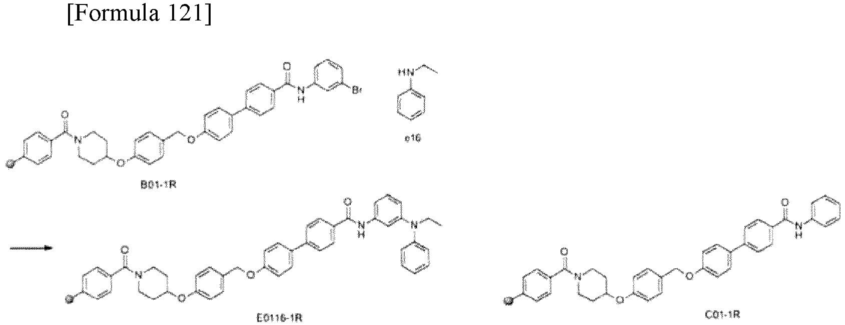

Under a nitrogen atmosphere, to a 0.6 mL glass vial, compound B01-1R (0.195 mmol/g, 20 mg, 0.0039 mmol) and a solvent (0.4 mL) were added, and the mixture was shaken at room temperature for 1 hour. N-ethylaniline (e16) (9.9 mL, 0.078 mmol) and (tBu)PhCPhos Pd G4 (0.6 mg, 0.00078 mmol) were added. A P2tBu/THF solution (2M, 58.5 mL, 0.117 mmol) was added and the mixture was shaken at room temperature for 2 hours.

The suspension (12 mL) of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and the progress of the reaction was analyzed by LCMS analysis. The results (UV area%) are as shown in Table 3-1-7.

**[Table 19]**

| [Table 3-1-7] | | | | | | |
|---|---|---|---|---|---|---|
| Solvent | Reaction time (hour) | Target material E0116 | Starting material B01 | H-product C01 | Impurity P01 | Total of other impurities |
| DMAc | 24 | 13.9 | 83.7 | 0.9 | 0.0 | 1.5 |
| DMPr | 24 | 92.3 | 0.0 | 2.7 | 0.0 | 5.0 |
| NMP | 24 | 20.8 | 0.4 | 69.0 | 7.4 | 2.4 |

From the above results, it was confirmed that, when DMPr is used, compared to the use of conventionally used solvents, the conversion rate is higher and the generation of H-product and other impurities are reduced, giving a high yield. Meanwhile, it was confirmed that, when DMAc is used, the reaction rate is slow. When NMP was used, the production of H-product (C01) was extremely high and impurity P01 with 83 m/z higher than H-product was observed, and the yield was low.

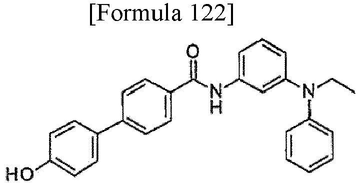

### Compound E0116

LRMS: m/z 409 [M+H]⁺
Retention time: 1.313 min (analysis condition FA05-1, 299 nm).

### Example 3-2: Study of various catalysts in C-N coupling reaction of aryl bromide supported on solid phase

### Example 3-2-1: Experiment of studying palladium catalysts using P2tBu as base and DMPr as solvent in C-N coupling reaction of compound B01-2R with piperidine (e08)

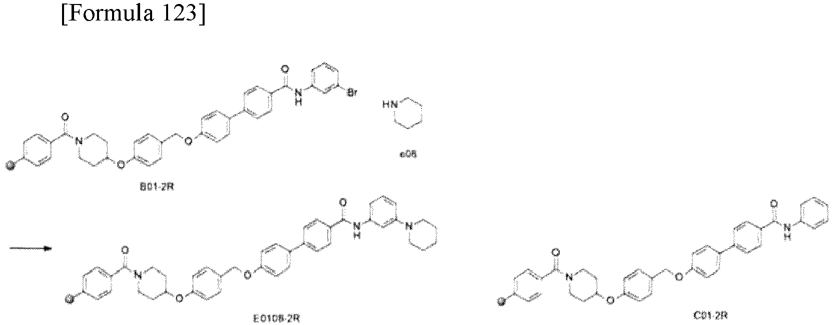

Under a nitrogen atmosphere, to a 0.6 mL glass vial, compound B01-2R (0.195 mmol/g, 20 mg, 0.0039 mmol), DMPr (0.4 mL) and piperidine (e08) (3.85 mL, 0.039 mmol) were added, and the mixture was shaken at room temperature for 1 hour. The catalyst described in Table 3-2-1 and a P2tBu/TH solution (2M, 29.3 mL, 0.059 mmol) were added, and the mixture was shaken at 80°C for the time described in Table 3-2-1.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and the progress of the reaction was analyzed by LCMS analysis. The results (UV area%) are as shown in Table 3-2-1.

**[Table 20]**

| [Table 3-2-1] | | | | |
|---|---|---|---|---|
| Catalyst | Reaction time (hour) | Target material E0108 | Starting material B01 | H-product C01 |
| RuPhos Pd G4 (0.7 mg, 0.00078 mmol) | 0.5 | 94.6 | | 3.5 |
| tBuXPhos Pd G4 (0.6 mg, 0.00078 mmol) | 0.5 | 96.1 | | 1.0 |
| (tBu)PhCPhos Pd G4 (0.6 mg, 0.00078 mmol) | 0.5 | 96.5 | | 1.7 |
| MorDalPhos Pd G4 (0.7 mg, 0.00078 mmol) | 24 | 65.7 | 19.4 | 11.5 |
| tBu₃P Pd G4 (0.5 mg, 0.00078 mmol) | 0.5 | 83.5 | | 11.6 |
| BippyPhos (0.8 mg, 0.0016 mmol) | 0.5 | 93.6 | | 1.5 |
| Pd₂dba₃CHCl₃ (0.4 mg, 0.00039 mmol) | | | | |
| cataCXium PIntB (0.5 mg, 0.0016 mmol) | 0.5 | 98.0 | | 2.0 |
| Pd₂dba₃CHCl₃ (0.4 mg, 0.00039 mmol) | | | | |
| PEPPSI-IPent (0.6 mg, 0.00078 mmol) | 2 | 61.0 | 32.5 | 3.3 |

From the above results, it was shown that a wide range of palladium catalysts are available in the C-N coupling reaction using DMPr as the solvent.

In particular, high yield was achieved when using catalysts of a combination of RuPhos Pd G4, tBuXPhos Pd G4, (tBu)PhCPhos Pd G4, BippyPhos with Pd₂dba₃CHCl₃ or a combination of cataCXium PIntB with Pd₂dba₃CHCl₃.

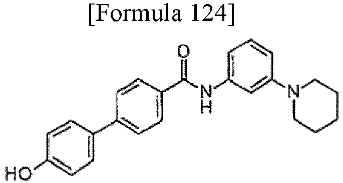

### Compound E0108

LRMS: m/z 373 [M+H]⁺
Retention time: 0.757 min (analysis condition FA05-01, 299 nm).

### Example 3-3: Study of various bases in C-N coupling reaction of aryl bromide supported on solid phase

### Example 3-3-1: Experiment of studying bases using (tBu)PhCPhos Pd G4 as palladium catalyst and DMPr as solvent in C-N coupling reaction of compound B01-2R with piperidine (e08)

Under a nitrogen atmosphere, to a 0.6 mL glass vial, compound B01-2R (0.195 mmol/g, 20 mg, 0.0039 mmol), DMPr (0.4 mL) and piperidine (e08) (3.85 mL, 0.039 mmol) were added, and the mixture was shaken at room temperature for 1 hour. (tBu)PhCPhos Pd G4 (0.6 mg, 0.00078 mmol) and the base described in Table 3-3-1 (0.0059 mmol) were added and the mixture was shaken at 80°C for the time described in Table 3-3-1.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and the progress of the reaction was analyzed by LCMS analysis. The results (UV area%) are as shown in Table 3-3-1.

**[Table 21]**

| [Table 3-3-1] | | | | |
|---|---|---|---|---|
| Base | Reaction time (hour) | Target material E0108 | Starting material B01 | H-product C01 |
| K₃PO₄ (12.4 mg, 0.0059 mmol) | 24 | 84.0 | | 9.2 |
| P2tBu (2 M in THF, 29.3 mL, 0.0059 mmol) | 0.5 | 96.5 | | 1.7 |
| NaOtBu (2 M in THF, 29.3 mL, 0.0059 mmol) | 0.5 | 83.5 | | 13.4 |

From the above results, it was shown that, in addition to the organic base P2tBu, inorganic bases K₃PO₄ and NaOtBu are available in the C-N coupling reaction using DMPr as a solvent. A preferred base was a phosphazene base P2tBu, which has a fast reaction rate and less H-product generation.

### Example 3-3-2: Experiment of adding salt when using (tBu)PhCPhos Pd G4 as palladium catalyst BTMG as base, and DMPr as solvent in C-N coupling reaction of compound B02-1R with 4-phenylpiperidine (e01)

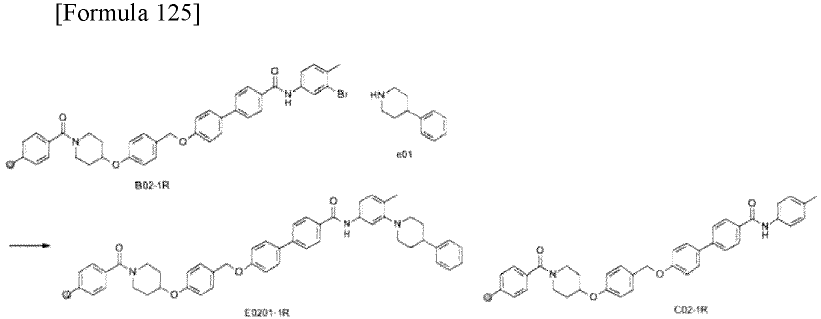

Under a nitrogen atmosphere, to a 0.6 mL glass vial, compound B02-1R (0.194 mmol/g, 20 mg, 0.0039 mmol) and DMPr (0.4 mL) were added, and the mixture was shaken at room temperature for 1 hour. 4-Phenylpiperidine (e01) (12.5 mg, 0.078 mmol) and (tBu)PhCPhos Pd G4 (0.6 mg, 0.00078 mmol) were added. The salt (0.116 mmol) described in Table 3-3-2 and BTMG (23.2 mL, 0.116 mmol) were added, and the mixture was shaken at 80°C for 2 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and the progress of the reaction was analyzed by LCMS analysis. The results (UV area%) are as shown in Table 3-3-2.

**[Table 22]**

| [Table 3-3-2] | | | | |
|---|---|---|---|---|
| Salt | Reaction time (hour) | Target material E0201 | Starting material B02 | H-product C02 |
| None | 2 | 3.9 | 74.5 | 2.3 |
| NaTFA (15.8 mg, 0.116 mmol) | 2 | 60.6 | 20.6 | 5.4 |
| KTFA (17.7 mg, 0.116 mmol) | 2 | 53.3 | 23.7 | 5.5 |

From the above results, it was shown that in the C-N coupling reaction using DMPr as a solvent, the addition of a salt such as NaTFA and KTFA may accelerate the progress of the reaction and improve the yield. The addition effect of the salt is not limited to the substrate used in this experiment.

### Example 3-4: Substrate generality

### Example 3-4-1: Synthesis of E0205-1R by C-N coupling reaction of compound B02-1R with cyclohexylamine (e05)

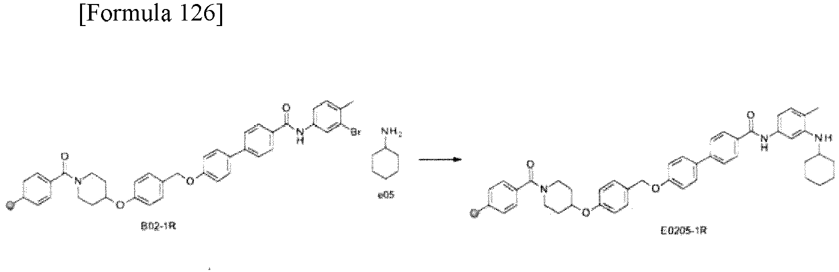

Under a nitrogen atmosphere, to a 0.6 mL glass vial, compound B02-1R (0.194 mmol/g, 20 mg, 0.0039 mmol) and DMPr (0.4 mL) were added, and the mixture was shaken at room temperature for 1 hour. Cyclohexylamine (e05) (8.9 mL, 0.078 mmol) and (tBu)PhCPhos Pd G4 (0.6 mg, 0.00078 mmol) were added. A P2tBu/THF solution (2M, 58.2 mL, 0.116 mmol) was added and the mixture was shaken at room temperature for 0.5 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and LCMS analysis was performed. As a result, the target material E0205 was observed as 100%.

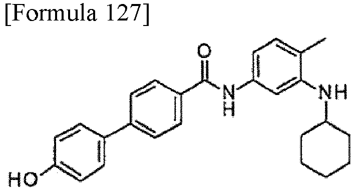

### Compound E0205

LRMS: m/z 401 [M+H]⁺
Retention time: 1.096 min (analysis condition FA05-1, 299 nm).

### Example 3-4-2: Synthesis of E0207-1R by C-N coupling reaction of compound B02-1R with 3-aminopentane (e07)

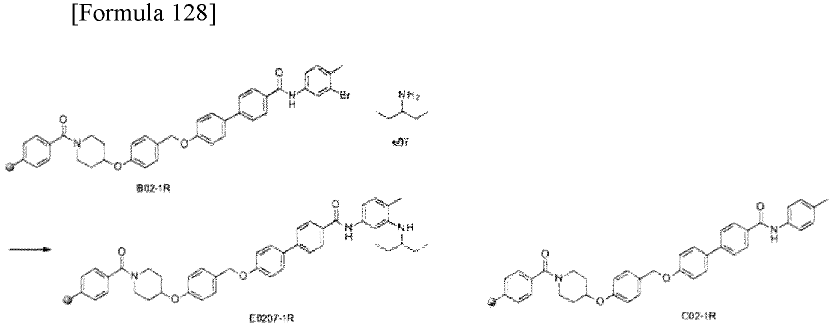

Under a nitrogen atmosphere, to a 0.6 mL glass vial, compound B02-1R (0.194 mmol/g, 20 mg, 0.0039 mmol) and DMPr (0.4 mL) were added, and the mixture was shaken at room temperature for 1 hour. 3-Aminopentane (e07) (9.0 mL, 0.078 mmol) and (tBu)PhCPhos Pd G4 (0.6 mg, 0.00078 mmol) were added. A P2tBu/THF solution (2M, 58.2 mL, 0.116 mmol) was added and the mixture was shaken at room temperature for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and LCMS analysis was performed. As a result, the target material E0207 was observed as 98.6%, and H-product (C02) was observed as 1.4%.

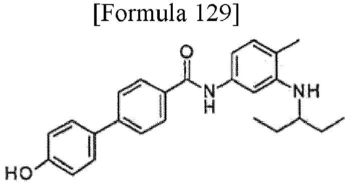

### Compound E0207

LRMS: m/z 389 [M+H]⁺
Retention time: 1.229 min (analysis condition FA05-1, 299 nm).

### Example 3-4-3: Synthesis of E0209-1R by C-N coupling reaction of compound B02-1R with o-Toluidine (e09)

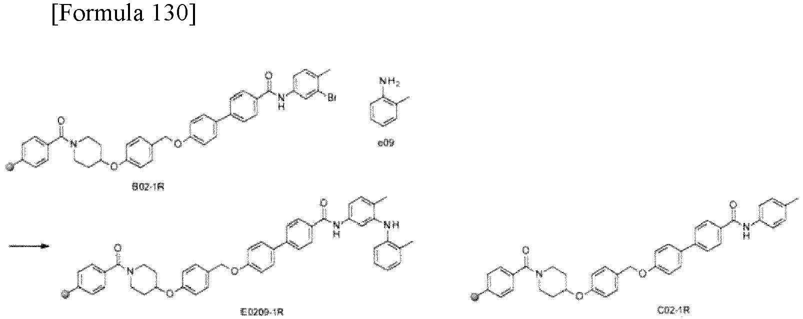

Under a nitrogen atmosphere, to a 0.6 mL glass vial, compound B02-1R (0.194 mmol/g, 20 mg, 0.0039 mmol) and DMPr (0.4 mL) were added, and the mixture was shaken at room temperature for 1 hour. o-Toluidine (e09) (8.3 mL, 0.078 mmol) and (tBu)PhCPhos Pd G4 (0.6 mg, 0.00078 mmol) were added. A P2tBu/THF solution (2M, 58.2 mL, 0.116 mmol) was added and the mixture was shaken at room temperature for 2 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and LCMS analysis was performed. As a result, the target material E0209 was observed as 98.2%, and H-product (C02) was observed as 1.1%.

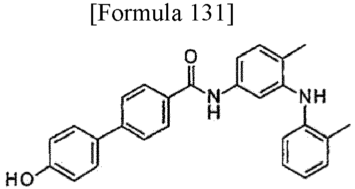

### Compound E0209

LRMS: m/z 409 [M+H]⁺
Retention time: 1.257 min (analysis condition FA05-1, 299 nm).

### Example 3-4-4: Synthesis of E0210-1R by C-N coupling reaction of compound B02-1R with 2-ethylaniline (e10)

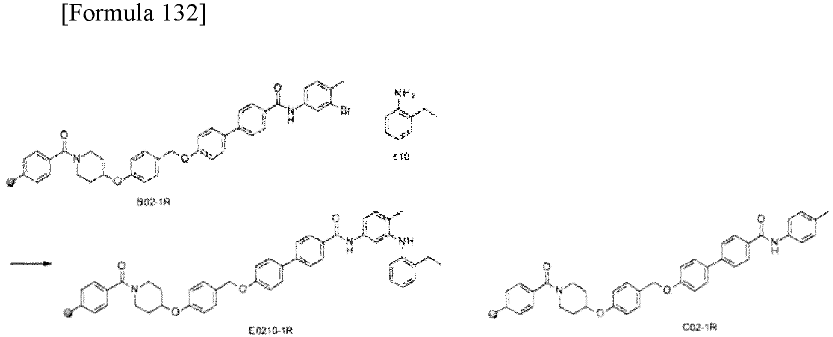

Under a nitrogen atmosphere, to a 0.6 mL glass vial, compound B02-1R (0.194 mmol/g, 20 mg, 0.0039 mmol) and DMPr (0.4 mL) were added, and the mixture was shaken at room temperature for 1 hour. 2-Ethylaniline (e10) (9.6 mL, 0.078 mmol) and (tBu)PhCPhos Pd G4 (0.6 mg, 0.00078 mmol) were added. A P2tBu/THF solution (2M, 58.2 mL, 0.116 mmol) was added and the mixture was shaken at room temperature for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and LCMS analysis was performed. As a result, the target material E0210 was observed as 98.7%, and H-product (C02) was observed as 1.3%.

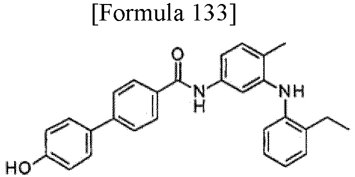

### Compound E0210

LRMS: m/z 423 [M+H]⁺
Retention time: 1.309 min (analysis condition FA05-1, 299 nm).

### Example 3-4-5: Synthesis of E0211-1R by C-N coupling reaction of compound B02-1R with 2-isopropylaniline (e11)

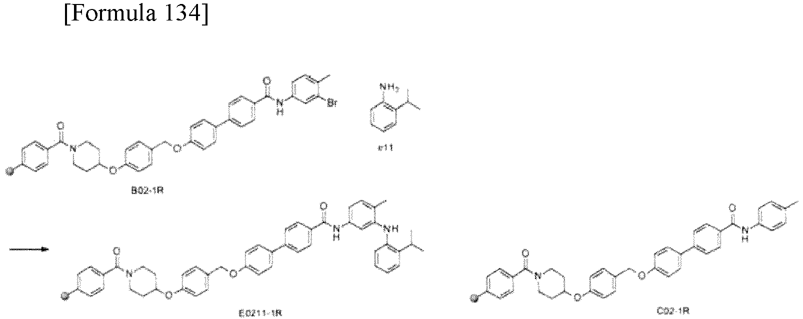

Under a nitrogen atmosphere, to a 0.6 mL glass vial, compound B02-1R (0.194 mmol/g, 20 mg, 0.0039 mmol) and DMPr (0.4 mL) were added, and the mixture was shaken at room temperature for 1 hour. 2-Isopropylaniline (e11) (10.8 mL, 0.078 mmol) and (tBu)PhCPhos Pd G4 (0.6 mg, 0.00078 mmol) were added. A P2tBu/THF solution (2M, 58.2 mL, 0.116 mmol) was added and the mixture was shaken at room temperature for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and LCMS analysis was performed. As a result, the target material E0211 was observed as 97.9%, and H-product (C02) was observed as 2.1%.

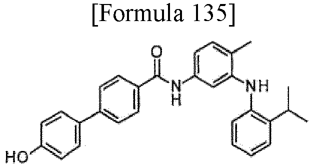

### Compound E0211

LRMS: m/z 437 [M+H]⁺
Retention time: 1.351 min (analysis condition FA05-1, 299 nm).

### Example 3-4-6: Synthesis of E0212-1R by C-N coupling reaction of compound B02-1R with 2-(tert-butyl)aniline (e12)

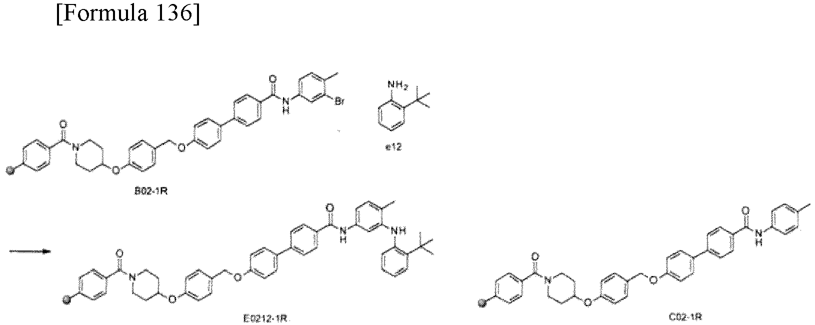

Under a nitrogen atmosphere, to a 0.6 mL glass vial, compound B02-1R (0.194 mmol/g, 20 mg, 0.0039 mmol) and DMPr (0.4 mL) were added, and the mixture was shaken at room temperature for 1 hour. 2-(tert-Butyl)aniline (e12) (12.1 mL, 0.078 mmol) and (tBu)PhCPhos Pd G4 (0.6 mg, 0.00078 mmol) were added. A P2tBu/THF solution (2M, 58.2 mL, 0.116 mmol) was added and the mixture was shaken at room temperature for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and LCMS analysis was performed. As a result, the target material E0212 was observed as 92.9%, and H-product (C02) was observed as 2.3%.

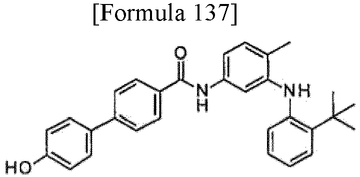

### Compound E0212

LRMS: m/z 451 [M+H]⁺
Retention time: 1.407 min (analysis condition FA05-1, 299 nm).

### Example 3-4-8: Synthesis of E0113-1R by C-coupling reaction of compound B01-1R with 2,6-dimethylaniline (e13)

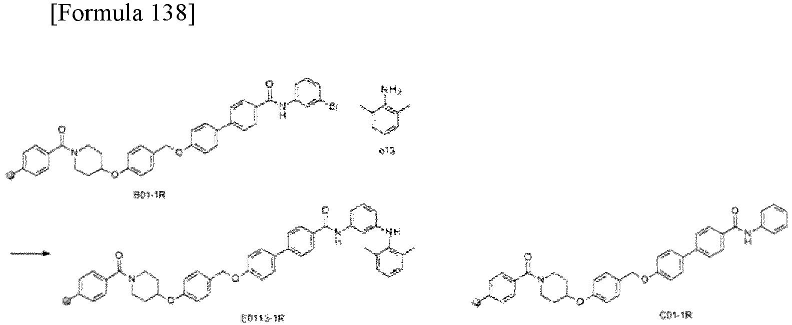

Under a nitrogen atmosphere, to a 0.6 mL glass vial, compound B01-1R (0.195 mmol/g, 20 mg, 0.0039 mmol) and DMPr (0.4 mL) were added, and the mixture was shaken at room temperature for 1 hour. 2,6-Dimethylaniline (e13) (9.7 mL, 0.078 mmol) and (tBu)PhCPhos Pd G4 (0.6 mg, 0.00078 mmol) were added. A P2tBu/THF solution (2M, 58.5 mL, 0.117 mmol) was added and the mixture was shaken at room temperature for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and LCMS analysis was performed. As a result, the target material E0113 was observed as 95.1% and H-product (C01) was observed as 1.5%.

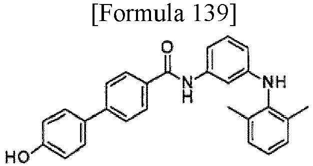

### Compound E0113

LRMS: m/z 409 [M+H]⁺
Retention time: 1.248 min (analysis condition FA05-1, 299 nm).

### Example 3-4-9: Synthesis of E0114-1R by C-N coupling reaction of compound B01-1R with 2,6-diethylaniline (e14)

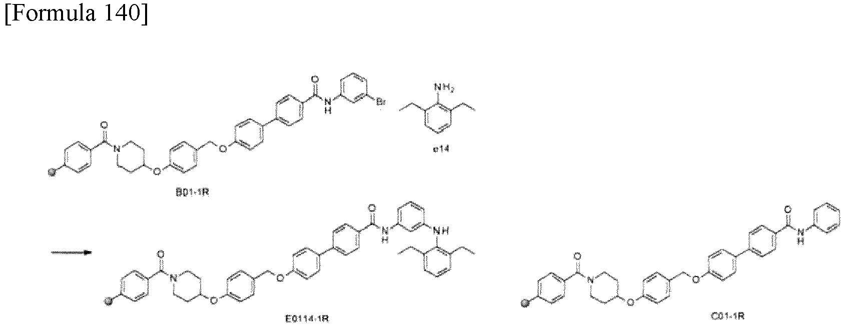

Under a nitrogen atmosphere, to a 0.6 mL glass vial, compound B01-1R (0.195 mmol/g, 20 mg, 0.0039 mmol) and DMPr (0.4 mL) were added, and the mixture was shaken at room temperature for 1 hour. 2,6-Diethylaniline (e14) (12.1 mL, 0.078 mmol) and (tBu)PhCPhos Pd G4 (0.6 mg, 0.00078 mmol) were added. A P2tBu/THF solution (2M, 58.5 mL, 0.117 mmol) was added and the mixture was shaken at room temperature for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and LCMS analysis was performed. As a result, the target material E0114 was observed as 90.2%, the substrate B01 as 3.8%, and H-product (C01) as 1.6%.

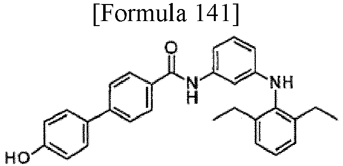

### Compound E0114

LRMS: m/z 437 [M+H]⁺
Retention time: 1.345 min (analysis condition FA05-1, 299 nm).

### Example 3-4-10: Synthesis of E0115-1R by C-N coupling reaction of compound B01-1R with N-methylaniline (e15)

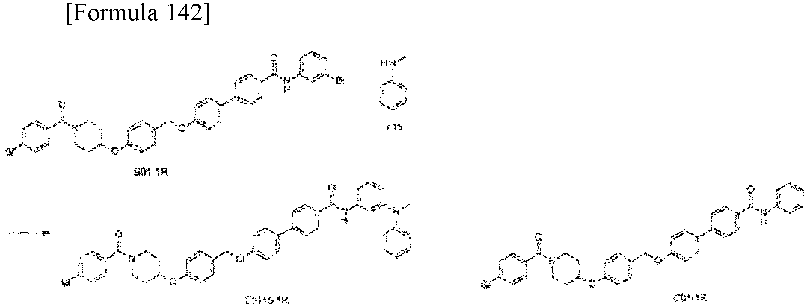

Under a nitrogen atmosphere, to a 0.6 mL glass vial, compound B01-1R (0.195 mmol/g, 20 mg, 0.0039 mmol) and DMPr (0.4 mL) were added, and the mixture was shaken at room temperature for 1 hour. N-methylaniline (e15) (8.4 mL, 0.078 mmol) and (tBu)PhCPhos Pd G4 (0.6 mg, 0.00078 mmol) were added. A P2tBu/THF solution (2M, 58.5 mL, 0.117 mmol) was added and the mixture was shaken at room temperature for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and LCMS analysis was performed. As a result, the target material E0115 was observed as 95.4%, and H-product (C01) was observed as 1.1%.

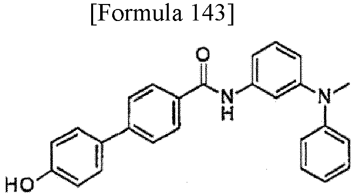

### Compound E0115

LRMS: m/z 395 [M+H]⁺
Retention time: 1.259 min (analysis condition FA05-1, 299 nm).

### Example 3-4-12: Synthesis of E0303-1R by C-N coupling reaction of compound B03-1R with dipropylamine (e03)

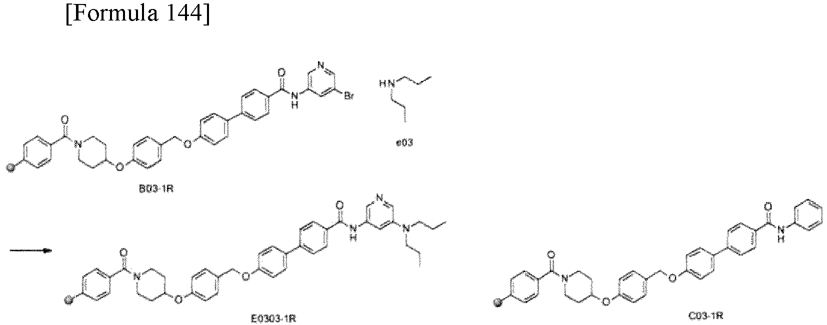

Under a nitrogen atmosphere, to a 0.6 mL glass vial, compound B01-1R (0.195 mmol/g, 20 mg, 0.0039 mmol) and DMPr (0.4 mL) were added, and the mixture was shaken at room temperature for 1 hour. Dipropylamine (e03) (10.7 mL, 0.078 mmol) and (tBu)PhCPhos Pd G4 (0.6 mg, 0.00078 mmol) were added. A P2tBu/THF solution (2M, 58.5 mL, 0.117 mmol) was added and the mixture was shaken at room temperature for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and LCMS analysis was performed. As a result, the target material E0303 was observed as 89.6%, and H-product (C03) was observed as 3.9%.

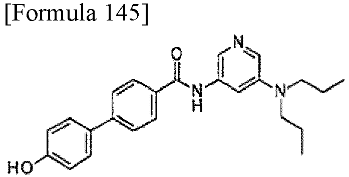

### Compound E0303

LRMS: m/z 390 [M+H]⁺
Retention time: 0.825 min (analysis condition FA05-1, 299 nm).

### Example 3-4-13: Synthesis of E0502-2R by C-N coupling reaction of compound B05-2R with N-methyl-3-phenylpropylamine (e02)

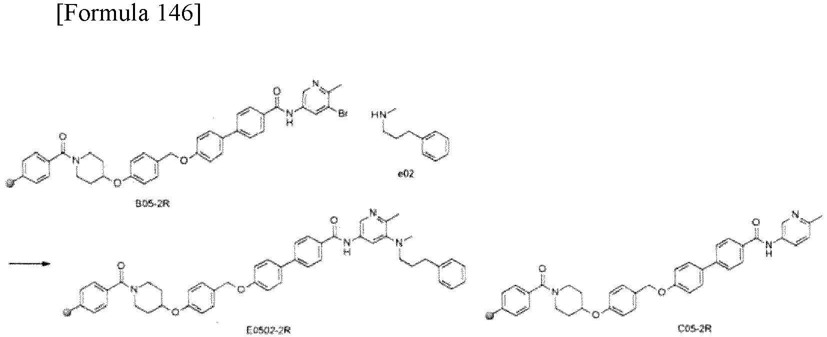

Under a nitrogen atmosphere, to a 0.6 mL glass vial, compound B05-2R (0.194 mmol/g, 20 mg, 0.0039 mmol) and DMPr (0.4 mL) were added, and the mixture was shaken at room temperature for 1 hour. N-methyl-3-phenylpropylamine (e02) (11.6 mg, 0.078 mmol) and (tBu)PhCPhos Pd G4 (0.6 mg, 0.00078 mmol) were added. A P2tBu/THF solution (2M, 58.2 mL, 0.116 mmol) was added and the mixture was shaken at room temperature for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 2 minutes. After filtration, the solid phase was washed with DMF (0.05 mL). MeCN (0.25 mL) was then added to the filtrate to prepare an LC sample, and LCMS analysis was performed. As a result, the target material E0502 was observed as 87.2%, and H-product (C05) was observed as 4.4%.

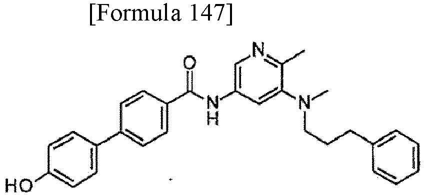

### Compound E0502

LRMS: m/z 452 [M+H]⁺
Retention time: 0.897 min (analysis condition FA05-1, 299 nm).

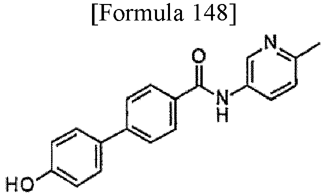

### Compound C05

LRMS: m/z 305 [M+H]⁺
Retention time: 0.605 min (analysis condition FA05-1, 299 nm).

### Example 3-4-14: Synthesis of E0101-3R by C-N coupling reaction of compound B08-3R with 4-phenylpiperidine (e01)

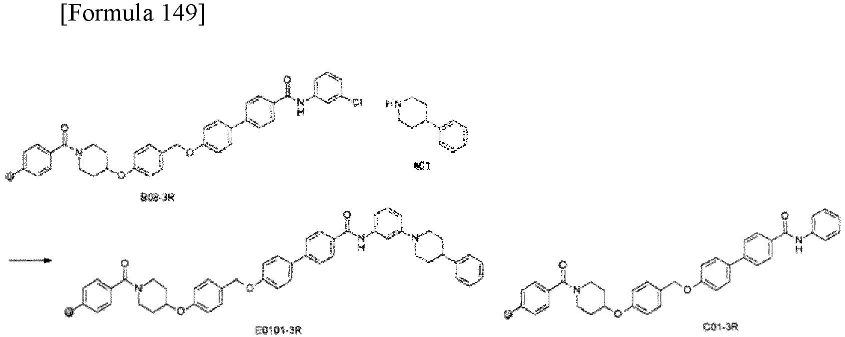

Under a nitrogen atmosphere, to a 0.6 mL glass vial, compound B08-3R (0.197 mmol/g, 15 mg, 0.0029 mmol) and a DMPr solution (0.3 mL) of 4-phenylpiperidine (e01) (4.8 mg, 0.030 mmol) were added, and the mixture was shaken at room temperature for 1 hour. RuPhos Pd G4 (0.5 mg, 0.00059 mmol) was added. A P2tBu/THF solution (2M, 22.5 mL, 0.045 mmol) was added and the mixture was shaken at 80°C for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM (0.05 mL) for 1 minute.

After filtration, the solid phase was washed with DMF (0.05 mL). MeCN (0.25 mL) was then added to the filtrate to prepare an LC sample, and LCMS analysis was performed. As a result, the target material E0101 was observed as 51.2%, H-product (C01) was observed as 7.5%, and the starting material (B08) was observed as 39.8%.

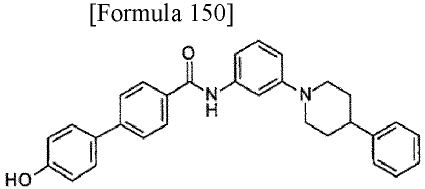

### Compound E0101

LRMS: m/z 449 [M+H]⁺
Retention time: 0.999 min (analysis condition FA05-1, 299 nm).
Retention time: 2.397 min (analysis condition FA05-long, 299 nm).

### Example 3-4-15: Synthesis of E0101-3R by C-N coupling reaction of compound B09-3R with 4-phenylpiperidine (e01)

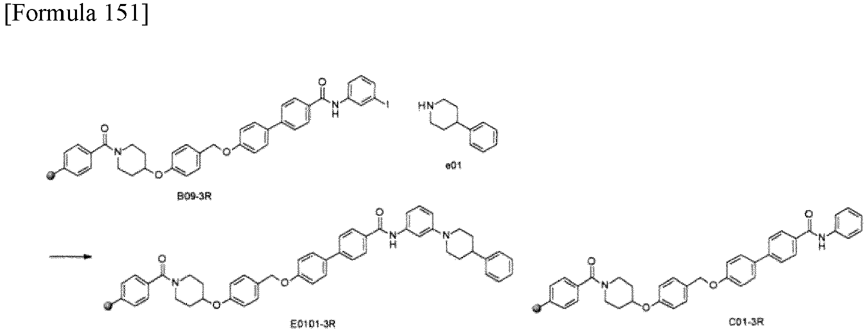

Under a nitrogen atmosphere, to a 0.6 mL glass vial, compound B09-3R (0.193 mmol/g, 15 mg, 0.0029 mmol) and a DMPr solution (0.3 mL) of 4-phenylpiperidine (e01) (4.8 mg, 0.030 mmol) were added, and the mixture was shaken at room temperature for 1 hour. A mixed solution of Pd₂dba₃CHCl₃ (0.025 M, 0.0012 mmol) and (tBu)PhCPhos (0.1 M, 0.0003 mmol) in DMPr (prepared by adding two compounds to DMPr and heating with a dryer for about 1 minute) (12.0 mL) was added. A P2tBu/THF solution (2M, 22.5 mL, 0.045 mmol) was added and the mixture was shaken at 80°C for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in TFA/HFIP/DCM = 2/9/9 (0.05 mL) for 1 minute. After filtration, the solid phase was washed with DMF (0.05 mL). MeCN (0.25 mL) was then added to the filtrate to prepare an LC sample, and LCMS analysis was performed. As a result, the target material E0101 was observed as 71.3%, and H-product (C01) was observed as 12.6%.

### Example 3-4-16: Synthesis of E1001-3R by C-N coupling reaction of compound B10-3R with 4-phenylpiperidine (e01)

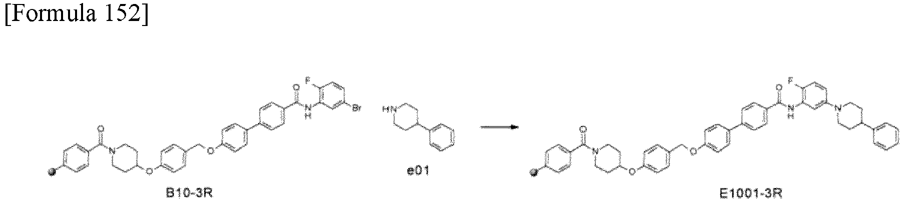

Under a nitrogen atmosphere, to a 0.6 mL glass vial, compound B10-3R (0.194 mmol/g, 15 mg, 0.0029 mmol) and a DMPr solution (0.3 mL) of 4-phenylpiperidine (e01) (4.8 mg, 0.030 mmol) were added, and the mixture was shaken at room temperature for 1 hour. A mixed solution of Pd₂dba₃CHCl₃ (0.025 M, 0.0012 mmol) and (tBu)PhCPhos (0.1 M, 0.0003 mmol) in DMPr (prepared by adding two compounds to DMPr and heating with a dryer for about 1 minute) (12.0 mL) was added. A P2tBu/THF solution (2M, 22.5 mL, 0.045 mmol) was added and the mixture was shaken at 80°C for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in TFA/HFIP/DCM = 2/9/9 (0.05 mL) for 1 minute. After filtration, the solid phase was washed with DMF (0.05 m). MeCN (0.25 mL) was then added to the filtrate to prepare an LC sample, and LCMS analysis was performed. As a result, the target material E1001 was observed as 90.8%.

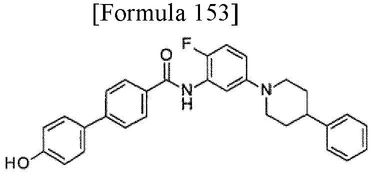

### Compound E1001

LRMS: m/z 467 [M+H]⁺
Retention time: 1.060 min (analysis condition FA05-1, 299 nm).

### Example 3-4-17: Synthesis of E0401-3R by C-N coupling reaction of compound B04-3R with 4-phenylpiperidine (e01)

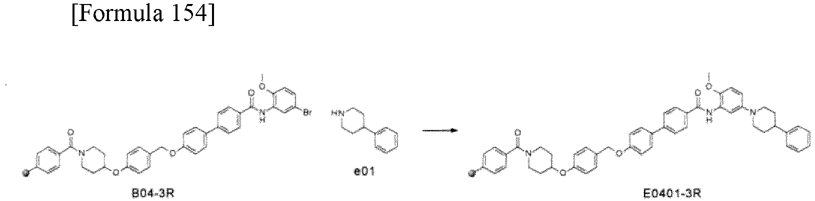

Under a nitrogen atmosphere, to a 0.6 mL glass vial, compound B04-3R (0.194 mmol/g, 15 mg, 0.0029 mmol) and a DMPr solution (0.3 mL) of 4-phenylpiperidine (e01) (4.8 mg, 0.030 mmol) were added, and the mixture was shaken at room temperature for 1 hour. A mixed solution of Pd₂dba₃CHCl₃ (0.025 M, 0.0012 mmol) and (tBu)PhCPhos (0.1 M, 0.0003 mmol) in DMPr (prepared by adding two compounds to DMPr and heating with a dryer for about 1 minute) (12.0 mL) was added. A P2tBu/THF solution (2M, 22.5 mL, 0.045 mmol) was added and the mixture was shaken at 80°C for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in TFA/HFIP/DCM = 2/9/9 (0.05 mL) for 1 minute. After filtration, the solid phase was washed with DMF (0.05 mL). MeCN (0.25 mL) was then added to the filtrate to prepare an LC sample, and LCMS analysis was performed. As a result, the target material E0401 was observed as 94.7%.

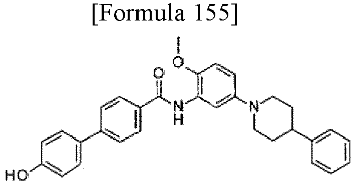

### Compound E0401

LRMS: m/z 479 [M+H]⁺
Retention time: 0.875 min (analysis condition FA05-1, 299 nm).

### Example 3-4-18: Synthesis of E1101-3R by C-N coupling reaction of compound B11-3R with 4-phenylpiperidine (e01)

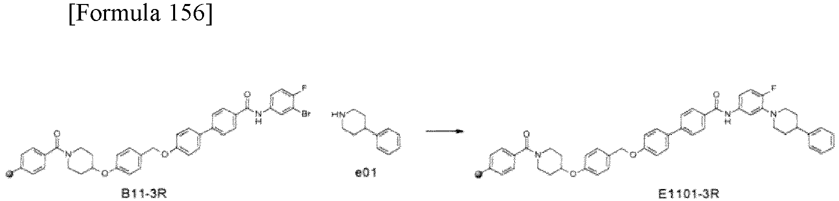

Under a nitrogen atmosphere, to a 0.6 mL glass vial, compound B11-3R (0.194 mmol/g, 15 mg, 0.0029 mmol) and a DMPr solution (0.3 mL) of 4-phenylpiperidine (e01) (4.8 mg, 0.030 mmol) were added, and the mixture was shaken at room temperature for 1 hour. A mixed solution of Pd₂dba₃CHCl₃ (0.025 M, 0.0012 mmol) and (tBu)PhCPhos (0.1 M, 0.0003 mmol) in DMPr (prepared by adding two compounds to DMPr and heating with a dryer for about 1 minute) (12.0 mL) was added. A P2tBu/THF solution (2M, 22.5 mL, 0.045 mmol) was added and the mixture was shaken at 80°C for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in TFA/HFIP/DCM = 2/9/9 (0.05 mL) for 1 minute. After filtration, the solid phase was washed with DMF (0.05 mL). MeCN (0.25 mL) was then added to the filtrate to prepare an LC sample, and LCMS analysis was performed. As a result, the target material E1101 was observed as 92.2%.

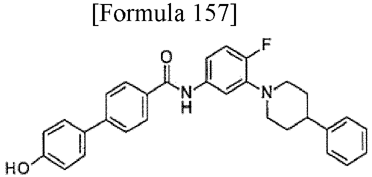

### Compound E1101

LRMS: m/z 467 [M+H]⁺
Retention time: 1.219 min (analysis condition FA05-1, 299 nm).

### Example 3-4-19: Synthesis of E1201-3R by C-N coupling reaction of compound B12-3R with 4-phenylpiperidine (e01)

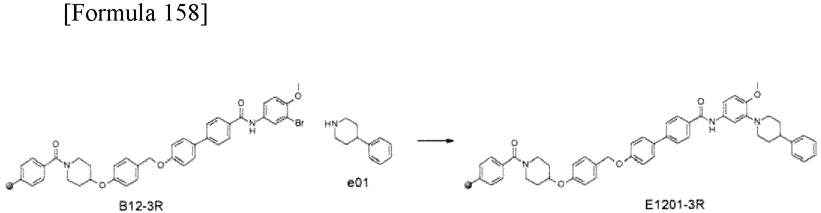

Under a nitrogen atmosphere, to a 0.6 mL glass vial, compound B12-3R (0.194 mmol/g, 15 mg, 0.0029 mmol) and a DMPr solution (0.3 mL) of 4-phenylpiperidine (e01) (4.8 mg, 0.030 mmol) were added, and the mixture was shaken at room temperature for 1 hour. A mixed solution of Pd₂dba₃CHCl₃ (0.025 M, 0.0012 mmol) and (tBu)PhCPhos (0.1 M, 0.0003 mmol) in DMPr (prepared by adding two compounds to DMPr and heating with a dryer for about 1 minute) (12.0 mL) was added. A P2tBu/THF solution (2M, 22.5 mL, 0.045 mmol) was added and the mixture was shaken at 80°C for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in TFA/HFIP/DCM = 2/9/9 (0.05 mL) for 1 minute. After filtration, the solid phase was washed with DMF (0.05 mL). MeCN (0.25 mL) was then added to the filtrate to prepare an LC sample, and LCMS analysis was performed. As a result, the target material E1201 was observed as 97.7%.

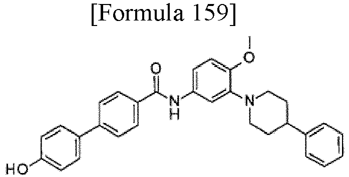

### Compound E1201

LRMS: m/z 479 [M+H]⁺
Retention time: 0.867 min (analysis condition FA05-1, 299 nm).

### Example 3-4-20: Synthesis of E1301-3R by C-N coupling reaction of compound B13-3R with 4-phenylpiperidine (e01)

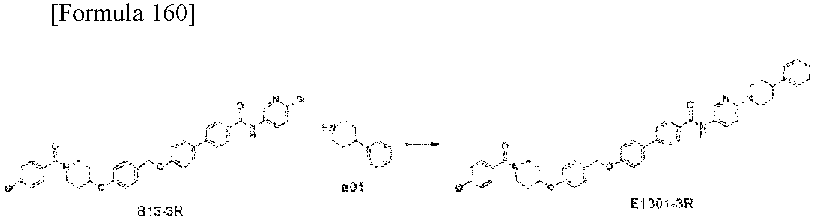

Under a nitrogen atmosphere, to a 0.6 mL glass vial, compound B13-3R (0.195 mmol/g, 15 mg, 0.0029 mmol) and a DMPr solution (0.3 mL) of 4-phenylpiperidine (e01) (4.8 mg, 0.030 mmol) were added, and the mixture was shaken at room temperature for 1 hour. A mixed solution of Pd₂dba₃CHCl₃ (0.025 M, 0.0012 mmol) and (tBu)PhCPhos (0.1 M, 0.0003 mmol) in DMPr (prepared by adding two compounds to DMPr and heating with a dryer for about 1 minute) (12.0 mL) was added. A P2tBu/THF solution (2M, 22.5 mL, 0.045 mmol) was added and the mixture was shaken at 80°C for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in TFA/HFIP/DCM = 2/9/9 (0.05 mL) for 1 minute. After filtration, the solid phase was washed with DMF (0.05 mL). MeCN (0.25 mL) was then added to the filtrate to prepare an LC sample, and LCMS analysis was performed. As a result, the target material E1301 was observed as 88.6%.

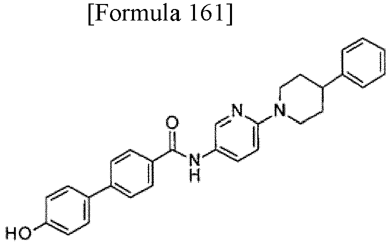

### Compound E1301

LRMS: m/z 450 [M+H]⁺
Retention time: 0.840 min (analysis condition FA05-1, 299 nm).

### Example 3-4-21: Synthesis of E1401-3R by C-N coupling reaction of compound B14-3R with 4-phenylpiperidine (e01)

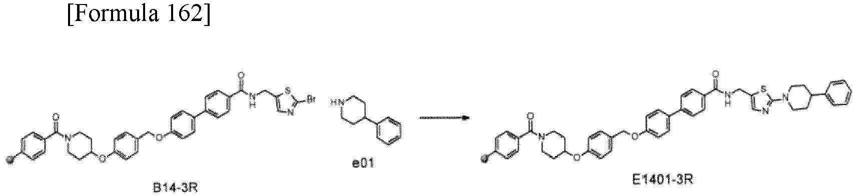

Under a nitrogen atmosphere, to a 0.6 mL glass vial, compound B14-3R (0.194 mml/g, 15 mg, 0.0029 mmol) and a DMPr solution (0.3 mL) of 4-phenylpiperidine (e01) (4.8 mg, 0.030 mmol) were added, and the mixture was shaken at room temperature for 1 hour. A mixed solution of Pd₂dba₃CHCl₃ (0.025 M, 0.0012 mmol) and (tBu)PhCPhos (0.1 M, 0.0003 mmol) in DMPr (prepared by adding two compounds to DMPr and heating with a dryer for about 1 minute) (12.0 mL) was added. A P2tBu/THF solution (2M, 22.5 mL, 0.045 mmol) was added and the mixture was shaken at 80°C for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in TFA/HFIP/DCM = 2/9/9 (0.05 mL) for 1 minute. After filtration, the solid phase was washed with DMF (0.05 mL). MeCN (0.25 mL) was then added to the filtrate to prepare an LC sample, and LCMS analysis was performed. As a result, the target material E1401 was observed as 79.0%.

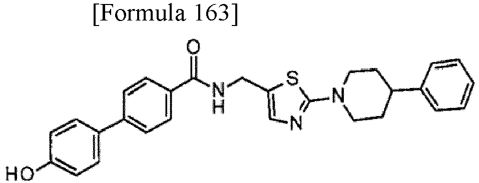

### Compound E1401

LRMS: m/z 470 [M+H]⁺
Retention time: 0.848 min (analysis condition FA05-1, 299 nm).

### Example 3-4-22: Synthesis of E1501-1R by C-N coupling reaction of compound B15-1R with 4-phenylpiperidine (e01)

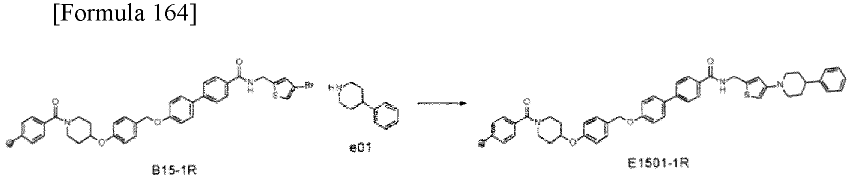

Under a nitrogen atmosphere, to a 0.6 mL glass vial, compound B15-1R (0.194 mmol/g, 15 mg, 0.0029 mmol) and a DMPr solution (0.3 mL) of 4-phenylpiperidine (e01) (4.8 mg, 0.030 mmol) were added, and the mixture was shaken at room temperature for 1 hour. A mixed solution of Pd₂dba₃CHCl₃ (0.025 M, 0.0012 mmol) and (tBu)PhCPhos (0.1 M, 0.0003 mmol) in DMPr (prepared by adding two compounds to DMPr and heating with a dryer for about 1 minute) (12.0 mL) was added. A P2tBu/THF solution (2M, 22.5 mL, 0.045 mmol) was added and the mixture was shaken at 80°C for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in TFA/HFIP/DCM = 2/9/9 (0.05 mL) for 1 minute. After filtration, the solid phase was washed with DMF (0.05 mL). MeCN (0.25 mL) was then added to the filtrate to prepare an LC sample, and LCMS analysis was performed. As a result, the target material E1501 was observed as 62.4%.

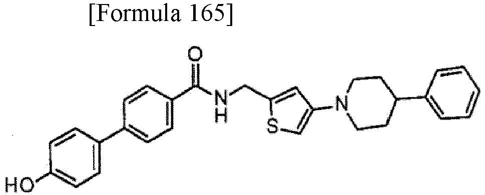

### Compound E1501

LRMS: m/z 469 [M+H]⁺
Retention time: 0.976 min (analysis condition FA05-1, 299 nm).

### Example 3-4-23: Synthesis of G0401-3R by C-N coupling reaction of compound H04-3R with methyl 4-bromobenzoate (g01)

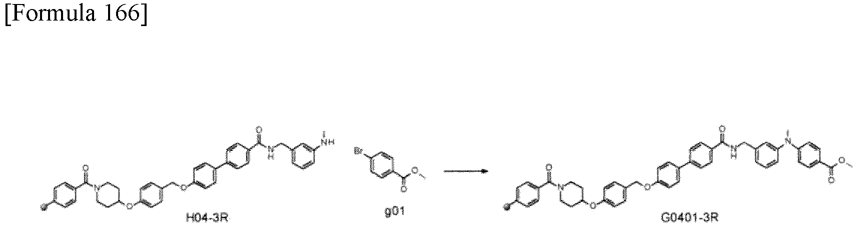

Under a nitrogen atmosphere, to a 0.6 mL glass vial, compound H04-3R (0.196 mmol/g, 15 mg, 0.0029 mmol) and methyl 4-bromobenzoate (g01) (6.3 mg, 0.029 mmol) and DMPr (0.3 mL) were added, and the mixture was shaken at room temperature for 1 hour. RuPhos Pd G4 (2.5 mg, 0.0029 mmol) and BTPP (13.5 mL, 0.044 mmol) were added and the mixture was shaken at 80°C for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 1 minute. After filtration, the solid phase was washed with DMF (0.05 mL). MeCN (0.25 mL) was then added to the filtrate to prepare an LC sample, and LCMS analysis was performed. As a result, the target material G0401 was observed as 92.9%.

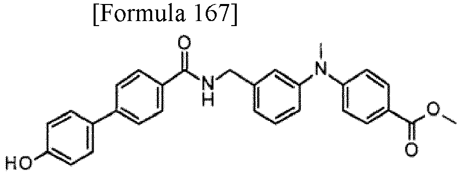

### Compound G0401

LRMS: m/z 467 [M+H]⁺
Retention time: 1.133 min (analysis condition FA05-1, 299 nm).

### Example 3-4-24: Synthesis of G0401-3R by CN coupling reaction of compound H04-3R with methyl 4-chlorobenzoate (g04)

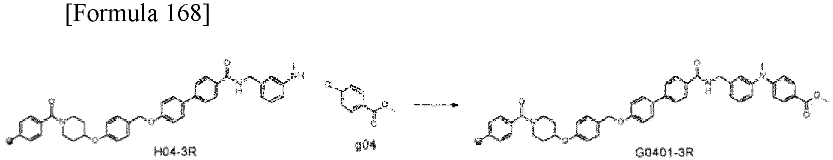

Under a nitrogen atmosphere, to a 0.6 mL glass vial, compound H04-3R (0.196 mmol/g, 15 mg, 0.0029 mmol), 4-chlorobenzoate (g04)(5.0 mg, 0.029 mmol) and DMPr (0.3 mL) were added, and the mixture was shaken at room temperature for 1 hour. RuPhos Pd G4 (2.5 mg, 0.0029 mmol) and BTPP (13.5 mL, 0.044 mmol) were added and the mixture was shaken at 80°C for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 1 minute. After filtration, the solid phase was washed with DMF (0.05 mL). MeCN (0.25 mL) was then added to the filtrate to prepare an LC sample, and LCMS analysis was performed. As a result, the target material G0401 was observed as 90.3%.

### Example 3-4-25: Synthesis of G0401-3R by C-N coupling reaction of compound H04-3R with methyl 4-iodobenzoate (g05)

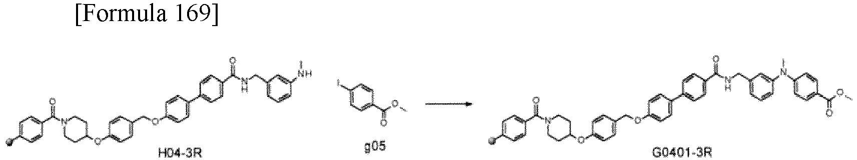

Under a nitrogen atmosphere, to a 0.6 mL glass vial, compound H04-3R (0.196 mmol/g, 15 mg, 0.0029 mmol) and methyl 4-iodobenzoate (g05) (7.7 mg, 0.029 mmol) and DMPr (0.3 mL) were added, and the mixture was shaken at room temperature for 1 hour. RuPhos Pd G4 (2.5 mg, 0.0029 mmol) and BTPP (13.5 mL, 0.044 mmol) were added and the mixture was shaken at 80°C for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 1 minute. After filtration, the solid phase was washed with DMF (0.05 mL). MeCN (0.25 mL) was then added to the filtrate to prepare an LC sample, and LCMS analysis was performed. As a result, the target material G0401 was observed as 89.8%.

### Example 3-4-26: Synthesis of G0202-4R by C-N coupling reaction of compound H02-4R with ethyl 4-bromopicolinate (g02)

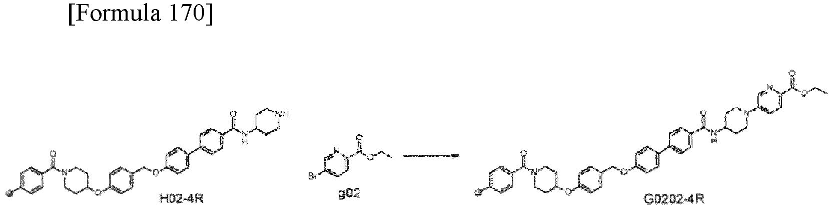

Under a nitrogen atmosphere, to a 0.6 mL glass vial, compound H02-4R (0.198 mmol/g, 15 mg, 0.0030 mmol) and ethyl 5-bromopicolinate (g02) (6.8 mg, 0.030 mmol) and DMPr (0.3 mL) were added, and the mixture was shaken at room temperature for 1 hour. RuPhos Pd G4 (2.5 mg, 0.0030 mmol) and BTPP (13.6 mL, 0.045 mmol) were added and the mixture was shaken at 80°C for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 1 minute. After filtration, the solid phase was washed with DMF (0.05 mL). MeCN (0.25 mL) was then added to the filtrate to prepare an LC sample, and LCMS analysis was performed. As a result, the target material G0202 was observed as 89.5%.

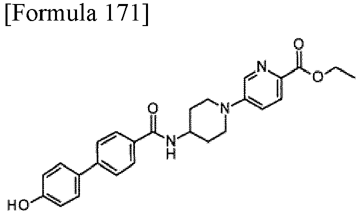

### Compound G0202

LRMS: m/z 446 [M+H]⁺
Retention time: 0.880 min (analysis condition FA05-1, 299 nm).

### Example 3-4-27: Synthesis of G0402-3R by C-N coupling reaction of compound H04-3R with ethyl 5-bromopicolinate (g02)

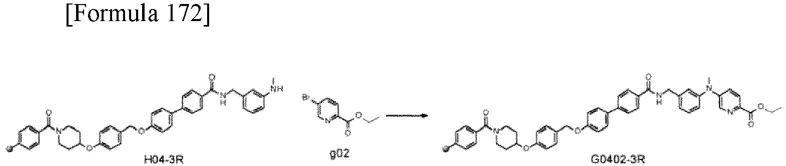

Under a nitrogen atmosphere, to a 0.6 mL glass vial, compound H04-3R (0.196 mmol/g, 15 mg, 0.0029 mmol) and ethyl 5-bromopicolinate (g02) (6.8 mg, 0.029 mmol) and DMPr (0.3 mL) were added, and the mixture was shaken at room temperature for 1 hour. RuPhos Pd G4 (2.5 mg, 0.0029 mmol) and BTPP (13.5 mL, 0.044 mmol) were added and the mixture was shaken at 80°C for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 1 minute. After filtration, the solid phase was washed with DMF (0.05 mL). MeCN (0.25 mL) was then added to the filtrate to prepare an LC sample, and LCMS analysis was performed. As a result, the target material G0402 was observed as 87.0%.

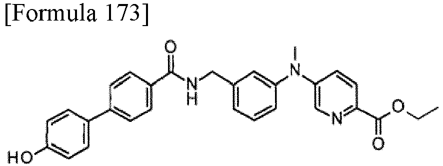

### Compound G0402

LRMS: m/z 482 [M+H]⁺
Retention time: 0.983 min (analysis condition FA05-1, 299 nm).

### Example 3-4-28: Synthesis of G0103-4R by C-N coupling reaction of compound H01-4R with ethyl 2-bromothiazole-4-carboxylate (g03)

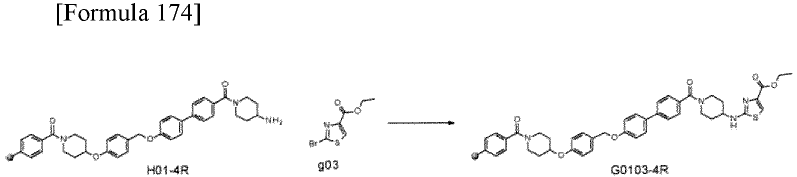

Under a nitrogen atmosphere, to a 0.6 mL glass vial, compound H01-4R (0.198 mmol/g, 15 mg, 0.0030 mmol) and ethyl 2-bromothiazole-4-carboxylate (g03) (7.0 mg, 0.030 mmol) and DMPr (0.3 mL) were added, and the mixture was shaken at room temperature for 1 hour. tBuXPhos Pd G4 (2.5 mg, 0.0030 mmol) and BTPP (13.6 mL, 0.045 mmol) were added and the mixture was shaken at 80°C for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 1 minute. After filtration, the solid phase was washed with DMF (0.05 mL). MeCN (0.25 mL) was then added to the filtrate to prepare an LC sample, and LCMS analysis was performed. As a result, the target material G0103 was observed as 89.5%.

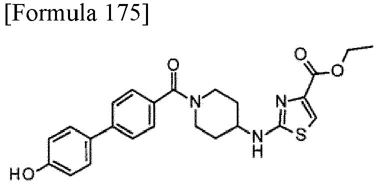

### Compound G0103

LRMS: m/z 452 [M+H]⁺
Retention time: 0.919 min (analysis condition FA05-1, 299 nm).

### Example 3-4-29: Synthesis of G0303-4R by C-N coupling reaction of compound H03-4R with ethyl 2-bromothiazole-4-carboxylate (g03)

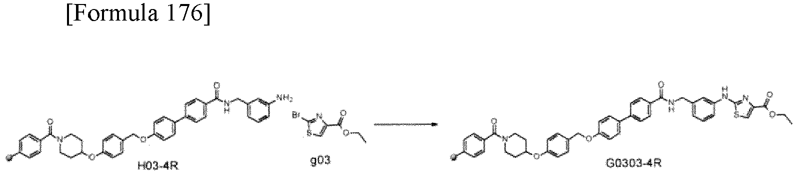

Under a nitrogen atmosphere, to a 0.6 mL glass vial, compound H03-4R (0.197 mmol/g, 15 mg, 0.0030 mmol) and ethyl 2-bromothiazole-4-carboxylate (g03) (7.0 mg, 0.030 mmol) and DMPr (0.3 mL) were added, and the mixture was shaken at room temperature for 1 hour. tBuXPhos Pd G4 (2.4 mg, 0.0030 mmol) and BTPP (13.6 mL, 0.045 mmol) were added and the mixture was shaken at 80°C for 24 hours.

The suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing 0.02 M pentamethylbenzene (0.05 mL) for 1 minute. After filtration, the solid phase was washed with DMF (0.05 mL). MeCN (0.25 mL) was then added to the filtrate to prepare an LC sample, and LCMS analysis was performed. As a result, the target material G0303 was observed as 87.6%.

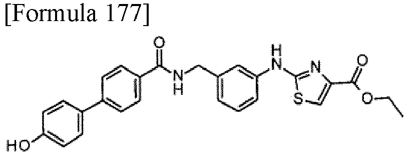

### Compound G0303

LRMS: m/z 474 [M+H]⁺
Retention time: 1.001 min (analysis condition FA05-1, 299 nm).

### Example 5: Application of Pd coupling conditions to mixture

### Example 5-1: Synthesis of substrate mixture

### Example 5-1-1: Synthesis of mixture 2-2-B00-0

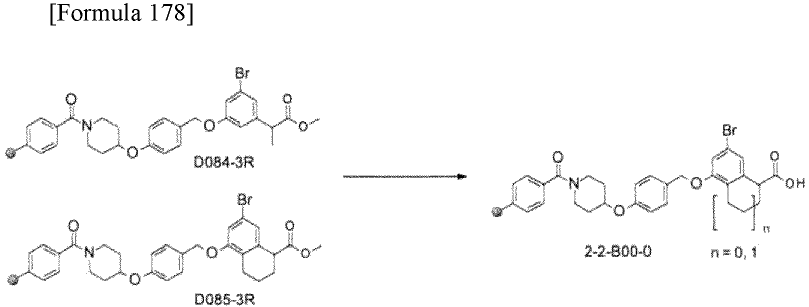

D084-3R and D085-3R can be prepared under the same conditions as described in Example 1-2-1 using separately prepared methyl 2-(3-bromo-5-((4-(piperidin-4-yloxy)benzyl)oxy)phenyl)propanoate and methyl 7-bromo-5-(((4-(piperidin-4-yloxy)benzyl)oxy)-1,2,3,4-tetrahydronaphthalene-1-carboxylate.

To a 200 mL column with a filter, compound D084-3R (3.2 g, 0.200 mmol/g), compound D085-3R (3.2 g, 0.200 mmol/g), NMP (90 mL), and tAmylOH (25.6 mL) were added. The column was capped to prevent leakage of the reaction solution, and shaken at room temperature for 5 minutes. An aqueous TBAOH solution (1M, 256 mL, 2.56 mmol) was added. The column was capped to prevent leakage of the reaction solution, and shaken at room temperature for 1 hour. The suspension of the reaction solution and solid phase was filtered through the filter of the column and washed 3 times with NMP/water = 1/1 (128 mL), 3 times with NMP (128 mL), 3 times with a NMP solution mixed with tetrabutylammonium bisulfate and 2,6-di-tert-butyl pyridine (both 0.05 M, 128 mL), 3 times with NMP (128 mL), 3 times with MeOH (128 mL), 3 times with DCM (128 mL), and 3 times with heptane (128 mL). The resulting solid phase was dried under reduced pressure to afford mixture 2-2-B00-0 (6.81 g, 0.201 mmol/g).

The compounds contained in mixture 2-2-B00-0 are as shown in [Table 5-1-1]. In [Table 5-1-1], each compound contained in the mixture is represented by ID, and the structure is represented by indicating the corresponding n-number in the structural formula of the mixture. That is, compound 2-2-B00-0-0001 has the n-number of 0 and represents compound D084-3R, and compound 2-2-B00-0-0002 has the n-number of 1 and represents compound D085-3R.

**[Table 23]**

| [Table 5-1-1] Compound contained in mixture 2-2-B00-0 | |
|---|---|
| ID | n-number |
| 2-2-B00-0-0001 | 0 |
| 2-2-B00-0-0002 | 1 |

### Example 5-1-2: Synthesis of mixtures 2-2-a1B01-0 to 2-2-a1B05-0

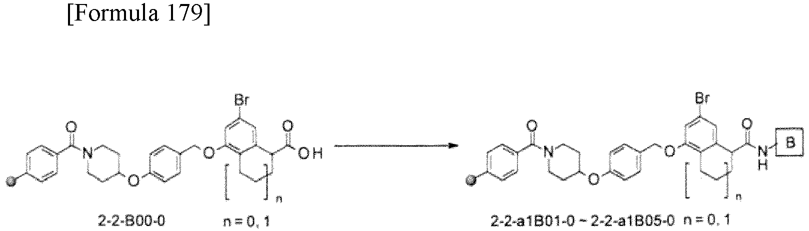

To five 20 mL columns with filters, mixture 2-2-B00-0 (1.28 g, 0.201 mmol/g) and DCM (15.4 mL) were added. The amines (1.80 mmol) described in Table 5-1-2-0 were added separately to each column. The column was capped to prevent leakage of the reaction solution, and shaken at room temperature for 1 hour. To this, a MeCN solution mixed with PipClU and NMI (both 1M, 0.013 mL, 0.770 mmol) was added. The column was capped to prevent leakage of the reaction solution, and shaken at room temperature for 2 hours.

The suspension of the reaction solution and solid phase was filtered through the filter of the column and washed 3 times with NMP/water = 1/1 (25 mL), 3 times with NMP (25 mL), 3 times with MeOH (25 mL), 3 times with DCM (25 mL), and 1 time with heptane (25 mL). The resulting solid phase was dried under reduced pressure to afford the mixture as described in Table 5-1-2-0.

**[Table 24]**

| [Table 5-1-2-0] | | | |
|---|---|---|---|
| Compound number of amine | Compound name of amine | Amount used | Number of resulting mixture |
| e17 | aniline | 0.164 mL | 2-2-a1B01-0 |
| e18 | 4-methylaniline | 0.193 g | 2-2-a1B02-0 |
| e19 | 4-fluoroaniline | 0.170 mL | 2-2-a1B03-0 |
| e20 | 1,3-benzothiazole-2-amine | 0.270 g | 2-2-a1B04-0 |
| e21 | 4-(trifluoromethyl)aniline | 0.226 mL | 2-2-a1B05-0 |

The compounds contained in mixtures 2-2-a1B01-0 to 2-2-a1B05-0 are as shown in [Table 5-1-2-1] to [Table 5-1-2-5]. In [Table 5-1-2-1] to [Table 5-1-2-5], each compound contained in the mixture is represented by ID, and the structure is represented by indicating the corresponding n-number and part B by a symbol in the structural formula of the mixture. Unless otherwise noted, the correspondence between the symbol for part B and the structural formula herein is as shown below.

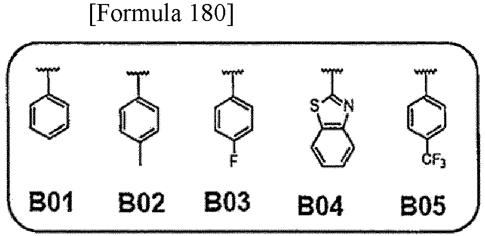

For example, compound 2-2-a1B01-0-0001 has the n-number of 0 and part B of B01 and represents the following structure.

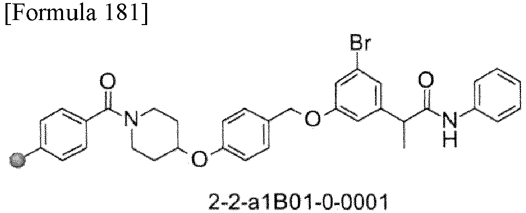

Further, for example, compound 2-2-a1B02-0-0002 has the n-number of 1 and part B of B02 and represents the following structure.

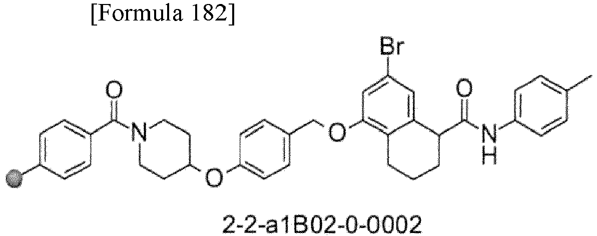

**[Table 25]**

| [Table 5-1-2-1] Compound contained in mixture 2-2-a1B01-0 | | |
|---|---|---|
| ID | n-number | B |
| 2-2-a1B01-0-0001 | 0 | B01 |
| 2-2-a1B01-0-0002 | 1 | B01 |

**[Table 26]**

| [Table 5-1-2-2] Compound contained in mixture 2-2-a1B02-0 | | |
|---|---|---|
| ID | n-number | B |
| 2-2-a1B02-0-0001 | 0 | B02 |
| 2- 2-a1B02-0-0002 | 1 | B02 |

**[Table 27]**

| [Table 5-1-2-3] Compound contained in mixture 2-2-a1B03-0 | | |
|---|---|---|
| ID | n-number | B |
| 2-2-a1B03-0-0001 | 0 | B03 |
| 2-2-a1B03-0-0002 | 1 | B03 |

**[Table 28]**

| [Table 5-1-2-4] Compound contained in mixture 2-2-a1B04-0 | | |
|---|---|---|
| ID | n-number | B |
| 2-2-a1B04-0-0001 | 0 | B04 |
| 2-2-a1B04-0-0002 | 1 | B04 |

**[Table 29]**

| [Table 5-1-2-5] Compound contained in mixture 2-2-a1B05-0 | | |
|---|---|---|
| ID | n-number | B |
| 2-2-a1B05-0-0001 | 0 | B05 |
| 2-2-a1B05-0-0002 | 1 | B05 |

### Example 5-1-3: Preparation of mixture 2-2-C00-0

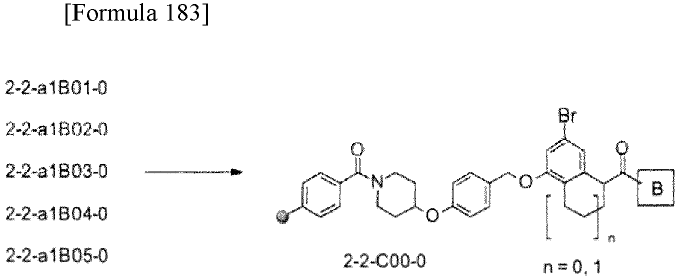

To a 120 mL column with a filter, five mixtures as described in [Table 5-1-3-0] and DCM (70 mL) were added. After 1 minute, the suspension of solvent and solid phase was filtered through the filter of the column, washed once with heptane (70 mL), and the resulting solid phase was dried under reduced pressure to obtain mixture 2-2-C00-0 (3.67 g, 0.196 mmol/g).

**[Table 30]**

| [Table 5-1-3-0] | |
|---|---|
| Mixture | Amount used |
| 2-2-a1B01-0 | 0.700 g |
| 2-2-a1B02-0 | 0.700 g |
| 2-2-a1B03-0 | 0.700 g |
| 2-2-a1B04-0 | 0.700 g |
| 2-2-a1B05-0 | 0.700 g |

The compounds contained in mixture 2-2-C00-0 are as shown in [Table 5-1-3-1]. In [Table 5-1-3-1], each compound contained in the mixture is represented by ID, and the structure is represented by indicating the corresponding n-number and part B by a symbol in the structural formula of the mixture. The correspondence between the symbol of part B and the structural formula is as shown in Example 5-1-2.

**[Table 31]**

| [Table 5-1-3-1] Compound contained in mixture 2-2-C00-0 | | |
|---|---|---|
| ID | n-number | B |
| 2-2-a1B01-0-0001 | 0 | B01 |
| 2-2-a1B01-0-0002 | 1 | B01 |
| 2-2-a1B02-0-0001 | 0 | B02 |
| 2-2-a1B02-0-0002 | 1 | B02 |
| 2-2-a1B03-0-0001 | 0 | B03 |
| 2-2-a1B03-0-0002 | 1 | B03 |
| 2-2-a1B04-0-0001 | 0 | B04 |
| 2-2-a1B04-0-0002 | 1 | B04 |
| 2-2-a1B05-0-0001 | 0 | B05 |
| 2-2-a1B05-0-0002 | 1 | B05 |

### Example 5-2: Application of Pd coupling conditions to mixture

### Example 5-2-1: Synthesis of mixture 2-2-b3C00-0 by hydroxylation of mixture 2-2-C00-0

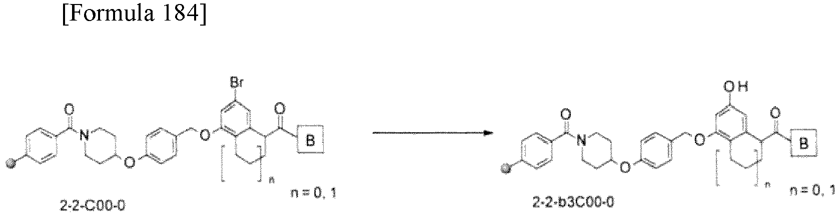

To a 15 mL glass vial, mixture 2-2-C00-0 (550 mg, 0.196 mmol/g), DEAc (11.0 mL), and water (0.275 mL, 15.3 mmol) were added, and the mixture was shaken at room temperature for 1 hour. AdBrettPhos Pd G6 Br (21.0 mg, 0.022 mmol) and BTMG (0.275 mL, 1.38 mmol) were added and the mixture was shaken at 60°C for 15 hours.

The suspension of the reaction solution and solid phase was transferred to a column with a filter, and filtered, and then washed 3 times with NMP (11 mL), 3 times with a solution of N-acetyl-L-cysteine in NMP/water = 5/1 (0.2 M, 11 mL), 3 times with an NMP solution mixed with hydrogen sulfate tetrabutylammonium and 2,6-di-tert-butyl pyridine (both 0.05 M, 11 mL), 3 times with a solution of 4-methylmorpholine in NMP (0.05 M, 11 mL), 3 times with NMP/water = 1/1 (11 mL), 3 times with NMP (11 mL), 3 times with MeOH (11 mL), 3 times with DCM (11 mL), and once with heptane (11 mL). The resulting solid phase was dried under reduced pressure to afford mixture 2-2-b3C00-0.

The compounds that may be contained in 2-2-b3C00-0 are as shown in [Table 5-2-1-1]. In [Table 5-2-1-1], each compound that may be contained in the mixture is represented by ID, and the structure is represented by indicating the corresponding n-number and part B by a symbol in the structural formula of the mixture. The correspondence between the symbol of part B and the structural formula is as shown in Example 5-1-2.

**[Table 32]**

| [Table 5-2-1-1] Compound that may be contained in mixture 2-2-b3C00-0 | | |
|---|---|---|
| ID | n-number | B |
| 2-2-b3C00-0-0001 | 0 | B01 |
| 2-2-b3C00-0-0002 | 0 | B02 |
| 2-2-b3C00-0-0003 | 0 | B03 |
| 2-2-b3C00-0-0004 | 1 | B01 |
| 2-2-b3C00-0-0005 | 1 | B02 |
| 2-2-b3C00-0-0006 | 1 | B03 |
| 2-2-b3C00-0-0007 | 0 | B04 |
| 2-2-b3C00-0-0008 | 0 | B05 |
| 2-2-b3C00-0-0009 | 1 | B04 |
| 2-2-b3C00-0-0010 | 1 | B05 |

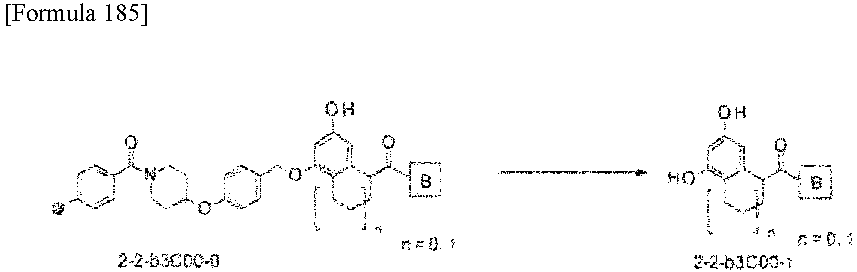

At the time of shaking at 60°C for 15 hours, 12 mL of the suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing pentamethylbenzene (0.02 M, 0.05 mL) for 1 minute. After filtration, the solid phase was washed with DMF (0.05 mL). MeCN (0.25 mL) was then added to the filtrate to obtain a solution of a mixture 2-2-b3C00-1. In LCMS analysis of this solution, all m/z ([M + H]+) derived from compounds that may be contained in the mixture 2-2-b3C00-1 described in [Table 5-2-1-2] were observed. In [Table 5-2-1-2], each compound that may be contained in the mixture is represented by ID, and the structure is represented by indicating the corresponding n-number and part B by a symbol in the structural formula of the mixture. This result showed that the mixture 2-2-b3C00-0 described in [Table 5-2-1-1] was correctly synthesized.

**[Table 33]**

| [Table 5-2-1-2] Compound that may be contained in mixture 2-2-b3C00-1 | | | | |
|---|---|---|---|---|
| ID | Exact Mass | m/z | n-number | B |
| 2-2-b3C00-1-0001 | 257.105 | 258 [M+H]⁺ | 0 | B01 |
| 2-2-b3C00-1-0002 | 271.121 | 272 [M+H]⁺ | 0 | B02 |
| 2-2-b3C00-1-0003 | 275.096 | 276 [M+H]⁺ | 0 | B03 |
| 2-2-b3C00-1-0004 | 283.121 | 284 [M+H]⁺ | 1 | B01 |
| 2-2-b3C00-1-0005 | 297.136 | 298 [M+H]⁺ | 1 | B02 |
| 2-2-b3C00-1-0006 | 301.111 | 302 [M+H]⁺ | 1 | B03 |
| 2-2-b3C00-1-0007 | 314.073 | 315 [M+H]⁺ | 0 | B04 |
| 2-2-b3C00-1-0008 | 325.093 | 326 [M+H]⁺ | 0 | B05 |
| 2-2-b3C00-1-0009 | 340.088 | 341 [M+H]⁺ | 1 | B04 |
| 2-2-b3C00-1-0010 | 351.108 | 352 [M+H]⁺ | 1 | B05 |

The above results showed that hydroxylation reaction using DEAc as the solvent is applicable even when the substrate is not a single compound, but a mixture of a plurality of compounds.

### Example 5-2-2: Synthesis of mixture 2-2-b1C19-0 by C-N coupling reaction of mixture 2-2-C00-0

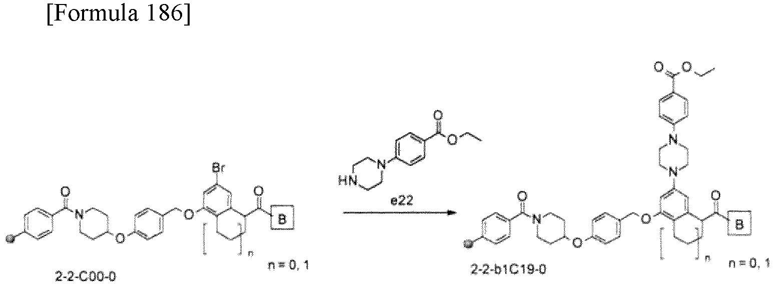

To a 4 mL glass vial, 2-2-C00-0 (128 mg, 0.196 mmol/g) and DMPr (2.56 mL) were added, and the mixture was shaken at room temperature for 1 hour. Ethyl 4-(piperazin-1-yl)benzoate (e22) (58.9 mg, 0.251 mmol) was added. (tBu)PhCPhos Pd G4 (19 mg, 0.025 mmol) and a solution of P2tBu in THF (2M, 0.189 mL, 0.377 mmol) were added and the mixture was shaken at 25°C for 1 hour.

The suspension of the reaction solution and solid phase was transferred to a 6 mL filter, and filtered, and then washed 3 times with NMP/water = 1/1 (2.6 mL), 3 times with NMP (2.6 mL), 3 times with a solution of N-acetyl-L-cysteine in NMP/water = 5/1 (0.2 M, 2.6 mL), 3 times with an NMP solution mixed with hydrogen sulfate tetrabutylammonium and 2,6-di-tert-butyl pyridine (both 0.05 M, 2.6 mL), 3 times with a solution of 4-methylmorpholine in NMP (0.05 M, 2.6 mL), 3 times with NMP/water = 1/1 (2.6 mL), 3 times with NMP (2.6 mL), 3 times with MeOH (2.8 mL), 3 times with DCM (2.8 mL), and 3 times with heptane (2.8 mL). The resulting solid phase was dried under reduced pressure to afford mixture 2-2-b1C19-0.

The compounds that may be contained in 2-2-b1C19-0 are as shown in [Table 5-2-2-1]. In [Table 5-2-2-1], each compound that may be contained in the mixture is represented by ID, and the structure is represented by indicating the corresponding n-number and part B by a symbol in the structural formula of the mixture.

**[Table 34]**

| [Table 5-2-2-1] Compound that may be contained in mixture 2-2-b1C19-0 | | |
|---|---|---|
| ID | n-number | B |
| 2-2-b1C19-0-0001 | 0 | B01 |
| 2-2-b1C19-0-0002 | 0 | B02 |
| 2-2-b1C19-0-0003 | 0 | B03 |
| 2-2-b1C19-0-0004 | 1 | B01 |
| 2-2-b1C19-0-0005 | 1 | B02 |
| 2-2-b1C19-0-0006 | 1 | B03 |
| 2-2-b1C19-0-0007 | 0 | B04 |
| 2-2-b1C19-0-0008 | 0 | B05 |
| 2-2-b1C19-0-0009 | 1 | B04 |
| 2-2-b1C19-0-0010 | 1 | B05 |

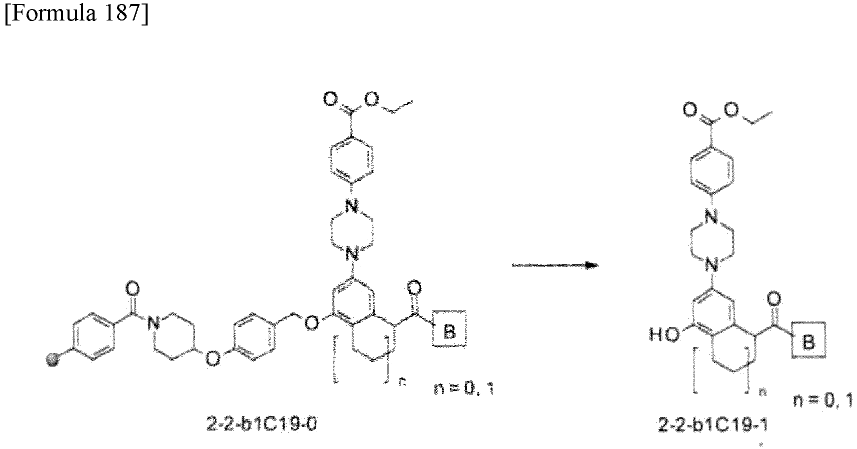

At the time of shaking at 25°C for 1 hour, 12 mL of the suspension of the reaction solution and the solid phase was transferred onto a filter, and washed 3 times with DMF (0.1 mL), 3 times with MeOH (0.1 mL), and 3 times with DCM (0.1 mL), and then immersed in a 10% TFA/DCM solution containing pentamethylbenzene (0.02 M, 0.05 mL) for 1 minute. After filtration, the solid phase was washed with DMF (0.05 mL). MeCN (0.25 mL) was then added to the filtrate to obtain a solution of a mixture 2-2-b1C19-1. In LCMS analysis of this solution, all m/z ([M + H]⁺) derived from compounds that may be contained in the mixture 2-2-b1C19-1 described in [Table 5-2-2-2] were observed. In [Table 5-2-2-2], each compound that may be contained in the mixture is represented by ID, and the structure is represented by indicating the corresponding n-number and part B by a symbol in the structural formula of the mixture. The correspondence between the symbol of part B and the structural formula is as shown in Example 5-1-2. This result showed that the mixture 2-2-b1C19-0 described in [Table 5-2-2-1] was correctly synthesized.

**[Table 35]**

| [Table 5-2-2-1] Compound that may be contained in mixture 2-2-b1C19-1 | | | | | |
|---|---|---|---|---|---|
| ID | Exact Mass | m/z | | n-number | B |
| 2-2-b1C19-1-0001 | 473.231 | 474 | [M+H]⁺ | 0 | B01 |
| 2-2-b1C19-1-0002 | 487.247 | 488 | [M+H]⁺ | 0 | B02 |
| 2-2-b1C19-1-0003 | 491.222 | 492 | [M+H]⁺ | 0 | B03 |
| 2-2-b1C19-1-0004 | 499.247 | 500 | [M+H]⁺ | 1 | B01 |
| 2-2-b1C19-1-0005 | 513.263 | 514 | [M+H]⁺ | 1 | B02 |
| 2-2-b1C19-1-0006 | 517.238 | 518 | [M+H]⁺ | 1 | B03 |
| 2-2-b1C19-1-0007 | 530.199 | 531 | [M+H]⁺ | 0 | B04 |
| 2-2-b1C19-1-0008 | 541.219 | 542 | [M+H]⁺ | 0 | B05 |
| 2-2-b1C19-1-0009 | 556.214 | 557 | [M+H]⁺ | 1 | B04 |
| 2-2-b1C19-1-0010 | 567.234 | 568 | [M+H]⁺ | 1 | B05 |

The above results showed that C-N coupling reaction using DMPr as the solvent is applicable even when the substrate is not a single compound, but a mixture of a plurality of compounds. It was also shown that in the production of compound libraries with various structures, it is possible to efficiently construct a compound library by performing a palladium coupling step in a plurality of compounds by applying the reaction conditions shown in Examples.

## Claims

1. A method of producing a compound by a cross-coupling reaction, the method comprising:
reacting compound 1 having a leaving group X¹ on a carbon atom of an aromatic ring with compound 2 having a reactive group capable of a C-O bond formation reaction or a C-N bond formation reaction by substitution with the leaving group, in the presence of a catalyst and a base, in a solvent containing an amide-based solvent represented by formula A: 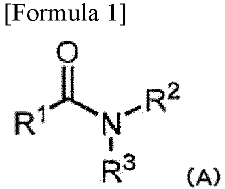
wherein R¹, R², and R³ are each independently C₁₋₄ alkyl provided that the sum of carbon atoms of R¹, R², and R³ is 4 or more and 6 or less, wherein
X¹ is a halogen atom or -O-SO₂-R⁴;
R⁴ is C₁₋₆ alkyl optionally substituted with one or more fluorine atoms, or phenyl optionally substituted with one or more fluorine atoms or C₁₋₆ alkyl optionally substituted with a fluorine atom; and
compound 2 has a hydroxy capable of forming a C-O bond or an H-N group capable of forming a C-N bond.

2. The method according to claim 1, wherein the catalyst is a palladium catalyst or a nickel catalyst.

3. The method according to claim 1 or 2, wherein compound 1 is a resin for solid-phase synthesis, having a leaving group X¹ on a carbon atom of an aromatic ring in a side chain, or a resin for solid-phase synthesis, having a reactive group capable of a C-O bond formation reaction or a C-N bond formation reaction by substitution with the leaving group in a side chain.

4. The method according to any one of claims 1 to 3, wherein compound 2 is
1) water or a compound having a hydroxy capable of forming a C-O bond, represented by HO-R⁵, wherein
R⁵ is C₁₋₆ alkyl, C₂₋₆ alkenyl, C₂₋₆ alkynyl, C₃₋₈ cycloalkyl, C₇₋₁₄ aralkyl, C₆₋₁₀ aryl, or 5- to 10-membered heteroaryl containing one or more ring heteroatoms independently selected from O, N and S, each of which is optionally substituted with one or more groups independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, aminocarbonyl, (C₁₋₆ alkyl)aminocarbonyl, di(C₁₋₆ alkyl)aminocarbonyl, and 4- to 8-membered cyclic aminocarbonyl; or
2) a compound having an H-N group capable of forming a C-N bond, represented by HNR⁶R⁷, wherein
R⁶ and R⁷ together with the nitrogen atom to which they are bonded form a 5- to 7-membered saturated heterocycle, wherein the heterocycle is optionally substituted with one or more substituents independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl, or
R⁶ and R⁷ are each independently a hydrogen atom, C₁₋₆ alkyl, C₂₋₆ alkenyl, C₂₋₆ alkynyl, C₃₋₈ cycloalkyl, (C₁₋₆ alkyl)carbonyl, (C₆₋₁₀ aryl)carbonyl, 5- to 10-membered heteroarylcarbonyl containing one or more ring heteroatoms independently selected from O, N and S, C₇₋₁₄ aralkyl, C₆₋₁₀ aryl, or 5- to 10-membered heteroaryl containing one or more ring heteroatoms independently selected from O, N and S, each of which is optionally substituted with one or more substituents independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl.

5. The method according to any one of claims 1 to 4, wherein compound 1 is a compound represented by X¹-Ar², wherein
X¹ is a chlorine atom, a bromine atom, an iodine atom, or -O-SO₂-R⁴;
R⁴ is C₁₋₆ alkyl optionally substituted with one or more fluorine atoms, or phenyl optionally substituted with one or more fluorine atoms or C₁₋₆ alkyl optionally substituted with a fluorine atom; and
Ar² is C₆₋₁₀ aryl, or 5- to 10-membered heteroaryl containing one or more ring heteroatoms independently selected from O, N and S, each of which is optionally substituted with one or more groups independently selected from the group consisting of a fluorine atom, cyano, C₁₋₆ alkyl, C₁₋₆ alkoxy, (C₁₋₆ alkoxy)carbonyl, (C₁₋₆ alkoxy)carbonylamino, (C₁₋₆ alkyl)carbonylamino, (C₆₋₁₀ aryl)carbonylamino, 5- to 10-membered heteroarylcarbonylamino containing one or more ring heteroatoms independently selected from O, N and S, di(C₁₋₆ alkyl)amino, 4- to 8-membered cyclic amino, aminocarbonyl, (C₁₋₆ alkyl)aminocarbonyl, di(C₁₋₆ alkyl)aminocarbonyl, and 4- to 8-membered cyclic aminocarbonyl.

6. The method according to any one of claims 1 to 5, wherein Ar² is independently selected from the group consisting of phenyl, naphthyl, pyrrolyl, thienyl, furyl, pyridyl, thiazolyl, isothiazolyl, pyrazolyl, oxazolyl, isoxazolyl, imidazolyl, triallyl, pyrimidyl, pyrazinyl, pyridazinyl, quinolinyl, isoquinolinyl, 4H-quinolidinyl, phthalazinyl, naphthyridinyl, quinoxalinyl, quinazolinyl, cinnolinyl, pteridinyl, indolyl, indolinyl, benzothiophenyl, benzofuranyl, benzisothiazolyl, benzisoxazolyl, indazolyl, benzimidazolyl, benzotriazolyl, azaindolyl, and imidazopyridyl, each of which is optionally substituted.

7. The method according to any one of claims 1 to 6, wherein the solvent is selected from the group consisting of N,N-dimethylpropionamide (DMPr), N,N-diethylacetamide (DEAc) and N,N-diethylpropionamide (DEPr).

8. The method according to any one of claims 1 to 7, wherein the solvent is N,N-dimethylpropionamide (DMPr).

9. The method according to any one of claims 1 to 8, wherein the solvent is a solvent containing at least one selected from the group consisting of N,N-dimethylpropionamide (DMPr), N,N-diethylacetamide (DEAc) and N,N-diethylpropionamide (DEPr) at 30 v/v% or more.

10. The method according to any one of claims 1 to 9, wherein the catalyst is a catalyst containing a palladium complex represented by any one of the following general formulae (Cat1), (Cat2), (Cat3), (Cat4) and (CatS): 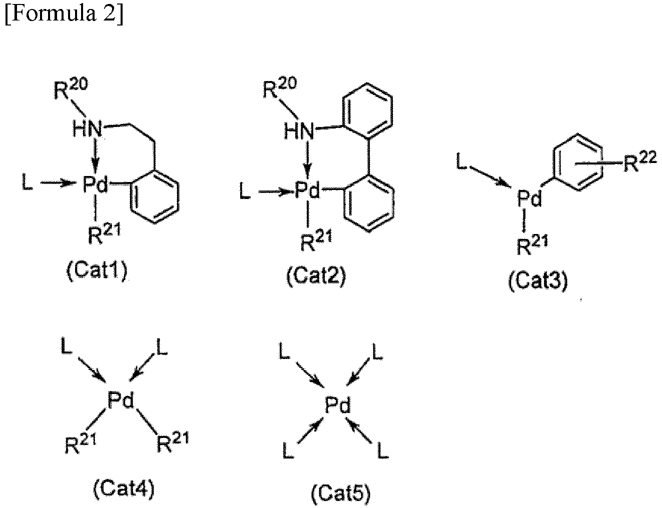 wherein R²⁰ is a hydrogen atom, C₁₋₆ alkyl, or C₆₋₁₀ aryl; R²¹ is halogen or -O-SO₂-CH₃; R²² is C₁₋₆ alkyl optionally substituted with one or more fluorine atoms, or (C₁₋₆ alkoxy)carbonyl optionally substituted with tri(C₁₋₆ alkyl)silyl; L is independently a monodentate ligand of the following general formula (L1), (L2), (L3), (L4), (L5), (L6) or (L7), or two L are a bidentate ligand of the following general formula (L8), (L9), (L10), (L11) or (L12): 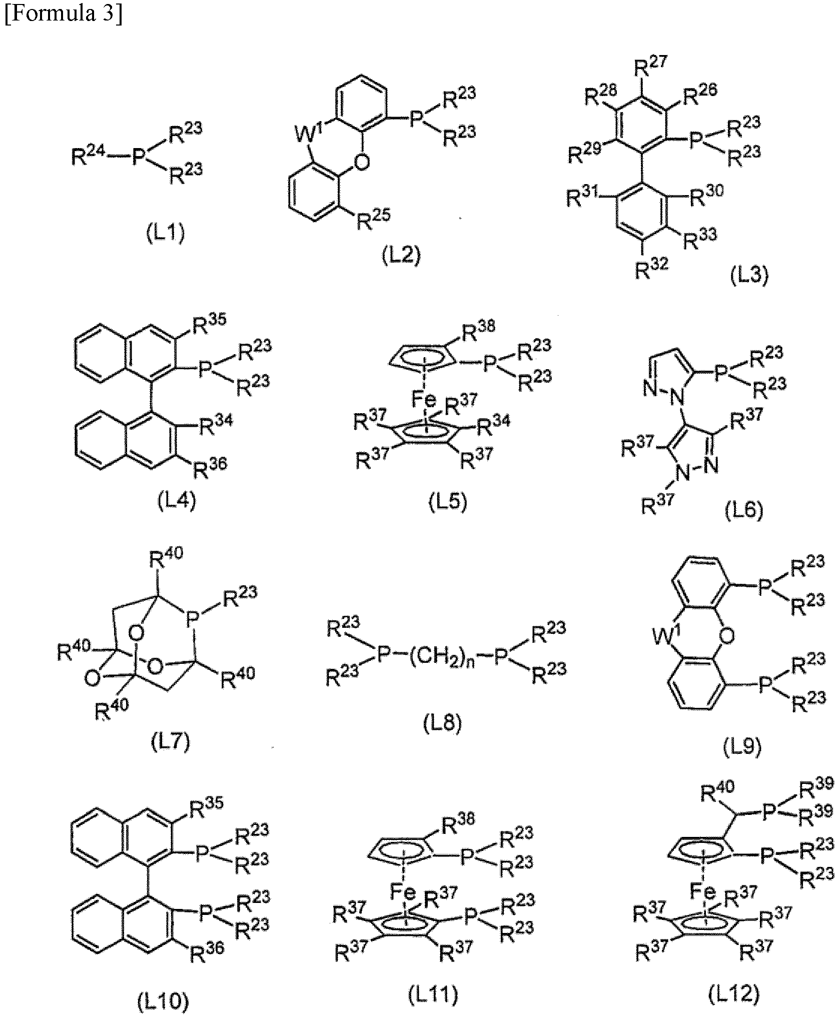
wherein R²³ is independently tert-butyl, cyclohexyl, 2-furanyl, 2-thienyl, 2-pyridyl, phenyl, or adamantyl, wherein the phenyl is optionally substituted with one or more fluorine atoms, C₁₋₆ alkyl optionally substituted with a fluorine atom, C₁₋₆ alkoxy, or dimethylamino;
R²⁴ is C₁₋₆ alkyl, cyclohexyl, 2-furanyl, 2-thienyl, 2-pyridyl, N-phenyl-2-pyrrolyl, N-phenyl-2-indolyl, phenyl, or adamantyl, wherein the phenyl is optionally substituted with one or more fluorine atoms, C₁₋₆ alkyl optionally substituted with a fluorine atom, C₁₋₆ alkoxy, morpholino, or dimethylamino;
R²⁵ is a hydrogen atom, C₁₋₆ alkyl, or C₁₋₆ alkoxy;
W¹ is -C(CH₃)₂-, or -NH-;
R²⁶, R²⁷, R²⁸, and R²⁹ are each independently a hydrogen atom, C₁₋₆ alkyl, C₁₋₆ alkoxy, or morpholino;
R³⁰, R³¹, and R³² are each independently a hydrogen atom, C₁₋₆ alkyl, C₁₋₆ alkoxy, or dimethylamino;
R³³ is a hydrogen atom or -SO₂-O-M, wherein M is lithium, sodium, or potassium;
R³⁴ is a hydrogen atom, C₁₋₆ alkyl, or C₁₋₆ alkoxy;
R³⁵ and R³⁶ are each independently a hydrogen atom, C₁₋₆ alkyl, or C₁₋₆ alkoxy;
R³⁷ is a hydrogen atom, or phenyl optionally substituted with C₁₋₆ alkyl;
R³⁸ is independently a hydrogen atom, phenyl optionally substituted with C₁₋₆ alkyl, or -CH(CH₃)-N(CH₃)₂;
R³⁹ is tert-butyl, cyclohexyl, or adamantyl;
R⁴⁰ is a hydrogen atom or C₁₋₆ alkyl; and an arrow represents a coordination bond.

11. The method according to any one of claims 1 to 9, wherein the catalyst is a catalyst containing a palladium complex represented by any of the following general formulae (Cat6) and (Cat7): 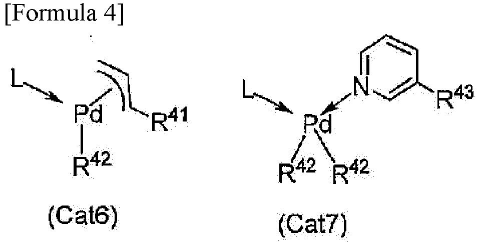 wherein R⁴¹ is a hydrogen atom or phenyl optionally substituted with C₁₋₆ alkyl; R⁴² is independently halogen; R⁴³ is a fluorine atom or a chlorine atom; and L is an N-heterocyclic carbene ligand represented by the following general formula (L12) or (L13): 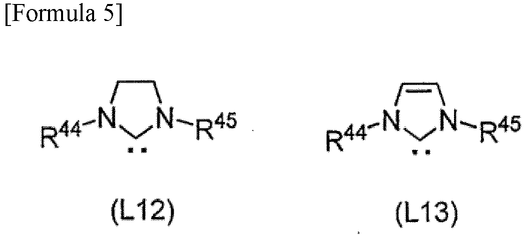 wherein R⁴⁴ and R⁴⁵ each independently represent C₁₋₆ alkyl, cyclohexyl, adamantyl, or phenyl, wherein the phenyl is optionally substituted with one or more C₁₋₆ alkyl, C₁₋₆ alkoxy, or dimethylamino, and a carbon atom with × × represents a carbene, and an arrow represents a coordination bond.

12. The method according to any one of claims 1 to 9, wherein the catalyst is a catalyst containing a palladium complex formed by a combination of a palladium compound selected from the group consisting of bis(allylchloropalladium(II)), tetrakis(triphenylphosphine)palladium(0), tris(dibenzylideneacetone)dipalladium(0)-chloroform adduct, a palladium(p-cinnamyl)chloride dimer, a (1-methylallyl)palladium chloride dimer, (1,5-cyclooctadiene)bis(trimethylsilylmethyl)palladium(II), a (2'-amino-1,1'-biphenyl-2-yl)methanesulfonatopalladium(II) dimer, and palladium(II) acetate, with a ligand selected from a ligand represented by a monodentate ligand of the following general formula (L1), (L2), (L), (L4), (L5), (L6) or (L7), or a bidentate ligand of the following general formula (L8), (L9), (L10), (L11) or (L12): 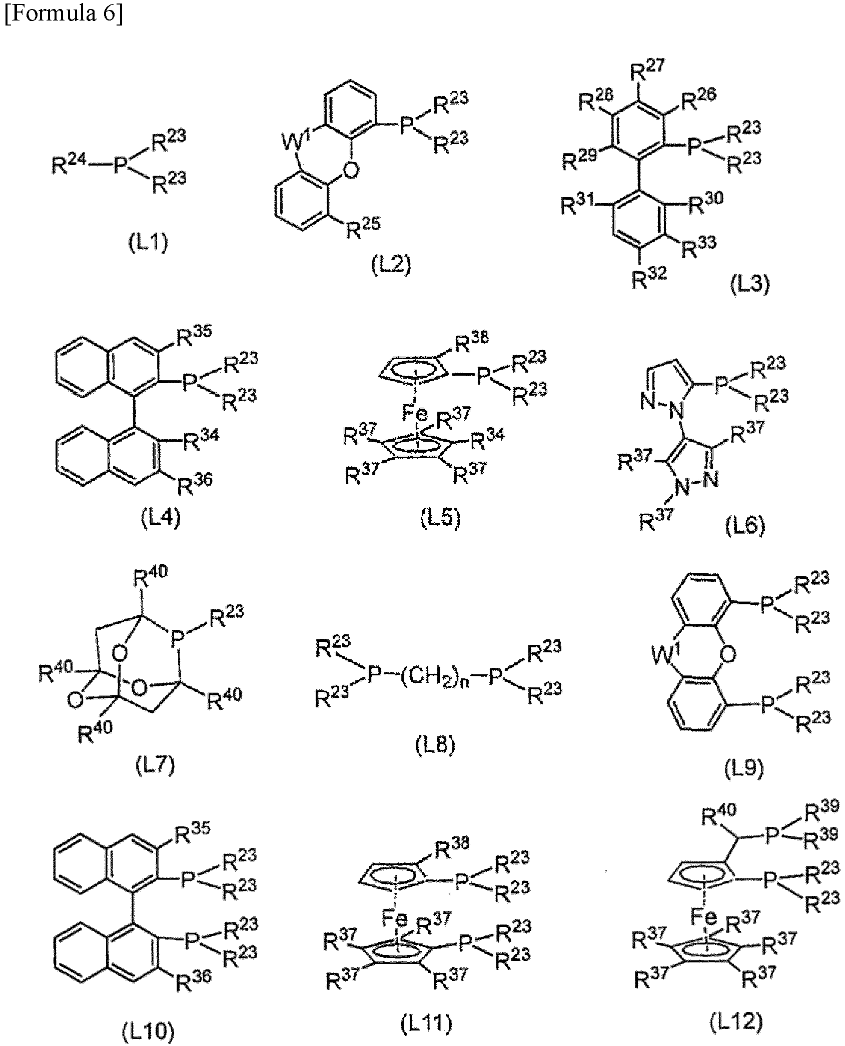 wherein R²³ is tert-butyl, cyclohexyl, 2-furanyl, 2-thienyl, 2-pyridyl, phenyl, or adamantyl, wherein the phenyl is optionally substituted with one or more fluorine atoms, C₁₋₆ alkyl optionally substituted with a fluorine atom, C₁₋₆ alkoxy, or dimethylamino;
R²⁴ is C₁₋₆ alkyl, cyclohexyl, 2-furanyl, 2-thienyl, 2-pyridyl, N-phenyl-2-pyrrolyl, N-phenyl-2-indolyl, phenyl, or adamantyl, wherein the phenyl is optionally substituted with one or more fluorine atoms, C₁₋₆ alkyl optionally substituted with a fluorine atom, C₁₋₆ alkoxy, morpholino, or dimethylamino;
R²⁵ is a hydrogen atom, C₁₋₆ alkyl, or C₁₋₆ alkoxy;
W¹ is -C(CH₃)₂-, or -NH-;
R²⁶, R²⁷, R²⁸, and R²⁹ are each independently a hydrogen atom, C₁₋₆ alkyl, C₁₋₆ alkoxy, or morpholino;
R³⁰, R³¹, and R³² are each independently a hydrogen atom, C₁₋₆ alkyl, C₁₋₆ alkoxy, or dimethylamino;
R³³ is a hydrogen atom or -SO₂-O-M, wherein M is lithium, sodium, or potassium;
R³⁴ is a hydrogen atom, C₁₋₆ alkyl, or C₁₋₆ alkoxy;
R³⁵ and R³⁶ are each independently a hydrogen atom, C₁₋₆ alkyl, or C₁₋₆ alkoxy;
R³⁷ is a hydrogen atom, or phenyl optionally substituted with C₁₋₆ alkyl;
R³⁸ is a hydrogen atom, phenyl optionally substituted with C₁₋₆ alkyl, or -CH(CH₃)-N(CH₃)₂;
R³⁹ is tert-butyl, cyclohexyl, or adamantyl;
R⁴⁰ is a hydrogen atom or C₁₋₆ alkyl; and an arrow represents a coordination bond, or a ligand being a salt thereof.

13. The method according to any one of claims 1 to 12, wherein the base includes at least one base selected from the group consisting of an organic base having a conjugate acid pKa of 23 or more in acetonitrile and an inorganic base having a conjugate acid pKa of 9 to 20 in water.

14. The method according to any one of claims 1 to 13, wherein a reaction system contains the base and further contains a salt.

15. The method according to claim 14, wherein the salt is an alkali metal salt of an acid selected from the group consisting of trifluoroacetic acid, trifluoromethanesulfonic acid, trifluoromethanesulfonimide, tetrafluoroboric acid, hexafluorophosphoric acid, and hexafluoroantimonic(V) acid.

16. A method of producing a compound, comprising the method according to any one of claims 1 to 15.

17. Use of a solvent in a cross-coupling reaction, the solvent containing an amide-based solvent represented by formula A: 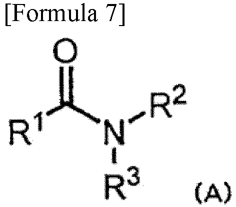 wherein R¹, R² and R³ are each independently C₁₋₄ alkyl provided that the sum of carbon atoms of R¹, R² and R³ is 4 or more and 6 or less.
